(19) Europäisches Patentamt
European Patent Office
Office européen des brevets

(11) EP 0 463 998 B1

(12) EUROPÄISCHE PATENTSCHRIFT

(45) Veröffentlichungstag und Bekanntmachung des Hinweises auf die Patenterteilung:
**15.01.1997 Patentblatt 1997/03**

(51) Int Cl.[6]: **C12P 21/08**, G01N 33/94, C12N 5/12, G01N 33/531, C07K 16/44, C07D 251/16

(21) Anmeldenummer: **91810483.7**

(22) Anmeldetag: **20.06.1991**

(54) **Immunologisches Nachweisverfahren für Triasulfuron**

Immunological detection of triasulfuron

Détection immunologique de triasulfuron

(84) Benannte Vertragsstaaten:
**AT BE CH DE DK ES FR GB GR IT LI LU NL SE**

(30) Priorität: **29.06.1990 CH 2173/90**
**21.12.1990 CH 4078/90**

(43) Veröffentlichungstag der Anmeldung:
**02.01.1992 Patentblatt 1992/01**

(83) **Erklärung nach Regel 28(4) EPÜ (Sachverständigenlösung)**

(73) Patentinhaber: **CIBA-GEIGY AG**
**4002 Basel (CH)**

(72) Erfinder:
- **Schlaeppi, Jean-Marc, Dr.**
  **CH-4051 Basel (CH)**
- **Ramsteiner, Klaus, Dr.**
  **CH-4132 Muttenz (CH)**
- **Meyer, Willy**
  **CH-4125 Riehen (CH)**

(56) Entgegenhaltungen:
**EP-A- 0 180 305** **WO-A-88/09798**

- **CHEMICAL ABSTRACTS, vol. 111, no. 23, 4 Dezember 89 Columbus, Ohio, USA Chigrin et al.: "Determination of chlorsulfuron & Agrokhimiya,8,119-23 Seite 205; ref. no. 111:210481U**
- **JOURNAL OF AGRICULTURAL FOOD CHEMISTRY vol. 33, 1985, WASHINGTON US Seiten 962**
- **- 965; M.M.Kelley et al.: "Chlorsulfuron determination in soil extracts by enzyme immunoassay"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 463 998 B1

Printed by Jouve, 75001 PARIS (FR)

**Beschreibung**

Gegenstand der vorliegenden Erfindung sind monoklonale Antikörper, die sich durch eine hohe Selektivität und Affinität gegenüber Herbiziden aus der Gruppe der Sulfonylharnstoffe, wie z.B. Triasulfuron, auszeichnen und die sich daher in hervorragender Weise für die Verwendung in einem Immunassay zum schnellen und effektiven Nachweis von Sulfonylharnstoff-Herbiziden in Boden-, Wasser- oder Luftproben oder auch in Pflanzenextrakten eignen sowie Verfahren zur Herstellung besagter monoklonaler Antikörper.

Ein weiterer Aspekt der vorliegenden Erfindung betrifft Hybridomazellinien, die besagte monoklonale Antikörper produzieren sowie immunologische Verfahren zum Nachweis von Sulfonylharnstoff-Herbiziden in Boden-, Wasser- oder Luftproben sowie in biologischem Material wie z.B. Pflanzenextrakten, unter Verwendung besagter monoklonaler Antikörper und die im Rahmen dieser Nachweisverfahren verwendbaren Testkits.

Der Einsatz synthetischer Herbizide für die Zwecke des Pflanzenschutzes und die damit in Zusammenhang stehenden Umweltbelastungen sind in jüngster Zeit immer häufiger in den Mittelpunkt der öffentlichen Diskussion gerückt.

Bei den Sulfonylharnstoffen handelt es sich um eine neue Klasse besonders wirksamer Herbizide, die für die Zwecke des Pflanzenschutzes eingesetzt werden, insbesondere zur selektiven und effektiven Bekämpfung breitblättriger Unkräuter in Getreidekulturen.

Ein Vertreter aus dieser Klasse ist das Triasulfuron [(3-(6-methoxy-4-methyl-1,3,5-triazin-2-yl-)-1-[2-(2-chlorethoxy)-phenylsulfonyl]-harnstoff], das in erster Linie in kleinkörnigen Getreidekulturen Anwendung findet zur selektiven Bekämpfung breitblättriger Unkräuter, wie z.B. *Viola tricolor oder Galium aparine.*

Der Nachweis von Herbiziden aus der Gruppe der Sulfonylharnstoffe in Boden- und Wasserproben wird heute in erster Linie mittels Gas- oder Flüssigchromatographie (z.B. HPLC) durchgeführt [GC: Ahmad und Crawford (1990); HPLC: Zahnow (1982); Iwanzik und Egli (1988)]; van Rensburg E (1985)] sowie unter Verwendung von Bioassays.

Da z.T. bereits geringste Rückstände dieser Herbizide im Boden immer noch eine hohe Restaktivität aufweisen, die insbesondere für Sulfonylharstoff-empfindliche Folgekulturen schädlich sein kann, ist es von besonderer Wichtigkeit ein zuverlässiges und in höchstem Masse sensitives Nachweisverfahren für die Rückstandsanalyse zur Verfügung zu haben.

Die meisten der zuvor genannten Verfahren zum Nachweis synthetischer Herbizide weisen zwar die für eine Rückstandsanalyse notwendige Sensitivität auf, deren Anwendung ist aber in der Regel noch mit zahlreichen Nachteilen verbunden. So müssen beispielsweise für die Bestimmung von Triasulfuron in Bodenproben mittels GC oder HPLC vor der Durchführung der eigentlichen chromatographischen Analyse aufwendige Reinigungs- und Aufkonzentrierungsschritte zwischengeschaltet werden.

Weitere Nachteile dieser Verfahren sind darin zu sehen, dass beispielsweise in der Gaschromatographie nur elementspezifische Detektoren eingesetzt werden, während bei der HPLC photometrische Detektoren verwendet werden, die relativ unspezifisch sind. Mit Ausnahme der massenspektroskopischen Detektion beruhen die chromatographischen Analysen auf der Bestimmung von Retentionszeiten der jeweiligen Substanz. Diese Werte sind aber relativ und damit nicht strukturspezifisch.

Die zuvor ebenfalls erwähnten Bioassay-Verfahren sind zwar ohne grossen Aufwand durchführbar, zeigen aber eine zu geringe Spezifität.

Um diese zuvor beschriebenen Nachteile der etablierten analytischen Verfahren zu vermeiden hat man in jüngster Zeit versucht auch für den Agrarsektor immunologische Verfahren zu entwickeln, - wie sie in der klinischen Diagnostik zum Nachweis der verschiedensten Antigene bereits routinemässig eingesetzt werden - , insbesondere für die quantitative und qualitative Bestimmung von Agrarchemikalien in Boden-, Wasser- oder Luftproben.

So hat man beispielsweise mittlerweile begonnen, immunologische Verfahren für den Nachweis bestimmter Herbizide wie 2,4-Dichlorphenoxyessigsäure (Fleeker, 1986) oder Chlorsulfuron (Kelley *et al*, 1985) sowie verschiedener Pestizide wie Diflubenzuron (Wie und Hammock, 1982), Metalaxyl (Newsome, 1985), Alachlor (Feng *et al*, 1990) oder Parathion (Ercegovich *et al,* 1981) zu entwickeln.

Auch für den immunologischen Nachweis von Herbiziden aus der Gruppe der Sulfonylharnstoffe sind somit bereits Verfahren beschrieben [Kelly *et al* (1985)], die jedoch, wie auch die zuvor genannten Methoden, auf der Verwendung polyklonaler Antiseren beruhen, die aus Tieren gewonnen werden, welche zuvor mit einem entsprechenden Antigen immunisiert wurden. Chirgin *et al* [Chem Abstr, Vol 111, No 23, Seite 205, Ref No 111: 210481U] beispielsweise beschreibt ein immunologisches Verfahren zum Nachweis von Chlorsulfuron unter Verwendung von Peroxidase-markierten Antikörpern gegen ein Chlorsulfuron (Glean)-Ovalbumin Konjugat. Die Herstellung monoklonaler Antikörper gegen Sulfonylharnstoff-Herbizide, die eine ausreichend hohe Affinität gegenüber der Zielsubstanz aufweisen und damit geeignet sind für den erfindungsgemässen Einsatz in einem der bekannten Immunoassays, ist dagegen bisher nicht gelungen.

Polyklonale Antiseren sind in ihrer Zusammensetzung sehr heterogen, d.h. sie enthalten eine Vielzahl verschiedener Antikörper, die mit unterschiedlichen Epitopen des jeweiligen Antigens reagieren. Diese heterogene Zusammensetzung polyklonaler Antiseren kommt dadurch zustande, dass bei der Immunisierung eines Versuchstieres mit einem

bestimmten Antigen immer mehrere Antikörper-produzierende Zellklone gleichzeitig stimuliert werden, von denen jeder ein anderes Epitop auf dem Antigenmolekül erkennt und daher von den stimulierten Zellklonen unterschiedliche Antikörper produziert werden. Aus diesem Grund sind Seren immunisierter Tiere immer polyklonal und somit heterogen, sowohl was ihre Spezifität und als auch ihre Zugehörigkeit zu den einzelnen Klassen von Immunglobulinen angeht. Diese Heterogenität in der Zusammensetzung polyklonaler Antiseren kann daher dazu führen, dass aufgrund zu geringer Affinität die Nachweisempfindlichkeit nicht ausreichend ist für einen selektiven Nachweis einer bestimmten Zielsubstanz oder dass strukturell nahe verwandte Verbindungen wie beispielsweise Triasulfuron und dessen Hydroxylierungsprodukte bei der Verwendung polyklonaler Antikörper im Rahmen eines Immunassays nicht in ausreichendem Masse differenziert werden können. Um diese Nachteile der polyklonalen Antiseren zu überwinden, wurden in jüngster Zeit vermehrt Anstrengungen unternommen auch für den Agrarsektor monoklonale Antikörper zu entwickeln. So berichten Schlaeppi *et al* (1989) über die Herstellung und Verwendung monoklonaler Antiköprer gegen Atrazin und Hydroxyatrazin in einem Enzym-gekoppelten Immunassay.

Die Aufgabe, die es im Rahmen dieser Erfindung zu lösen galt, betraf somit in erster Linie die Bereitstellung eines einfach handhabbaren, effektiven und in hohem Masse selektiven Immunassays für einen schnellen und zuverlässigen Nachweis von Sulfonylharnstoff-Herbiziden, insbesondere aber von Triasulfuron, in Boden-, Wasser- und Luftproben.

Diese Aufgabe konnte jetzt im Rahmen der vorliegenden Erfindung überraschenderweise gelöst werden und zwar durch die Bereitstellung von monoklonalen Antikörpern mit hoher Spezifität und Affinität gegenüber Sulfonylharnstoff-Herbiziden, insbesondere aber gegenüber Triasulfuron, unter Verwendung der an sich bekannten Hybridoma/monoklonalen Antikörper-Technologie.

Die Verwendung hybrider somatischer Zellinien (Hybridomas) als Quelle für Antikörper gegen ganz bestimmte Antigene geht auf die Arbeiten von Köhler und Milstein (Nature, 256: 495-97, 1975) zurück.

Die Antikörper, die sich mit dem dort beschriebenen Verfahren gewinnen lassen, unterscheiden sich sehr stark von denjenigen, die man aus Antiseren konventionell immunisierter Tiere erhält.

Das Prinzip der Hybridoma/monoklonalen Antikörper-Technologie gründet sich auf die Beobachtung, dass beim Verschmelzen zweier somatischer Zellen die resultierende Hybridzelle charakteristische Merkmale beider Elterntypen aufweist.

Im Falle der monoklonalen Antikörperproduktion stammt die Fähigkeit zur Synthese des spezifischen Antikörpers von einer immunkompetenten B-Zelle (gewöhnlich einer Milzzelle), die einem zuvor immunisierten Donor-Tier entnommen wurde, während die Fähigkeit zur kontinuierlichen Zellteilung in Kultur durch den anderen Fusionspartner, eine Tumorzellinie (oft ein Myeloma) beigesteuert wird.

Für die Immunisierung der Donortiere werden in der Regel Konjugate bestehend aus dem Zielmolekül und einem damit verknüpften hochmolekularen Carriermolekül verwendet.

Jede dieser Hybrid-Zellinien synthetisiert ein homogenes Immunglobulin, das nur einen einzigen Vertreter aus der grossen Anzahl möglicher Antikörper repräsentiert, die von einem Tier als Antwort auf ein Antigen *in vivo* synthetisiert werden kann.

Da jeder Immunglobulin-produzierende Klon durch einen einzigen Typ von Antikörpern charakterisiert wird, hat sich die Bezeichnung monoklonale Antikörper eingebürgert.

Die Vorteile monoklonaler gegenüber polyklonalen Antikörpern sind zahlreich:

a) monokolonale Antikörper können in grosser Anzahl und in hohem Reinheitsgrad erhalten werden,

b) die Herstellung monoklonaler Antikörper ist homogen im Hinblick auf die Antigen-Reaktivität und ändert sich auch nicht im Verlaufe der Zeit,

c) monoklonale Antikörper produzierende Hybridomas können über Jahre und Jahrzehnte hinweg aufbewahrt werden, ohne dabei ihre spezifischen Eigenschaften, i.e. die Produktion spezifischer monoklonaler Antikörper, zu verlieren,

d) monoklonale Antikörper sind für die Verwendung als Standardreagentien besser geeignet als polyklonale Antiseren, da letztere negativ beeinflusst werden durch eine grosse Variationsbreite beispielsweise im Hinblick auf

α) die Blutentnahme immunisierter Tiere zur Gewinnung des Antiserums,

β) eine ständige Verfügbarkeit von Material für zusätzliche Immunisierungen,

γ) die begrenzte Lebenszeit der Donortiere.

Monoklonale Antikörper, die mittlerweile gegen eine Vielzahl von Antigenen hergestellt wurden, sind in erster Linie

in der medizinischen Diagnostik gut etabliert und aus dieser nicht mehr wegzudenken.

Im Rahmen der vorliegenden Erfindung wird jetzt erstmals ein Verfahren zur Verfügung gestellt, das es erlaubt unter Anwendung der an sich bekannten und zuvor kurz skizzierten Hybridoma/monoklonalen Antikörper-Technologie monokonale Antikörper mit hoher Spezifität und Affinität gegenüber Sulfonylharnstoff-Herbiziden, insbesondere aber gegenüber Triasulfuron, herzustellen, die aufgrund ihrer hohen Affinität für einen erfindungsgemässen Einsatz in einem der bekannten Immunoassays geeignet sind.

Im einzelnen handelt es sich dabei um ein Verfahren, das im wesentlichen dadurch gekennzeichnet ist, dass man bei der Herstellung von Konjugaten für die Immunisierung der Donortiere anstelle des gesamten Sulfonylharnstoff-Moleküls vorzugsweise nur ein Fragment verwendet, das insbesondere den Sufonamidteil des Moleküls umfasst, und dieses Fragment mit einem der üblicherweise verwendeten, hochmolekularen Carriermoleküle verknüpft.
Nach Auslösen der Immunantwort im Donortier werden die für die Antikörperproduktion verantwortlichen Zellen in der nachfolgend im einzelnen beschriebenen Weise aus dem Donortier isoliert und zur Herstellung von Hybridomazellen mit geeigneten Myelomazellen fusioniert.

Gegenstand der vorliegenden Erfindung ist somit ein Verfahren zur Herstellung eines monoklonalen Antikörpers mit hoher Spezifität und Affinität gegenüber einem oder mehreren Sulfonylharnstoff-Herbiziden, das sich dadurch kennzeichnet, dass man

(a) eine Kupplungskomponente, die im wesentlichen aus dem Sulfonamidteil des Zielmoleküls besteht, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert.

Die aus dem erfindungsgemässen Verfahren resultierenden monoklonalen Antikörper bilden einen weiteren Gegenstand der vorliegenden Erfindung. Bevorzugt sind monoklonale Antikörper mit hoher Spezifität bzw Affinität gegenüber Sulfonylharnstoff-Herbiziden, insbesondere aber gegenüber Triasulfuron, die aufgrund ihrer geringen Kreuzreaktivität in hervorragender Weise für eine Verwendung in einem Immunassay zum schnellen und zuverlässigen Nachweis von Sulfonylharnstoff-Herbiziden, insbesondere aber von Triasulfuron, geeignet sind und die damit auch für eine Differenzierung der Zielsubstanzen, wie z.B. Triasulfuron, gegenüber strukturell verwandten Verbindungen, insbesondere aber gegenüber den Hydroxylierungsprodukten sowie inaktiven Metaboliten, verwendet werden können.

Besonders bevorzugt im Rahmen der vorliegenden Erfindung sind somit in erster Linie monoklonale Antikörper und Derivate davon, die eine hohe Spezifizität und Affinität gegenüber Triasulfuron aufweisen und die mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) aus Tabelle 1 eine Kreuzreaktivität von $<1.0\%$ zeigen.

Ebenfalls besonders bevorzugt sind monoklonale Antikörper und Derivate davon, die eine hohe Spezifizität und Affinität gegenüber Triasulfuron aufweisen und mit strukturell verwandten Triasulfuronanalogen ausgewählt aus der Gruppe bestehend aus den Verbindungen C, D, E. F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) aus Tabelle 1 eine Kreuzreaktivität von $<0{,}01\%$ zeigen.

Ganz besonders bevorzugt sind monoklonale Antikörper und Derivate, welche die genannten Kreuzreaktivitäten zeigen und die aus Hybridomazellinien erhältlich sind, die unter der Nummer ECACC 9002 1703 hinterlegt worden sind , sowie aus Klonen und Subklonen davon.

Ebenfalls besonders bevorzugt sind monoklonale Antikörper und Derivate, welche die genannten Kreuzreaktivitäten zeigen und die aus Hybridomazellinien erhältlich sind, die unter der Nummer ECACC 9002 1704 hinterlegt worden sind, sowie aus Klonen und Subklonen davon.

Unter Derivaten monoklonaler Antikörper sind im Rahmen der vorliegenden Erfindung z.B. Antikörperfragmente zu verstehen, die noch die hohe Spezifität und Affinität für die antigenen Determinanten von Triasulfuron besitzen, desweiteren radioaktiv markierte monoklonale Antikörper, die beispielsweise mit radioaktivem Jod ($^{125}I$, $^{131}I$) Kohlenstoff ($^{14}C$), Schwefel ($^{35}S$), Tritium ($^{3}H$) oder ähnlichem markiert sind, Konjugate monoklonaler Antikörper mit Biotin oder Avidin, mit Enzymen, wie Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase oder Glucose-6-phosphatdehydrogenase, desweiteren Konjugate monoklonaler Antikörper mit biolumineszierenden (z.B. Luciferase), chemolumineszierenden (z.B. Acridiniumester) oder fluoreszierenden (z.B. Phycobiliproteine) Agentien. Ebenso umfasst

von der vorliegenden Anmeldung sind bispezifische sowie sog. "cross-linked" Antikörper. Diese beispielhafte Aufzählung möglicher Antikörperderivate dient lediglich der Illustration der vorliegenden Erfindung und soll den Erfindungsgegenstand in keiner Weise limitieren.

Unter der Bezeichnung "im wesentlichen keine Kreuzreaktivität" soll im Rahmen dieser Erfindung verstanden werden, dass die Reaktivität der für Triasulfuron spezifischen monoklonalen Antikörper mit unspezifischen Epitopen anderer Verbindungen, insbesondere strukturell verwandter Verbindungen wie z.B. den Hydroxylierungsprodukten, in der Mehrzahl der Fälle weniger als 10 %, vorzugsweise aber weniger als 1.5 % und ganz besonders bevorzugt weniger als 0.1 % beträgt.

Der prozentuale Anteil der Kreuzreaktivität soll im Rahmen dieser Erfindung definitionsgemäss durch die folgende Beziehung wiedergegeben werden:

[Triasulfuronkonzentration für 50 % Hemmung ($I_{50}$)/Konzentration der

Triasulfuronanalogen für 50 % Hemmung ($I_{50}$)] x 100.

Der $I_{50}$-Wert kann beispielsweise mit Hilfe eines kompetitiven ELISA Assays (vgl. Beispiel 8) bestimmt werden. Diese entspricht dann beispielsweise derjenigen Antigenkonzentration, die zu einer 50 %igen Hemmung der Antikörperbindung an das Träger-gebundene Antigen führt.

Ein weiterer Gegenstand der vorliegenden Erfindung betrifft Hybridomazellinien, welche die zuvor näher charakterisierten monoklonalen Antikörper synthetisieren und vorzugsweise in das umgebende Medium sezernieren.

Besonders bevorzugt ist eine Hybridomazellinie, die einen monoklonalen Antikörper synthetisiert und vorzugsweise in das umgebende Medium sezerniert, der eine hohe Spezifizität und Affinität gegenüber Triasulfuron aufweist und mit strukturell verwandten Triasulfuronanalogen ausgewählt aus der Gruppe bestehend aus den Verbindungen C, D, E. F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) aus Tabelle 1 eine Kreuzreaktivität von <1,0% zeigt.

Ebenfalls besonders bevorzugt ist eine Hybridomazellinie, die einen monoklonalen Antikörper synthetisiert und vorzugsweise in das umgebende Medium sezerniert, der eine hohe Spezifizität und Affinität gegenüber Triasulfuron aufweist und mit strukturell verwandten Triasulfuronanalogen ausgewählt aus der Gruppe bestehend aus den Verbindungen C, D, E. F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) aus Tabelle 1 eine Kreuzreaktivität von <0,01% zeigt.

Ganz besonders bevorzugt ist eine Hybridomazellinien, welche monoklonale Antikörper produziert, die die obengenannten Kreuzreaktivitäten zeigen und die unter der Nummer ECACC 9002 1703 hinterlegt ist, sowie Klone und Subklone davon.

Ebenfalls besonders bevorzugt ist eine Hybridomazellinien, welche monoklonale Antikörper produziert, die die obengenannten Kreuzreaktivitäten zeigen und die unter der Nummer ECACC 9002 1704 hinterlegt ist, sowie Klone und Subklone davon.

Ebenso umfasst von der vorliegenden Erfindung sind Varianten und Mutanten der zuvor näher charakterisierten Hybridomazellinien, die spontan entstehen oder aber artifiziell mit Hilfe bekannter Verfahren hergestellt werden können und die noch die charakteristischen Eigenschaften des Ausgangsmaterials aufweisen, d.h. die noch in der Lage sind die erfindungsgemässen Antikörper oder Derivate davon zu produzieren und diese vorzugsweise ins umgebende Medium zu sezernieren.

Ebenso umfasst von der vorliegenden Erfindung sind Verfahren zur Herstellung besagter Hybridomazellinien sowie Verfahren zur Herstellung besagter monoklonaler Antikörper.

Unter Klonen und Subklonen von Hybridomazellinien sind Hybridomas zu verstehen, die durch wiederholtes Klonieren aus dem Ausgangsklon hervorgehen und die noch die erfindungswesentlichen Merkmale des Ausgangsklons aufweisen.

Ein weiterer Gegenstand dieser Erfindung betrifft ein Verfahren zum immunologischen Nachweis von Sulfonylharnstoff-Herbiziden, insbesondere aber von Triasulfuron, z.B. in Boden-, Wasser- oder Luftproben sowie in biologischem Material, wie z.B. in pflanzlichen oder tierischen Extrakten, unter Verwendung der erfindungsgemässen monoklonalen Antikörper.

Ebenso ein Bestandteil der vorliegenden Erfindung sind Mittel für die qualitative und quantitative Bestimmung von Sulfonylharnstoff-Herbiziden, insbesondere aber von Triasulfuron, in Form gebrauchsfertiger Test-Kits, welche mindestens einen der erfindungsgemässen monoklonalen Antikörper als Reagenz enthalten und die für die Verwendung unter Feldbedingungen für einen schnellen und zuverlässigen Nachweis von Sulfonylharnstoff-Herbiziden, insbesondere aber von Triasulfuron geeignet sind.

Die Herstellung monoklonaler Antikörper erfolgt in der Regel mit Hilfe an sich bekannter Verfahren, die im wesentlichen auf den von Köhler und Milstein (1975) entwickelten Methoden beruhen.

Da es sich bei den zu analysierenden Zielsubstanzen [Sulfonylharnstoff-Herbizide] wie beispielsweise Triasulfuron, für die spezifische monoklonale Antikörper entwickelt werden sollen, um relativ kleine und einfache Moleküle handelt, die nach der Applikation in ein Versuchstier alleine nicht in der Lage sind dort eine entsprechende Immunantwort auszulösen, müssen vor der eigentlichen Immunisierung zunächst vorbereitende Massnahmen getroffen werden.

Man bezeichnet solche Verbindungen, die aufgrund ihrer Grösse und einfachen Strukturierung nicht zur Induktion einer Immunreaktion in der Lage sind, als Haptene oder inkomplette Antigene und stellt sie somit den kompletten Antigenen (= Immunogene) gegenüber, die sowohl als Antigen wirken als auch eine Immunantwort zu induzieren vermögen. Solche Haptenmoleküle können an hochmolekulare Verbindungen (Trägermoleküle) konjugiert werden, wodurch sie in ihren Eigenschaften kompletten Antigenen vergleichbar werden, d.h. sie besitzen jetzt die Fähigkeit zur Auslösung einer Immunantwort.

Einige der im Verlaufe der Immunisierungsreaktion gebildeten Antikörper sind dann in der Lage mit spezifischen Epitopen auf dem Haptenmolekül zu reagieren, unabhängig davon, ob das Haptenmolekül alleine oder aber nach wie vor gekoppelt an das Carriermolekül vorliegt.

Unter der im folgenden häufig verwendeten Bezeichnung Hapten soll im Rahmen dieser Erfindung in erster Linie das für die Immunisierung verwendete Triasulfuron-Molekül sowie Teile davon verstanden werden.

Im Rahmen dieser Erfindung wird daher die als Hapten wirkende Zielsubstanz vor der Immunisierung von Versuchstieren an einen hochmolekularen Träger ('Carrier') gekoppelt, der geeignet ist dieser eine komplette antigene Wirksamkeit zu verleihen.

Unter geeigneten Carrier-Molekülen sind im Rahmen dieser Erfindung in erster Linie makromolekulare Verbindungen zu verstehen, die frei zugängliche reaktive Gruppen für die Kupplungsreaktion mit dem Hapten aufweisen und die in der Lage sind, durch Kupplung an das Hapten, diesem eine immunogene Potenz zu verleihen oder aber deren bereits vorhandene Immunogenizität zu verstärken.

Besonders bevorzugt im Rahmen dieser Erfindung sind makromolekulare Verbindungen, die frei zugängliche reaktive Aminogruppen enthalten.

Ganz besonders bevorzugt für die erfindungsgemässe Verwendung als Carriermolekül sind lysinreiche Proteine mit einem Molekulargewicht zwischen 10'000 und 1'500'000, wie z.B. "Bovine Serum Albumin" (BSA: MG 66'200), Humanes Serum Albumin (HSA,; MG 58'000) oder "Keyhole Limpet Hemocyanin" (KLH; MG >1'000'000), die kommerziell erhältlich sind und somit in beliebiger Menge zur Verfügung stehen.
Selbstverständlich können im Rahmen der vorliegenden Erfindung auch andere makromolekulare Verbindungen als Carrier-Moleküle verwendet werden, sofern sie die oben genannten Voraussetzungen erfüllen, wie z.B. "Porcine Thyroglobulin", B2 Microglobulin, Hemocyanin, Immunglobuline, Toxine (Cholera-, Tetanus-, Diphtheria-Toxin u.a.), Polysaccharide, Lipopolysaccharide, natürliche oder synthetische Polyadenyl- und Polyuridylsäuren, Polyalanyl- und Polylysin-Polypeptide oder Zellmembrankomponenten, wie beispielsweise Formalin- oder Glutaraldehyd-behandelte Erythrozytenzellmembranen.

Ebenso geeignet für die Verwendung als Carriermolekül in dem erfindungsgemässen Verfahren ist beispielsweise die gereinigte IgG Fraktion gegen Maus IgG (H+L) aus Kaninchen gemäss dem bei H Kawamura und JA Berzofsky (1986) beschriebenen Verfahren.

Die Konjugation des Haptens an das Trägermolekül kann entweder direkt oder aber vorzugsweise über ein Brükkenglied erfolgen, welches gegebenenfalls zunächst an das Haptenmolekül angelagert wird.

Die Kupplung der zu analysierenden Substanz an das Trägermolekül muss dabei in einer Weise erfolgen, dass die relevanten Strukturelemente der Zielsubstanzen frei zugänglich bleiben und somit in der Lage sind eine spezifische Immunantwort auszulösen, d.h. die Bildung spezifischer Antikörper zu induzieren. Bei der Herstellung der Sulfonylharnstoff-Derivate, insbesondere aber der Triasulfuron-Derivate, ist daher darauf zu achten, dass diese Strukturelemente erhalten bleiben.

Im Rahmen der vorliegenden Erfindung hat es sich überraschenderweise gezeigt, dass für die Auslösung einer spezifischen Immunantwort nicht notwendigerweise das gesamte, intakte Sulfonylharnstoffmolekül vorliegen muss, sondern dass im Gegenteil auch ausgewählte Molekül-Fragmente zu ausgezeichneten und im Hinblick auf die Affinität des resultierenden Antikörpers letztlich sogar besseren Ergebnissen führen. So lassen sich beispielsweise monoklonale Antikörper mit hoher Affinität und Selektivität gegenüber Herbiziden der Sulfonylharnstoffklasse herstellen, indem man anstelle des komplexen Gesamtmoleküls, bestehend aus Sulfonamidteil, Harnstoffbrücke und Heterocyclusteil, ausschliesslich den Sulfonamidteil als Kupplungskomponente verwendet und diesen, wie zuvor beschrieben, mit einem geeigneten Carriermolekül verknüpft. Dies ist von grossem verfahrenstechnischem Vorteil, da die Handhabung von Molekül-Fragmenten einfacher ist als diejenige des sehr viel komplexeren Gesamtmoleküls.

Wie im Rahmen der vorliegenden Erfindung gezeigt werden konnte, führt die Immunisierung von Donortieren mit dem zuvor beschriebenen Konjugat, bestehend aus einem der üblicherweise verwendeten, hochmolekularen Carrier und dem Sulfonamidteil eines Sulfonylharnstoff-Herbizids, zur Produktion hochspezifischer Antikörper, was letztlich

die Herstellung monoklonaler Antikörper ermöglicht, die eine im Durchschnitt sehr viel höhere Affinität gegenüber dem jeweiligen Zielmolekül aufweisen, als dies bei Verwendung des Gesamtmoleküls als Kupplungskomponente der Fall ist.

Ein wesentlicher Aspekt der vorliegenden Erfindung besteht somit in der Verwendung des Sulfonamidteils von Sulfonylharnstoff-Herbiziden zur Herstellung von Konjugaten, die für die Immunisierung von Donortieren eingesetzt werden können und letztlich zur Produktion monoklonaler Antikörper mit hoher Affinität gegenüber besagten Herbizidmolekülen führen.

Bei besagtem Sulfonamidteil handelt es sich vorzugsweise um substituiertes Arylsulfonyl, wobei der Arylrest normalerweise ein Phenyl, Pyridyl, Thienyl oder Pyrazolyl ist. Typische Vertreter von Sulfonylharnstoff-Herbiziden sind z. B. in EP-0,158,600 sowie in EP-0,367,887 und der darin jeweils zitierten Literatur beschrieben.

In einer bevorzugten Ausführungsform der vorliegenden Erfindung wird daher nicht das gesamte Triasulfuronmolekül an das Trägermolekül gekoppelt, sondern nur ein ausgewählter Teil davon, der die jeweils gewünschte determinante Gruppe in einer Form enthält, die eine sehr spezifische Immunantwort auslöst. Besonders bevorzugt im Rahmen dieser Erfindung ist ein Triasulfuron-Fragment, das auf den Sulfonamidanteil des Triasulfuron beschränkt bleibt.

Besagter Sulfonamidanteil kann dabei beispielsweise dadurch erhalten werden, dass man das entsprechende Sulfonylharnstoff-Herbizid an der Sulfonamid-Funktion nach an sich bekannten Methoden hydrolytisch spaltet. Das auf diese Weise erhältliche Sulfonamid-Kupplungsfragment weist somit eine endständige $-SO_2-NH_2$ Gruppe auf, an die jetzt sehr einfach ein geeignetes Brückenglied angekoppelt werden kann, das für die nachfolgende Konjugation des Haptenmoleküls einsetzbar ist. Die Ankopplung des Brückenglied kann beispielsweise dadurch erreicht werden, dass man die zuvor erwähnte $-SO_2-NH_2$ Gruppe mit einem reaktionsfähigen Carbonsäurederivat wie z.B. einem Bernsteinsäurederivat, oder aber vorzugsweise mit dem entsprechenden Anhydrid umsetzt. Bei Verwendung eines Bernsteinsäurederivates entsteht dabei die Gruppe $-SO_2-NH-C(O)-CH_2CH_2COOH$. Die Umsetzung wird vorzugsweise in basischem Milieu durchgeführt, ganz besonders bevorzugt in Gegenwart eines geeigneten Katalysators wie beispielsweise des in Beispiel 1.2 verwendeten 1,8-Diazabicyclo[5.4.0.]undec-7-en.

Die endständige Carboxylgruppierung kann anhand bekannter, in der Literatur vielfach beschriebener Verfahren auch in eine Amino- oder eine SH-Gruppe überführt werden, die ebenfalls über eine für die Ankopplung des Haptenmoleküls ausreichenden Reaktivität verfügen.

Als Brückenglieder für eine Konjugation des Haptens an das Carriermolekül kommen somit in erster Linie Verbindungen in Betracht, die mindestens eine oder aber mehrere reaktive Gruppen enthalten, welche in der Lage sind mit den frei zugänglichen reaktiven Gruppen des Carrier-Moleküls in Wechselwirkung zu treten.

Besonders bevorzugt im Rahmen dieser Erfindung ist die Verwendung von Brückengliedern die zwischen 2 und 10 Brücken-C-atome umfassen und die als reaktive Gruppe(n) eine oder mehrere reaktive Gruppen, wie z.B. Amino-, Carboxyl- oder SH-Gruppe(n) besitzen. Diese reaktiven Gruppen können mit Hilfe an sich bekannter Verfahren mit den reaktiven Gruppen des Hapten- und Carrier-Moleküls zur Reaktion gebracht werden, unter Ausbildung eines Hapten-Carrier-Konjugates.

So ist es beispielsweise möglich ein Brückenglied über eine reaktive Aminogruppe mit Hilfe von Dialdehyden (z. B. Glutardialdehyd) an eine der freien Aminogruppen des Carrier-Moleküls zu binden. Besitzt das Brückenglied eine reaktive SH-Gruppe, so kann die Konjugation des Haptens an das Carrier-Molekül durch eine Oxidation mit freien SH-Gruppen des Carriers durchgeführt werden.

Besonders bevorzugt im Rahmen dieser Erfindung ist die Verwendung von Brückengliedern mit einer Carboxylgruppe, die mit Hilfe von wasserbindenden Mitteln, wie beispielsweise einem Carbodiimid, vorzugsweise N,N'-Dicyclohexylcarbodiimid, mit einer freien Aminogruppe des Carriermoleküls verknüpft werden kann.

Um das Antigen an das Trägerprotein zu kuppeln, ist es somit vorteilhaft zunächst ein zu dieser Kupplung befähigtes Derivat herzustellen.

Es hat sich in Rahmen der vorliegenden Erfindung überraschenderweise gezeigt, dass anstelle der komplexen Moleküle der Formel (A)

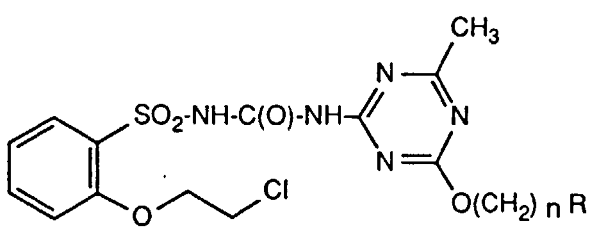

(A)

die in 4 Stellung des Triazinringes eine $R-(CH_2)_n-O-$ Gruppierung aufweisen, worin

R für COOH steht, wenn n eine ganze Zahl von 1 bis 10, vorzugsweise von 1 bis 6, repräsentiert und
R für COOH, , $NH_2$ oder SH, insbesondere aber für $NH_2$ steht, wenn n eine ganze Zahl von 2 bis 10, vorzugsweise von 2 bis 6, repräsentiert,

die wesentlich einfacheren Fragmente der Formel (B) als Kupplungskomponenten eingesetzt werden können.

Im Rahmen der vorliegenden Erfindung verwendet man daher zur Herstellung von Konjugaten, die nach Immunisierung eines Donortieres zu einer spezifischen Immunantwort und letztlich zur Herstellung von monoklonalen Antikörpern mit hoher Affinität gegenüber Sulfonylharnstoff-Herbiziden der Formel (A) führen, den Sulfonamidteil von besagtem Sulfonylharnstoff-Herbizid. Durch Überführen dieses Sulfonamidteils in das stabilere Fragment (B) erhält man eine geradezu ideale Kupplungskomponente, die sich in hervorragender Weise für die Herstellung monoklonaler Antikörper mit einer hohen Affinität gegenüber Sulfonylharnstoff-Herbiziden der Formel (A) eignen.

Besonders bevorzugt sind Triasulfuron-Fragmente der Formel (B),

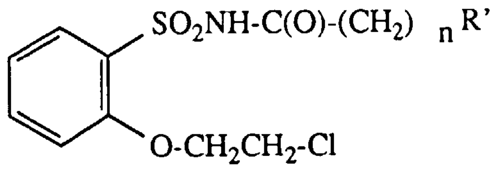

(B)

die man sich durch Austausch der Triazinamin-Gruppierung gegen eine $R'\text{-}(CH_2)_n$-Gruppe entstanden denken kann, worin

R′ für COOH, $NH_2$ oder SH, insbesondere aber für COOH steht und
n eine ganze Zahl von 1 bis 10, vorzugsweise von 1 bis 6 repräsentiert, und die somit auf den Sulfonamidanteil beschränkt sind.

Bevorzugt im Rahmen der vorliegenden Erfindung ist das Triasulfuron-Derivat der Formel (IV), das, wie nachfolgend im einzelnen beschrieben wird, in einem 4-stufigen Verfahren hergestellt werden kann. Die in diesem Verfahren verwendbaren Ausgangsverbindungen und Reaktanten sind bekannt oder können analog zu bekannten strukturähnlichen Verbidnungen hergestellt werden.

Ganz besonders bevorzugt im Rahmen dieser Erfindung ist das Triasulfuron-Derivat der Formel (V), das man sich durch Austausch der Triazinamin-Gruppierung gegen eine $-(CH_2)(CH_2)COOH$ Gruppierung entstanden denken kann.

Die resultierenden, zur Kupplung befähigten Triasulfuronderivate sind neu und stellen durch ihre spezifische Kupplungsfähigkeit ein wertvolles Ausgangsmaterial bei der Herstellung monoklonaler Antikörper mit Triasulfuronspezifität dar. Sie sind daher ein wichtiger Bestandteil vorliegender Erfindung.

Die Durchführung der eigentlichen Kupplungsreaktion erfolgt je nach verwendetem Triasulfuronderivat vorzugsweise mit Hilfe der aktiven Estermethode oder dem Diazoniumverfahren [Kelly *et al* (1985)]. Bei Verwendung der aktiven Estermethode wird das Triasulfuronderivat zunächst in einem geeigneten Lösungsmittel solubilisiert. Als geeignete Lösungsmittel kommen insbesondere aprotische Lösungsmittel, die eine geringe Verdampfungsrate aufweisen, wie z.B. N,N-Dimethylformamid (DMF) oder Dimethylsulfoxid (DMSO) in Betracht.

Anschliessend werden die Carboxylgruppen zu einem aktiven Ester derivatisiert, indem das zuvor solubilisierte Triasulfuronderivat z.B. mit N-Hydroxysuccinimid, N-Hydroxysulfosuccinimid, N,N′-Dicyclohexylcarbodiimid oder N,N′-Carbonyldiimidazol oder mit Derivaten dieser Verbindungen zur Reaktion gebracht wird.

Der aktive Ester wird dann aus dem Reaktionsgemisch abgetrennt und zu BSA oder KLH zugegeben. Nach einer Inkubationszeit von 0.1 bis 12 Stunden vorzugsweise von 4 bis 5 Stunden wird das Präzipitat entfernt. Der Ueberstand kann dann gegebenenfalls nach Zwischenschaltung weiterer Reinigungsschritte für die eigentliche Immunisierungsreaktion verwendet werden.

Neben der im Rahmen dieser Erfindung bevorzugten aktiven Estermethode, können auch alternative Verfahren für die Kupplung des Haptens an das Carrier-Molekül verwendet werden, wie z.B. die gemischte Anhydridmethode. Dabei wird die Carboxylgruppe des Brückengliedes unter Verwendung von Essigsäureanhydrid oder des Carbodiimidderivates 1-Ethyl-3-(3′-dimethylaminopropyl)carbodiimid an das Carrier-Molekül geknüpft.

Erfolgt die Kopplung des Derivates über eine reaktive $NH_2$-Gruppe, so kömmt vorzugsweise das bei Kelly *et al* (1985) beschriebene Diazonium-Verfahren zur Anwendung.

Dabei wird die entsprechende reaktive $NH_2$-Gruppe vorzugsweise bei niedriger Temperatur, vorzugsweise bei einer Temperatru um 0°C, in stark saurer Lösung mit Natriumnitrit versetzt. Das resultierende Diazoniumsalz wird kann

langsam zu einer gepufferten, basichen Lösung des Carriermoleküls gegeben. Diese Lösung kann dann, nach Zwischenschaltung weiterer Reinigungsschritte für die eigentliche Immunisierung verwendet werden.

Die Immunisierung der Donortiere erfolgt durch eine ein- bis mehrmalige Applikation des an ein hochmolekulares Trägermolekül gekoppelten Haptens. Besonders bevorzugt ist eine 2 bis 3 malige Applikation, die in Abständen von 7 bis 30 Tagen insbesondere aber von 12 bis 16 Tagen erfolgt.

Die im Rahmen der vorliegenden Erfindung bevorzugte Applikationsform ist die Injektion, die sowohl intravenös, intraperitoneal oder subcutan erfolgen kann. Bevorzugt ist eine Kombination von subcutaner und intraperitonealer Injektion.

Das Antigen (Triasulfuron-konjugat) liegt in diesem Fall in einem geeigneten Puffer vor, wie z.B. einem PBS-Puffer, der eines der üblicherweise verwendeten Adjuvantien enthält.

Besonders bevorzugt im Rahmen dieser Erfindung ist die Verwendung von Freund's Adjuvans.

Nach einer Ruheperiode von 0,5 bis 4 Monaten erfolgt eine weitere einmalige Applikation des Haptenkonjugates in einer Dosierung von 10 µg bis 1000 µg, insbesondere aber von 50 µg bis 500 µg.

In einem Zeitraum von 1 bis 6 Tagen nach der letzten Dosierung werden die Donortiere getötet und es wird eine Milzzellensuspension hergestellt.

Die isolierten Milzzellen werden dabei in einem geeigneten Puffer (z.B. einem BSS-Puffer) suspendiert und bis zu ihrer Fusion mit geeigneten Myeloma-Zellen in Form einer Zellsuspension aufbewahrt.

Anfänglich wurden diese Fusionen dadurch kompliziert, dass auch die Myeloma-Zellinien monoklonale Antikörper synthetisierten, so dass das Hybrid zwei Typen monoklönaler Antikörper produzierte, einen, der seinen Ursprung in der Myeloma-Zelle hatte und einen zweiten, der durch die genetische Information der immunkompetenten Zelle bestimmt wurde.

Im Rahmen der vorliegenden Erfindung werden daher vorzugsweise solche Tumorzellen verwendet, die selbst keine monoklonale Antikörper herstellen können, wie z.B. SP2/0-Ag14 (Shulman *et al*, 1978), X63-Ag8.653 oder PAI [Stocker *et al* (1982)], wodurch die Analyse der resultierenden Fusionsprodukte sehr stark vereinfacht wird. Für den Fusionserfolg ist es vorteilhaft, wenn die Milzzellen in einem 2 bis 20fachen Ueberschuss im Verhältnis zu den Myelomzellen vorliegen.

Die Fusion von Milz- und Myelomzellen wird in einem speziellen Fusionsmedium durchgeführt, das eine Zusammensetzung aufweist, die für die beabsichtigte Zellfusion optimale Voraussetzungen liefert.

Vorzugsweise handelt es sich bei besagtem Fusionsmedium um eine Pufferlösung, die einen der üblicherweise für die Fusionierung von Zellen verwendeten Fusionspromotoren enthält, wie z.B. Sendai Viren oder andere Paramyxoviren, gegebenenfalls in UV-inaktivierter Form, Calcium-Ionen, oberflächenaktive Lipide, wie z.B. Lysolecithin oder Polyethylenglykol. Besonder bevorzugt im Rahmen dieser Erfindung ist die Verwendung von Polyethylenglykol (PEG), insbesondere von Polyethylenglykol (PEG) mit einem mittleren MG von 600 bis 6000, und in einer Konzentration von 30 % bis 60 %. Besonders bevorzugt ist PEG 4000 mit einer Konzentration von ca.50 %. Die optimale Fusionstemperatur beträgt zwischen 18°C und 39°C. Besonders bevorzugt ist eine Temperatur von 37°C.

Nach der erfolgten Fusion der immunkompetenten Milzzellen mit den Myeloma-Zellen werden die fusionierten Antikörper-produzierenden Hybridzellen mit Hilfe an sich bekannter Verfahren selektioniert.

Für das Auslesen erfolgreicher Fusionsereignisse (Hybridzellen) aus den 2 Typen von Elternzellen existieren verschiedene Möglichkeiten. Routinemässig werden eine Million und mehr Zellen von jedem Elterntyp in dem Fusionsprotokoll verwendet. Da die Fusion nicht mit einer 100 %igen Frequenz erfolgt, kann es zu einem schwierigen Unterfangen werden, die Fusionsprodukte von der grossen Ueberzahl nicht fusionierter oder mit sich selbst fusionierter Elternzellen abzutrennen.

Wie bereits zuvor erwähnt, entstehen die Hybridomazellen durch Fusion kurzlebiger Antikörper produzierender (Milz) B-Zellen sowie langlebiger Myeloma Zellen.

Das gewünschte Resultat ist eine langlebige Zellinie, die Antikörper produziert. Da die Milzzellen nur eine begrenzte Lebenszeit in Kultur besitzen, kann man daher im Prinzip einfach abwarten, bis alle nicht-fusionierten sowie alle mit sich selbst fusionierten Milzzellen abgestorben sind. Dann bleibt jedoch immer noch die Aufgabe bestehen, die langlebigen Antikörper-produzierenden Zellen von den langlebigen nicht Antikörperproduzierenden Zellen abzutrennen.

Ein gängiges Selektionssystem basiert auf der Verfügbarkeit oder Nichtverfügbarkeit des Enzyms Hypoxanthinguaninphosphoribosyltransferase (HGPRT). Dieses Enzym ist Bestandteil des Purin "Salvage Pathway" in Säugerzellen. Diese Zellen sind darüberhinaus auch in der Lage Purine *de novo* zu synthetisieren.

Unter normalen Umständen arbeiten wahrscheinlich beide Synthese-Wege bis zu einem gewissen Grade parallel.

Falls eine Zelle jedoch kein HGPRT besitzt, ist der "Salvage Pathway" blockiert und die Purine müssen aus nicht-Purin Material hergestellt werden.

Für die Selektion HGPRT-negativer Myelomazellen verwendet man in der Regel sog. Purin-Antimetaboliten, wie z.B. das 8-Azaguanin, das dem Purin Guanin strukturell sehr ähnlich ist und dieses daher in einigen seiner normalen Reaktionen ersetzen kann.

Azaguanin wird in die DNA eingebaut, was zu einer Beeinträchtigung des normalen Wachstumsverhaltens und

schliesslich zum Zelltod führt. Da Azaguanin über den "Salvage Pathway" ersetzt werden muss, können alle diejenigen Zellen, die keine HGPRT Aktivität besitzen, Azaguanin nicht verwerten und daher in dessen Gegenwart wachsen.

Ein selektives System, das mit demselben Enzym aber unter umgekehrten Vorzeichen arbeitet, indem in diesem Fall HGPRT positive Zellen selektiert werden, ist bei J.W. Littlefield (1964) beschrieben.

Dieses Selektionssystem basiert auf der Verwendung des sogenannten HAT-Mediums, das u.a. Hypoxanthin, Aminopterin und Thymidin (HAT-Medium) als Bestandteile enthält. Aminopterin ist ein Antimetabolit, der die *de novo* Purin-Synthese sowie die Methylierung von Desoxyuridylat zu Thymidylat hemmt.

Hypoxanthin kann als Hilfspurin fungieren für den Fall, dass Aminopterin die *de novo* Purin-Synthese blockiert, während Thymidin die Notwendigkeit einer Methylierung von Desoxyuridylat überflüssig macht.

Daher werden in Gegenwart von Aminopterin alle HGPRT positiven Zellen proliferieren, während die HGPRT negativen Zellen absterben.

In dem Hybridsystem, das im Rahmen dieser Erfindung für die Selektion verwendet wird, sind die Myeloma-Zellen vorzugsweise resistent gegenüber Azaguanin und empfindlich gegenüber Aminopterin, d.h. sie sind HGPRT negativ. Die Antikörper-produzierenden Zellen dagegen sind HGPRT positiv.

Durch Fusion der Zellen und Kultivierung in einem HAT-Medium, können die erfolgreich miteinander fusionierten Zellen selektiert werden, da die Myeloma-Zellen, die für die Proliferation verantwortlich sind, nur in Gegenwart einer HGPRT-Aktivität wachsen können und diese Aktivität von der HGPRT-positiven Zellinie geliefert werden muss.

Die HGPRT-positiven Antikörper-produzierenden Zellinien werden in diesem Medium zwar nicht abgetötet. Sie überleben aber nur eine bestimmte Zeit, und sind nicht in der Lage zu proliferieren.

Damit wird durch Fusion der Zellen in einem HAT-Medium ein System geschaffen, in welchem zwar die Myeloma Zellen und die Antikörper produzierenden Zellen für einen Zeitraum wachsen können, der ausreicht für die Produktion von Hybrid-Zellen, in dem aber nur die Hybrid-Zellen überleben und proliferieren können.

In einer besonderen Ausführungsform der vorliegenden Erfindung werden die fusionierten Hybridzellen in Gegenwart von zuvor aus dem Peritoneum unbehandelter, nicht-immunisierter Versuchstiere isolierten Macrophagen, sogenannter "Feederzellen", kultiviert. Für die Kultivierung und die Selektion der fusionierten Hybridzellen wird die Zellsuspension in mehrere Aliquots aufgeteilt und die einzelnen Aliquots werden ständig auf die Ausbildung von Hybridzellkulturen sowie auf die Bildung von Antikörpern hin untersucht.

Besonders bevorzugt im Rahmen dieser Erfindung ist die Kultivierung der fusionierten Hybridzellen auf Mikrotiterplatten.

Dabei wird die nach der Fusion erhaltene Zellsuspension auf die einzelnen Vertiefungen einer Mikrotiterplatte verteilt und für einen Zeitraum von 7 bis 30 Tagen unter geeigneten, das Wachstum der fusionierten Hybridzellen fördernden Bedingungen (z.B. HAT/HT-Medien) kultiviert.
Die Ueberstände gewachsener Hybridkulturen werden ständig auf die Bildung von Antikörpern hin untersucht.

Positive Hybridzellkulturen werden dann mit Hilfe bekannter Verfahren, vorzugsweise aber mit Hilfe des limitierenden Verdünnungsverfahrens vereinzelt und anschliessend in geeigneten Kultivierungsmedien kloniert.

Die Ueberstände der gewachsenen Zellklone werden ebenfalls auf die Bildung von Antikörpern hin überprüft.

Das Screening der gemäss der vorangegangenen Beschreibung hergestellten erfindungsgemässen Hybridoma-Zellklone auf die Bildung geeigneter monoklonaler Antikörper erfolgt vorzugsweise mit Hilfe eines der üblicherweise für diesen Zweck verwendeten Immunassays, wie z.B. einem Enzym-gekoppelten Immunassay oder einem Radioimmunassay.

Beim Enzym-gekoppelten Immunassay werden die zuvor näher charakterisierten Hapten-Konjugate zunächst an einen festen Träger adsorbiert. Die verbleibenden freien Bindungsstellen werden anschliessend durch Zugabe von Carriermolekülen abgesättigt und damit blockiert.

Zum Nachweis monoklonaler Antikörper werden Aliquots der Ueberstände besagter Hybridoma-Zellklone mit den Träger-gebundenen Haptenkonjugaten inkubiert.

Ein weiterer Gegenstand der vorliegenden Erfindung betrifft die Herstellung monoklonaler Antikörper unter Verwendung an sich bekannter Verfahren, die dadurch gekennzeichnet sind, dass man die zuvor näher charakterisierten erfindungsgemässen Hybridomazellinien oder aber Klone oder Subklone davon, welche die erfindungsgemässen Antikörper synthetisieren und vorzugsweise in das umgebende Medium sezernieren, *in vitro* oder *in vivo* mit Hilfe bekannter Verfahren kultiviert.

Die *in-vitro*-Kultivierung der erfindungsgemässen Hybridoma-Zellen erfolgt in geeigneten Kultivierungsmedien, insbesondere in den üblicherweise verwendeten, standardisierten Kulturmedien wie z.B. Dulbecco's Modified Eagle Medium (DMEM) oder RPMI 1640 Medium, die gegebenenfalls durch Zugabe von Säuger-Seren, wie z.B. Foetales Kälberserum, oder durch wachstumsfördernde Zusätze und Spurenelemente ergänzt werden können.

Die Isolierung der monoklonalen Antikörper beginnt vorzugsweise mit einer Präzipitation der Immunglobulinfraktion aus den jeweiligen Ueberständen der Hybridomakulturen, z.B. mit Hilfe von Ammoniumsulfat. Es schliessen sich weitere Aufarbeitungs- und Reinigungsschritte an, die dem Fachmann auf diesem Gebiet bekannt sind und die beispielsweise die Verwendung chromatographischer Methoden, wie z.B. Gelfiltration, Ionenaustausch-Chromatographie,

DEAE-Cellulose Chromatographie, Protein A oder Immunaffinitätschromatographie miteinschliessen.

Grosse Mengen der erfindungsgemässen monoklonalen Antikörper können aber auch mit Hilfe von *in vivo* Verfahren gewonnen werden.

So ist es beispielsweise möglich Antikörper-produzierende Hybridoma-Zellklone in geeignete Säugetiere zu injizieren, welche die Entwicklung von Antikörper-produzierenden Tumoren in den behandelten Tieren induzieren. Nach einen Zeitraum von 1 bis 3 Wochen können die Antikörper aus den Körperflüssigkeiten der so behandelten Tiere isoliert werden.

In einer besonderen Ausführungsform der vorliegenden Erfindung wird weiblichen Balb/c Mäusen, die gegebenenfalls mit einem Kohlenwasserstoff wie z.B. Pristan vorbehandelt wurden, ein erfindungsgemässer Hybridoma-Zellklon intraperitoneal injiziert.

Ein bis drei Wochen nach der Injektion des Hybridoma-Zellklons wird die Ascitesflüssigkeit gesammelt und bis zur weiteren Aufbereitung aufbewahrt.

Die Isolierung der monoklonalen Antikörper erfolgt in genau analoger Weise zu der zuvor beschriebenen Isolierung aus den Ueberständen *in vitro* kultivierter Hybridomas.

Die vorgehend für die Triasulfuron-Derivate beschriebenen Verfahrensmassnahmen können selbstverständlich in analoger Weise auch auf andere Sulfonylharnstoff-Derivate angewendet werden.

Ein weiterer Gegenstand der vorliegenden Erfindung betrifft die Verwendung der erfindungsgemässen Antikörper in einem der gebräuchlichen Immunassays zum Nachweis von Sulfonylharnstoff-Herbiziden, insbesondere aber zum Nachweis von Triasulfuron, sowie zur Differenzierung von Sulfonylhamstoff-Herbiziden insbesondere aber von Triasulfuron, gegenüber strukturell verwandten Verbindungen, vorzugsweise aber Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) in Boden-, Luft-, und Wasserproben sowie gegebenenfalls in Extrakten aus Pflanzen oder anderem biologischen Material.

Die erfindungsgemässen monoklonalen Antikörper können somit in allen bekannten Immunassays verwendet werden, die auf der spezifischen Bindung zwischen Antigen und dem entsprechenden monoklonalen Antikörper aufbauen, wie z.B. in einem Radioimmunassay (RIA), einem Enzym-gekoppelten Immunassay (ELISA), einem Immunfluoreszenz-Test etc.

Beim RIA Test kann der erfindungsgemässe monoklonale Antikörper als solcher oder aber in Form eines radioaktiv markierten Derivates verwendet werden. Dabei können alle bis heute bekannten Modifikationen des RIA Tests für den Nachweis der im Rahmen dieser Erfindung relevanten Zielsubstanzen verwendet werden, wie z.B. ein RIA Test in homogener oder fester Phase, ein heterogener RIA Test sowie ein einfacher ('Sandwich') RIA Test mit direktem oder indirektem (kompetitiven) Nachweis des Antigens. Das gleiche gilt auch für die Verwendung eines enzymgekoppelten Immunassays.

Bevorzugt im Rahmen dieser Erfindung ist die Verwendung eines erfindungsgemässen monoklonalen Antikörpers in einem kompetitiven Immunassay zum Nachweis von Triasulfuron

Das Prinzip des kompetitiven Immunassays beruht auf einer Konkurrenz zwischen einem markierten bzw. einem an einen festen Träger gebundenen Antigen und einem freien Antigen um die relevanten Bindungsstellen auf dem Antikörpermolekül.

Grundsätzlich unterscheidet man zwei Möglichkeiten zur Durchführung dieses kompetitiven Immunassays.

a) Das erste Verfahren beruht auf der Konkurrenz zwischen dem an einen festen Träger gebundenen Antigen und freiem Antigen um die freien Bindungsstellen auf dem Antikörper, der mit einem Marker versehen ist. Die Bindung des Antigens an einen festen Träger kann dabei entweder direkt oder aber über ein Carriermolekül erfolgen.

Die Bestimmung der Konzentration an freiem Antigen erfolgt in diesem Fall über die Abnahme des markierten, an das Träger-fixierte Antigen gebundenen Antikörpers. Diese Abnahme ist proportional der in der Probe enthaltenen Menge an freiem Antigen.

b) Ein alternatives Verfahren basiert darauf, dass freies und markiertes Antigen miteinander um die relevanten Bindungsstellen des Antikörpers konkurrieren, der in diesem Fall an einen festen Träger gebunden vorliegt.

Die Bestimmung der Konzentration an freiem Antigen erfolgt über die Abnahme an markiertem Antigen, die in Abhängigkeit von der Konzentration an freiem Antigen variiert.

Als festes Trägermaterial, das für die Bindung des Antigens oder des Antikörpers geeignet ist, kommen beispielsweise die Plastikoberfläche einer Mikrotiterplatte oder eines Teströhrchens, die Oberfläche von Kugeln aus Polystyrol, Polypropylen, Polyvinylchlorid, Glas oder Kunststoff oder aber die Oberfläche von Filterpapier-, Dextran-Cellulose- oder Nitrozellulose-Streifen oder ähnliche Materialien in Betracht. Diese werden mit einem der erfindungsgemässen monoklonalen Antikörper oder einem Antigen überzogen, wobei die Bindung an das Trägermaterial durch einfache Adsorption oder aber gegebenenfalls nach vorangegangener Aktivierung des Trägermaterials mit z.B. Glutaraldehyd oder Cyanogenbromid vermittelt werden kann.

Besonders bevorzugt im Rahmen dieser Erfindung ist die Verwendung des erfindungsgemässen monoklonalen

Antikörpers in einem Enzym-gekoppelten Immunassay [ELISA ('Enzyme Linked Immuno Sorbent Assay)]. Dabei kann der erfindungsgemässe monoklonale Antikörper als solcher oder in Form eines Enzymgekoppelten Derivates verwendet werden.

Der ELISA Assay basiert entweder auf der Verwendung eines Enzym-gekoppelten Derivates des erfindungsgemässen Antikörpers oder aber von an sich bekannten Enzym-gekoppelten Antikörpern, die ein Epitop eines erfindungsgemässen Antikörpers erkennen und daran binden.

Besonders bevorzugt im Rahmen dieser Erfindung ist die Verwendung eines ELISA-Assays, bei dem eines der zuvor beschriebenen Trägermaterialien zunächst mit einem Antigen beschichtet wird. Das trägergebundene Antigen wird anschliessend mit einer Testlösung inkubiert, die das nachzuweisende Antigen sowie einen der erfindungsgemässen Antikörper enthält. Das nachzuweisende Antigen kann dabei entweder in freier Form oder aber als Bestandteil einer Wasser- oder Bodenprobe vorliegen.

Nach einer Inkubationszeit von 10 Minuten bis 2 Stunden wird der gesamte Ansatz mit einem enzymmarkierten Antikörper inkubiert, der den erfindungsgemässen monoklonalen Antikörper erkennt und an diesen bindet. Ein Beispiel eines solchen enzymmarkierten Antikörpers ist ein Phosphatase-markiertes Ziegen anti-Schaf Immunglobulin, oder ein entsprechender Ziegen anti-Maus Antikörper, die kommerziell bezogen werden können.

Die Menge des gebundenen Antikörperproteins lässt sich anhand einer Enzym-Substrat-Reaktion, z.B. mit Hilfe spektroskopischer Verfahren bestimmen.

Ebenfalls bevorzugt im Rahmen dieser Erfindung ist ein ELISA-Test, der auf der Konkurrenz von markiertem sowie von freiem Antigen um den an eines der zuvor genannten Trägermaterialien gebundenen Antikörper beruht. Der Anteil an freiem Antigen, der in einer bestimmten Probe vorliegt, wird in diesem Fall über die Abnahme an markiertem Antigen bestimmt, die umso präziser ist, je mehr freies Antigen die Probe enthält.

Die vorliegende Erfindung betrifft weiterhin Mittel für die qualitative und quantitative Bestimmung von Sulfonylharnstoff-Herbiziden, insbesondere aber von Triasulfuron, in Form eines Test-Kits, die neben den erfindungsgemässen monoklonalen Antikörpern und/oder deren Derivaten, gegebenfalls auch noch andere monoklonale oder polyklonale Antikörper, insbesondere aber markierte monoklonale oder polyklonale Antikörper, sowie weitere Zusätze enthalten können.

Besonders bevorzugt im Rahmen dieser Erfindung sind Test-Kits, die auf einem der üblicherweise verwendeten Immunassays basieren, ausgewählt aus der Gruppe bestehend aus Radioimmunassay, Enzym-gekoppelter Immunassay und Chemilumineszenzassay. Ganz besonders bevorzugt sind Test-Kits, bei denen der Nachweis der Zielsubstanz auf einem kompetitiven Immunassay, insbesondere aber auf einem Enzym-gekoppelten Immunassay (ELISA) beruht.

Test-Kits für einen radioimmunologischen Nachweis von Triasulfuron, die im Rahmen dieser Erfindung bevorzugt sind, können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines radioaktiv markierten Derivates davon oder radioaktiv markiertes Antigen oder standardisierte Lösungen des Antigens;
(c) Pufferlösungen und
(d) gegebenenfalls Polypeptide, Detergentien und weitere Zusätze, die beispielsweise eine unspezifische Adsorption und Aggregatbildung verhindern sowie
(e) Pipetten, Reaktionsgefässe, Eichkurven, Beipackzettel etc.

Test-Kits für den immunologischen Nachweis von Triasulfuron, die auf einem Enzymgekoppelten Immunassy (ELISA) basieren, können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines zweiten Enzym-markierten monoklonalen oder polyklonalen Antikörpers, der gegen das zu bestimmende Antigen oder einen das Antigen erkennenden Antikörper gerichtet ist;
(c) Enzymsubstrate in fester oder gelöster Form;
(e) das Antigen oder standardisierte Lösungen des Antigens;
(f) Pufferlösungen;
(g) gegebenenfalls Polypeptide, Detergentien und weitere Zusätze, die beispielsweise eine unspezifische Adsorption und Aggregatbildung verhindern sowie
(h) Pipetten, Reaktionsgefässe, Eichkurven, Farbtafeln, Beipackzettel, etc.

Ein Test-Kit für den Nachweis von Triasulfuron, der auf einem Chemilumineszenztest basiert, kann beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und eines zweiten polyklonalen Antikörpers, der in der Lage ist, den erfindungsgemässen ersten Antikörper zu erkennen und der mit einem chemilumineszierenden Marker verknüpft ist;
(c) Lösungen enthaltend eine Komponente, die die Emission von Licht auslöst, wie z.B $H_2O_2$ und NaOH;
(d) Pufferlösungen;
(e) gegebenenfalls Polypeptide, Detergentien und weitere Zusätze, die eine unspezifische Adsorption und Aggregatbildung verhindern sowie
(f) Pipetten, Reaktionsgefässe, Beipackzettel, etc.

Trägermaterialien, die im Rahmen der vorliegenden Erfindung Verwendung finden können, umfassen in erster Linie unlösliche, polymere Materialien, ausgewählt aus der Gruppe bestehend aus Polystyrol, Polyethylen, Polypropylen, Polyester, Polyacrylnitril, Polyvinylchlorid, Polyacrylamid, Nitrocellulose, quervernetztes Dextran, fluorierte Harze, Agarose, quervernetzte Agarose, Polysaccharide, etc. Daneben sind aber auch andere Materialien denkbar, wie z.B. Glas, Metall, Netzgewebe auf Nylonbasis, etc.

Die zuvor im einzelnen genannten Trägermaterialien können sehr unterschiedlich ausgestaltet sein und in Abhängigkeit vom jeweils angestrebten spezifischen Verwendungszweck sehr verschiedenartige Formen aufweisen. Diese umfassen beispielsweise Schalen, Kugeln, Platten, Stäbchen, Zellen, Fläschchen, Röhrchen, Fasern, Netze, etc.

Häufige Verwendung bei der Herstellung von Test-Kits finden beispielsweise Mikrotiterplatten aus durchsichtigen Plastikmaterialien, wie z.B. Polyvinylchorid oder Polystyrol, die unbeschichtet oder aber mit einem der erfindungsgemässen Antikörper, mit freiem Antigen oder einem Antigenkonjugat beschichtet sein können. Ebenso verwendet werden Kügelchen, Röhrchen oder Stäbchen aus Polystyrol sowie Polystyrollatex, wobei das umgebende Latexmaterial via Zentrifugation von den Polystyrolpartikeln abgetrennt werden kann.

Einen weiteren Bestandteil des erfindungsgemässen Testkits bilden Marker oder Indikatoren, mit deren Hilfe es möglich ist, das Vorliegen einer Komplexbildungsreaktion, insbesondere aber einer Immunreaktion nachzuweisen, die vorzugsweise zu einem Antigen-Antikörper-Komplex oder aber zu einem Ligand-Rezeptor-Komplex führt, wobei neben qualitativen gegebenenfalls auch quantitative Aussagen über das nachzuweisende Antigen möglich sind. Als Marker oder Indikatoren kommen sowohl einzelne Atome als auch Moleküle in Betracht, die entweder direkt oder aber indirekt an der Erzeugung eines nachweisbaren Signals beteiligt sein können. Diese Marker oder Indikatoren können entweder direkt mit dem nachzuweisenden Antigen oder mit einem der erfindungsgemässen monoklonalen Antikörper verknüpft sein oder aber in diese eingebaut vorliegen. Sie können aber auch als Einzelsubstanzen oder als Bestandteil einer separaten Verbindung vorliegen, die weder das nachzuweisende Antigen selbst noch einer der erfindungsgemässen monoklonalen Antikörper ist, die ihrerseits aber in der Lage ist mit dem Rezeptormolekül zu reagieren, z.B. in Form einer Komplexbildung.

Bei diesen separat vorliegenden Verbindungen handelt es sich vorzugsweise um ein zweites Antikörpermolekül, das sowohl monoklonalen als auch polyklonalen Ursprungs sein kann, um ein Komplementprotein oder Fragmente davon, um *S.aureus* protein A, etc. Diese separaten Verbindungen erkennen und binden spezifisch an ein Rezeptormolekül, wie z.B. das nachzuweisende Antigen oder einen der erfindungsgemässen monoklonalen Antikörper, vorzugsweise aber an ein Rezeptormolekül, das in Form eines Komplexes vorliegt.

In vielen Fällen sind weitere, zusätzliche Reagentien notwendig, die dann erst im Zusammenwirken mit dem Marker zu einem nachweisbaren Signal führen. Dies gilt insbesondere dann, wenn Enzyme involviert sind.

Marker oder Indikatoren, die im Rahmen der vorliegenden Erfindung verwendet werden können, sind dem Fachmann auf dem Gebiet der Immunologie und Immunchemie bestens bekannt. Sie umfassen beispielsweise radioaktiv markierte Elemente oder Substanzen, Enzyme oder chemilumineszierende Substanzen. Die folgende Aufzählung möglicher Marker oder Indikatoren soll lediglich dazu diesen, die grosse Vielfalt der verwendbaren Substanzen und Reagentien beispielhaft zu veranschaulichen, ohne dadurch jedoch den Erfindungsgegenstand in irgendeiner Weise einzuschränken.

Geeignete Marker oder Indikatoren sind beispielsweise innerhalb der Gruppe der radioaktiven Elemente zu finden. Dabei sind insbesondere solche Elemente bevorzugt, die entweder selbst $\gamma$ Strahlen emittieren, wie z. B. $^{124}J$, $^{125}J$, $^{128}J$, $^{132}J$, $^{51}Cr$ oder aber eine Emittierung dieser Strahlen induzieren, wie z.B. $^{11}C$, $^{18}F$, $^{13}N$. Ebenfalls geeignet sind sog. $\beta$-Stahler wie $^{111}In$, $^{14}C$ und $^{3}H$.

Weitere geeignete Marker umfassen chemilumineszierende Substanzen, insbesondere aber fluoreszierende Substanzen, die sehr einfach auf chemischem Wege mit dem Antigen oder einem Antikörper verbunden werden können ohne diesen zu denaturieren. Das entstehende Fluorochrom lässt sich sehr einfach mit Hilfe fluorometrischer Verfahren

nachweisen. Im einzelnen zu nennen sind an dieser Stelle Fluorochrome ausgewählt aus der Gruppe bestehend aus Fluoresceinisocyanat, Fluoresceinisothiocyanat, 5-Dimethylamino-1 -naphthalinsulfonylchlorid, Tetramethylrhodaminisothiocyanat, Lissamin, Rhodamin 8200 Sulfonylchlorid, etc.

Weitere fluoreszierende Agentien sowie eine Beschreibung von Analysetechniken findet sich bei DeLuca, "Immunofluorescence Analysis", in: Antibody As a Tool, Marchalonis et al, John Wiley & Sons, Ltd., pp 189-231 (1982).

Besonders bevorzugt im Rahmen dieser Erfindung ist die Verwendung von Enzymen als Marker- oder Indikatorsubstanzen, wie z.B. Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase, Glucose-6-phosphatdehydrogenase, etc. Bei der Verwendung von Enzymen als Markersubstanzen ist es notwendig zusätzliche Reagentien zuzugeben, die es erlauben, die Bildung eines Immunkomplexes über die Enzymaktivität zu verfolgen sowie gegebenenfalls ein Stop-Reagenz, mit welchem die Enzymreaktion beendet werden kann.

Besonders bevorzugt sind dabei Reagentien, die zu einer Farbreaktion führen. Im Falle der Meerrettichperoxidase sei an dieser Stelle beispielhaft Hydrogenperoxid genannt, das in Kombination mit einem zusätzlichen, oxidierten Farbstoffprecursor wie z.B. Diaminobenzidin oder o-Phenylendiamin, zu einer Braun bzw. Gelbfärbung führt. Bei Verwendung der Glucoseoxidase als Markersubstanz kann beispielsweise 2,2'-Azino-di-(3-ethyl-benzthiazolin-6-sulfonsäure) [ABTS] als Substrat eingesetzt werden.

Ein weiterer Gegenstand der vorliegenden Erfindung betrifft somit die Verwendung von Test-Kits, die zumindest einen der erfindungsgemässen monoklonalen Antikörper als Reagenz enthalten, zum schnellen und effektiven, qualitativen und/oder quantitativen Nachweis von Sulfonylharnstoff-Herbiziden, insbesondere aber zum Nachweis von Triasulfuron, sowie zur Differenzierung von Sulfonylharnstoff-Herbiziden insbesondere aber von Triasulfuron, gegenüber strukturell verwandten Verbindungen, vorzugsweise aber Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) in Boden-, Luft-, und Wasserproben sowie gegebenenfalls in Extrakten aus Pflanzen oder anderem biologischen Material.

## I. NICHT-LIMITIERENDE AUSFÜHRUNGSBEISPIELE

### Beispiel 1: Synthese von Triasulfuron-Kupplungskomponenten

#### 1.1: Herstellung eines zur Kupplung an ein Trägerprotein befähigten Triasulfuron-Derivates

Die Herstellung der zur Kupplung an ein Trägerprotein befähigten Triasulfuron-Derivate kann gemäss dem nachfolgenden Reaktionsschema durchgeführt werden:

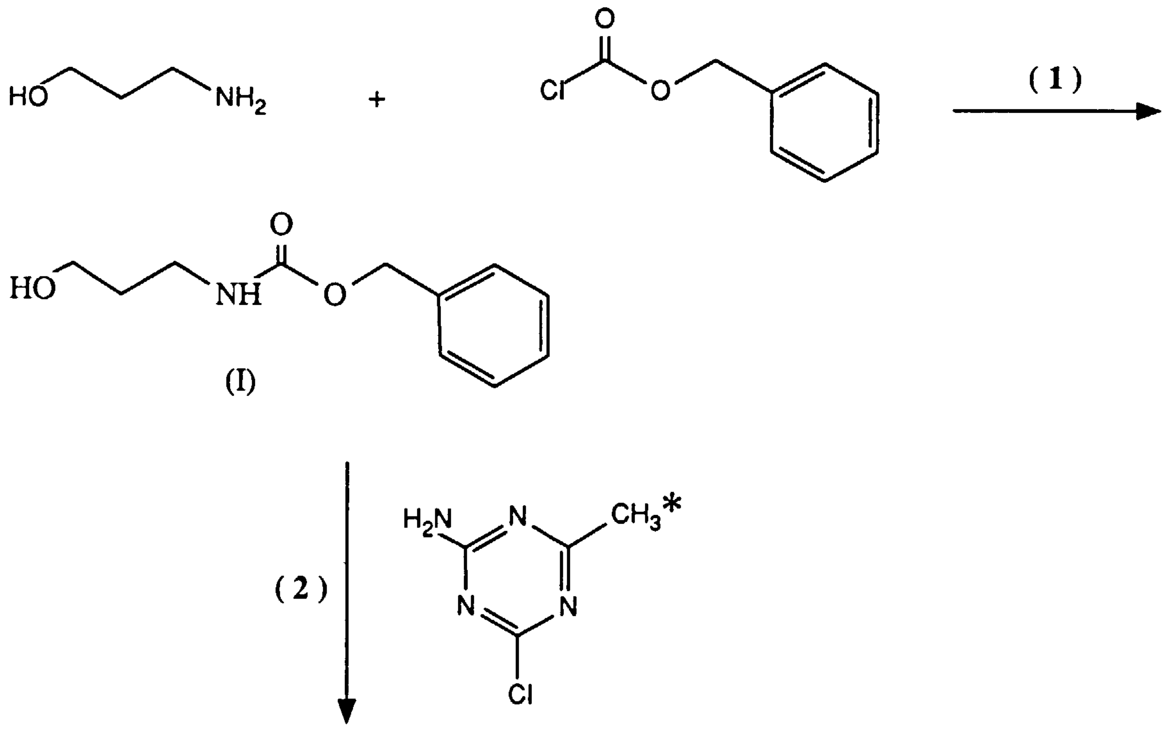


*hergestellt gemäss J Med Chem 12(1), 39-42, 1969

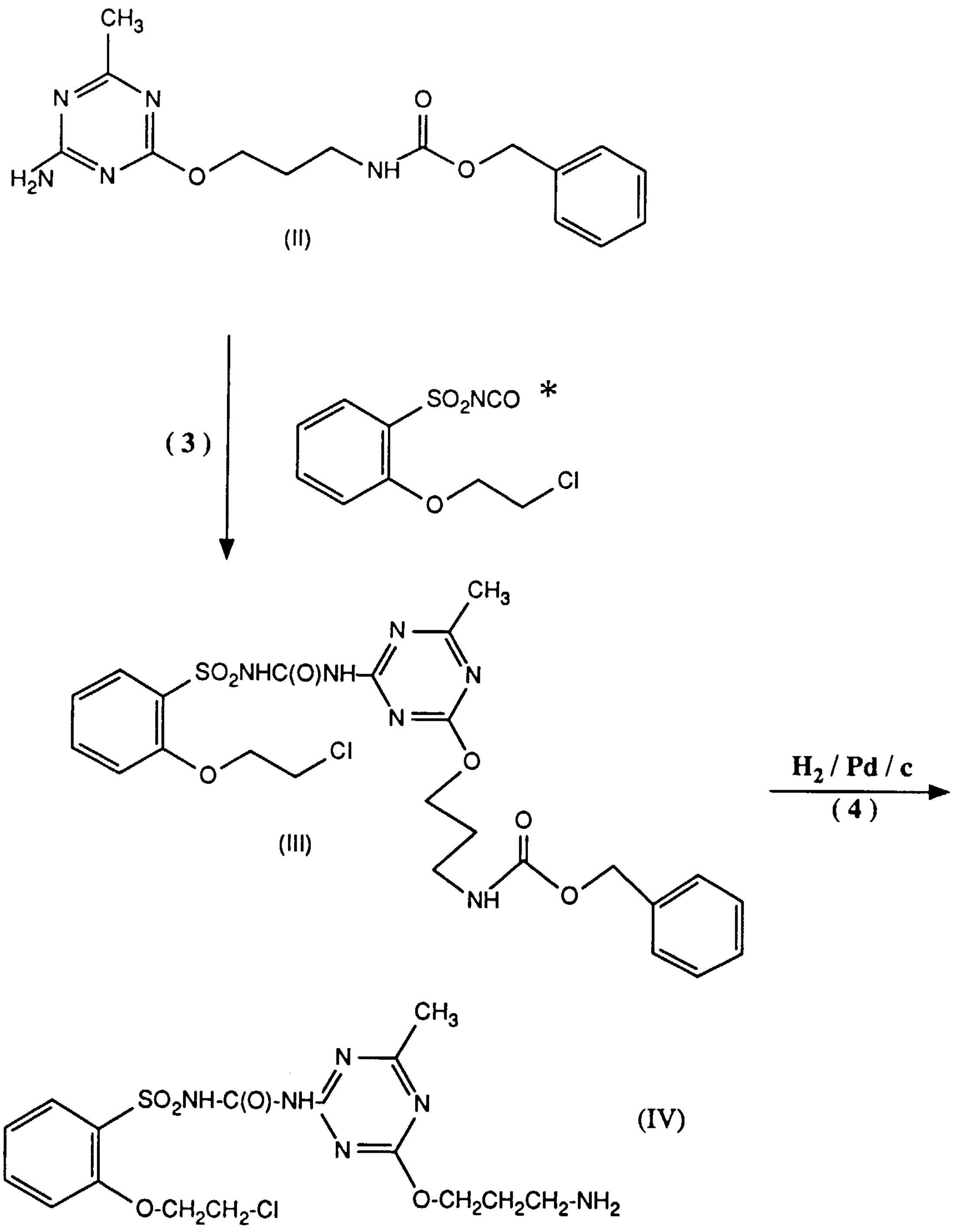


(II)

(III)

(IV)

*hergestellt aus dem Sulfonamid gemäss in EP-0,044,808 beschriebenen Verfahren.

Im einzelnen wird der oben dargestellte Vierstufenprozess folgendermassen durchgeführt:

Stufe 1: Herstellung von 2-Hydroxypropylcarbamoyloxymethylbenzol

22.5 g 3-Amino-1-propanol (Aldrich) und 30.3 g Triethylamin werden in 400 ml Methylenchlorid vorgelegt und bei 15°C bis 20°C unter leichter Kühlung innerhalb eines Zeitraumes von 30 Minuten mit 51.2 g 1-Chlorameisensäurebenzylester versetzt. Die so gewonnene Lösung wird noch 2 Stunden bei Raumtemperatur nachgerührt, 3 mal mit

deionisiertem Wasser gewaschen, mit $Na_2SO_4$ getrocknet und total eingeengt.

Das Rohprodukt wird anschliessend über eine Kieselgelsäule (300 g) chromatographisch gereinigt. Als Elutionsmittel dient ein 1:1 (v/v) Gemisch aus Toluol und Essigsäureethylester. Das Eluat wird aus Petrolether (Fraktion 30°C bis 70°C) auskristallisiert. Nach Abfiltrieren und Trocknen erhält man die Verbindung der Formel (I) in einer Ausbeute von 13 g (20.7% d.Th); Fp. 52°C-53°C.

Stufe 2:

Zu einer Suspension bestehend aus 4.12 g 2-Amino-4-chlor-6-methyltriazin, 3.9 g Kaliumcarbonat und 6.3 g einer Verbindung der Formel (I) [2-Hydroxypropylcarbamoyloxymethylbenzol] in 80 ml Aceton gibt man innerhalb einer Minute 1.85 g Trimethylamin, gelöst in 20 ml Aceton, zu. Das Gemisch wird noch 15 Stunden bei einer Temperatur von 40°C bis 45°C nachgerührt, bevor es bis zur Trockene eingedampft und in Essigsäureethylester aufgenommen wird. Nach zweimaligem Waschen mit deionisiertem Wasser wird die Essigesterphase über $Na_2SO_4$ getrocknet und auf ein Restvolumen von wenigen ml eingeengt. Die ausgefallenen Kristalle werden abfiltriert, gewaschen und getrocknet. Man erhält auf diese Weise die Verbindung der Formel (II) (weisse Kristalle) in einer Ausbeute von 3.1 g (34.2% d. Th.); Fp 126°C-128°C.

Stufe 3 und 4: Herstellung von (3-(6-(3-amino-n-propoxy)-4-methyl-1,3,5-triazin-2-yl)-1-[2-(2-chlorethoxy)-phenylsulfonyl]-harnstoff

2.75 g 2-(2-Chlorethoxy)-phenylsulfonylisocyanat und 3.35 g Aminotriazin der Formel (II) werden in 20 ml Dioxan (abs.) 5 Stunden bei einer Temperatur von 80°C bis 85°C gerührt. Die kare Lösung wird bis zur Trockene eindedampft und das Rohprodukt mit Toluol:Essigsäureethylester = 1:2 (v/v) über Kieselgel chromatrographiert.

Die Fraktionen, welche den Sulfonylharnstoff der Formel (III) enthalten werden vereinigt und direkt in 200 ml deionisiertem Wasser und 1.83 g $Na_2CO_3$ mit Wasserstoff hydriert, in Gegenwart von 0.5 g Pd/C 5%. Nach Abfiltrieren des Katalysators wird die farblose Lösung durch Zugabe von 2M(N) HCl auf pH 5.5 bis 6.0 angesäuert. Das auf diese Weise gebildete Präzipitat wird abfiltriert und getrocknet. Man erhält das Endprodukt der Formel (IV) in einer Ausbeute von 2.6 g; Fp 146°C-147°C.

Die in dem oben beschriebenen Vierstufenprozess verwendeten Ausgangsverbindungen und Reaktanten sind bekannt oder analog bekannter Verfahren herstellbar.

Beispiel 1.2 Herstellung eines zur Kupplung an ein Trägerprotein befähigten Triasulfuron-Fragmentes

Die Herstellung eines zweiten, zur Kupplung an ein Trägerprotein befähigten Triasulfuron-Fragmentes kann gemäss dem nachfolgenden Reaktionsschema durchgeführt werden:

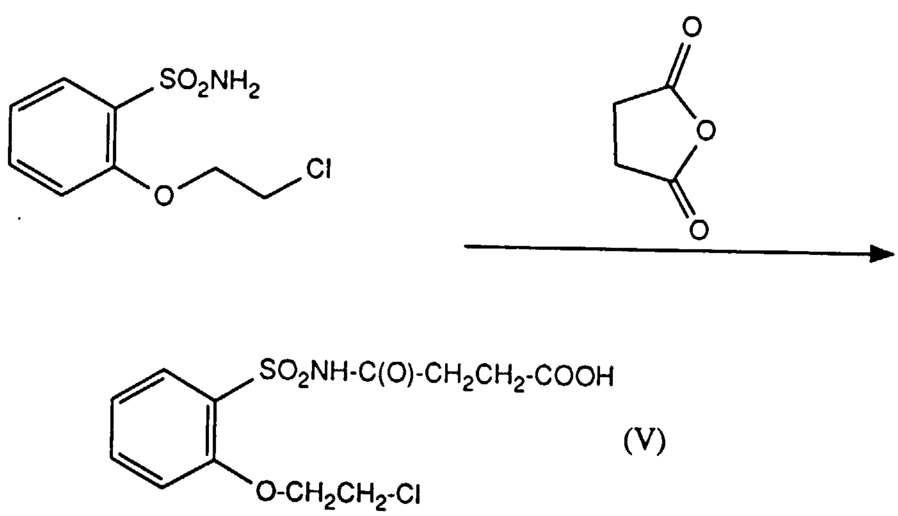

(V)

Im einzelnen werden die folgenden Verfahrensschritte durchgeführt.

Zu einer Lösung von 14.1 g 2-(2-Chlorethoxy)phenylsulfonamid und 6.0 g Bernsteinsäureanhydrid in 150 ml Dioxan werden unter leichtem Kühlen bei Raumtemperatur über einen Zeitraum von einer halben Stunde 18.5 g 1,8-Diazabicyclo[5.4.0]undec-7-en, gelöst in Dioxan, zugetropft.

Die resultierende weisse Suspension wird 2 Stunden bei Raumtemperatur weitergerührt, mit 65 ml 2M(N) HCl angesäuert und bis zur Trockene eingedampft. Der ölige Rückstand wird in Ethylacetat aufgenommen und 2 mal mit deionisiertem Wasser gewaschen. Nach Trocknen über $Na_2SO_4$ und erneutem Eindampfen erhält man durch Umkristallisieren aus 40 ml Ethanol 7.1 g (35.3% d.Th.) weisse Kristalle der Verbidnung der Formel (V) mit einem Fp von 166°C-168°C.

Beispiel 1.3: Triasulfuron-Protein Konjugat

Das gemäss Beispiel 1.3.1 hergestellte Triasulfuron-Derivat und das gemäss Beispiel 1.2 hergestellte Triasulfuron-Fragment wird entweder an "Bovine Serum Albumine" (BSA; Fluka) oder an "Keyhole Limpet Hemocyanin" (KLH; Calbiochem) konjugiert, unter Anwendung des bei Kelly *et al* (1985) beschriebenen Diazonium-Verfahrens bzw. der aktivierten Ester-Methode (Kulkarni *et al.,* 1981).

1.3.1: Kupplung der Verbindungen der Formel (IV) an BSH oderKLH nach dem-Diazonium-Verfahren

100 mg des Triasulfuron Derivates der Formel (IV) werden in einer Mischung aus Essigsäure (10 ml) und Propionsäure (5 ml) suspendiert. Das resultierende Gemisch wird auf 0°C abgekühlt und konzentrierte HCl (0.2 ml) sowie Natriumnitrit (0.05 g) werden zugegeben. Die Temperatur dieses Reaktionsansatzes wird für einen Zeitraum von 30 Minuten konstant bei 0°C gehalten. Die resultierende gelbe Lösung wird anschliessend in 2 Teile von jeweils 7.5 ml aufgeteilt, die langsam zu je 0.1 g BSH oder KLH in 20 ml 0.05 M Boratpuffer (pH 9.0) zugegeben werden. Der pH dieser Lösung wird durch Zugabe von 1 M NaOH auf einem pH-Wert von 9.0 konstant gehalten.
Bevor das Endprodukt dieser Reaktion für die Immunisierung verwendet wird, wird es ausgiebig gegen eine Phosphat-gepufferte Salzlösung [(PBS) 0.01 M Natriumphosphat und 0.145 M NaCl, pH 7.0] dialysiert.

1.3.2: Kupplung der Verbindung der Formel (V) an BSH oderKLH nach der aktivierten-Ester Methode

Im einzelnen wird dabei die Carboxylgruppe des Triasulfuron-Fragmentes der Formel (V) in N,N-Dimethylformamid (DMF) (7.2 mg/200 µl) bei Raumtemperatur solubilisiert und anschliessend mit einem 4 molaren Ueberschuss von α-Hydroxysuccinimid (9.1 mg/200 µl) sowie N,N'-Dicyclohexylcarbodiimid (16 mg/200 µl) versetzt. Das Reaktionsgemisch wird zunächst 1 Stunde bei 22°C und anschliessend 18 Stunden bein 4°C gerührt.

Das bei der Reaktion gebildete Präzipitat wird durch 3 minütige Zentrifugation mit 12'000 g bei Raumtemperatur entfernt und der aktivierte Ester anschliessend zu BSA [12 mg] oder KLH [12 mg] gegeben, das zuvor in 3.3 ml einer Phosphat-gepufferten Salzlösung (PBS-Puffer) solubilisiert worden ist.

Nach 4stündiger Inkubation bei einer Temperatur von 4°C wird das gebildete Präzipitat durch 10 minütige Zentrifugation mit 2'000 g bei 4°C entfernt und der verbleibende Ueberstand ausgiebig gegen PBS dialysiert, bevor er dann für die Immunisierungs-Experimente verwendet wird.

Das Ausmass der Kupplungsreaktion wird durch Absorptionsspektrophotometrie bestimmt. Das molare Verhältnis von Triasulfuron-Fragment zu BSA liegt bei ca. 10/1, vorzugsweise aber bei 12/1.

Beispiel 2: Immunisierung

2 Gruppen von jeweils 5 weiblichen, 4 bis 6 Wochen alten BALB/c Mäusen (Tierfarm Sisseln, Schweiz) erhalten in einem Abstand von jeweils 2 Wochen eine Serie von 3 intraperitonealen bzw. subkutanen Injektionen mit KLH-konjugiertem Triasulfuron. Die Dosierung für das in Beispiel 1.3.1 hergestellte Triasulfuron-Konjugat der Formel (IV) beträgt 80 µg/Injektion, während das in Beispiel 1.3.2 hergestellte Triasulfuron-Konjugat der Formel (V) in einer Dosierung von 60 µg/Injektion verabreicht wird.

Die erste Injektion beinhaltet 0,1 ml des Konjugates in PBS, das in einem Verhältnis von 1:1 mit 0,1 ml komplettem Freund Adjuvanz vermischt ist.
50 µl dieser Injektionslösung werden intraperitoneal injiziert, die restlichen 150 µl subcutan.

Bei der zweiten und dritten Injektionsserie, die 14 bzw. 30 Tage nach der Erstapplikation erfolgt, wird das komplette Freund Adjuvans durch inkomplettes ersetzt.

1 Woche nach der letzten Injektion wird Blutserum von den Versuchstieren entnommen und die Bluttiter mit Hilfe eines ELISA-Tests bestimmt, wobei die Mikrotiterplatten zuvor mit BSA-konjugiertem Hapten beschichtet werden (siehe Abschnitt 6).

Nach einer Ruheperiode von 2 Monaten erfolgt eine weitere einmalige intraperitoneale Injektion der KLH-Konjugate in einer Dosierung von 830 µg/200 µl PBS [Triasulfuron-Konjugat der Formel (IV)] bzw. 540 µg/200 µl PBS [Triasulfuron-Konjugat der Formel (V)]. Drei bis vier Tage später werden die Mäuse getötet und die aus diesen isolierten Milzzellen mit der Myelomazellinie PAI [Stocker *et al* (1982)] fusioniert [vergl. Beispiel 3.4].

Beispiel 3: Fusionsprotokoll

3.1. Gewinnung von "Feeder"-Zellen (peritoneale Makrophapen).

Unbehandelte, ca. 6 bis 8 Wochen alte Balb/c-Mäuse werden einen Tag vor der beabsichtigten Fusion getötet und durch Eintauchen in 70 %igem Alkohol sterilisiert.

Anschliessend wird das Fell und die obere Bauchhaut steril angeschnitten ohne das Peritoneum zu verleten. Mit einer sterilen 5 ml Plastikspritze und einer sterilen 18-er Injektionsnadel werden 4 ml BSS (ohne $Ca^{2+}$ und $Mg^{2+}$) und 1 ml Luft in die Bauchhöhle injiziert.

Nach leichtem Massieren des Bauches (wobei Spritze und Nadel in der Bauchhöhle verbleiben) wird der zuvor injizierte BSS-Puffer wieder aus dem Peritoneum abgezogen und in ein steriles Falconröhrchen gegeben. Dieses Procedere wird noch 2 mal wiederholt. Die so gewonnenen Makrophagen werden mit Eis gekühlt und anschliessend 2 x mit je 20 ml BSS gewaschen.

Die Makrophagen werden dabei je 10 Min. bei 300 g und einer Temperatur von 5°C zentrifugiert. Das Pellet wird dann in 50 ml HAT-Medium resuspendiert und die Zellsuspension auf 4 Costarplatten mit insgesamt 24 Vertiefungen verteilt (0,5 ml/Vertiefung).

Anschliessend werden die so vorbereiteten Makrophagen bei einer Temperatur von 37°C und einer $CO_2$-Konzentration von 6 % in einem Inkubator aufbewahrt.

Pro Fusionsvorgang werden ca. 4 x $10^6$ Makrophagen benötigt.

3.2. Anzucht der Myeloma-Zellinie PAI

Bei der genannten Myeloma-Zellinie PAI handelt es sich um eine Myeloma-Zellinie, die selbst keine Antikörper sezerniert und die bei Stocker *et al.* (1982) beschrieben ist.

Pro Fusion benötigt man 50 ml einer gut gewachsenen Kultur, die mindestens 10 Millionen Zellen enthält. Die Kultivierung der Myelomzellen erfolgt vorzugsweise in T 175 "Falcon"-Flaschen (Fa. Beckton & Dickenson).

Einen Tag vor der Fusion wird das Kultivierungsmedium (RPMI 1640) durch frisches RPMI 1640-Medium ersetzt. Am Tag der Fusion werden die PAI Zellen geerntet, in ein steriles 50 ml Plastikröhrchen gegeben und 10 Min. bei 300 g und einer Temperatur von 5°C zentrifugiert (MSE Zentrifuge, Modell Chilspin, UK). Nach der Zentrifugation wird der Ueberstand abgezogen und verworfen. Die Zellen werden 2 x mit je ca. 30 ml BSS-Puffer ($Ca^{2+}$ und $Mg^{2+}$ frei) gewaschen (10 Min. bei 300 g, 5°C) und anschliessend in 5 ml BSS resuspendiert.

Ein Aliquot der Zellsuspension wird zur Bestimmung der Zellzahl entnommen und mit Fluoresceindiacetat (FDA) gefärbt. Bis zur Weiterverwendung werden die Myelomzellen auf Eis aufbewahrt.

3.3. Herstellung einer Milzzellsuspension

Die Entnahme der Milz aus einer zuvor gemäss Beispiel 2 immunisierten Balb/c-Maus erfolgt unter sterilen Bedingungen und unter Kühlung mit Eis.

Die zuvor immunisierte Balb/c-Maus wird durch Genickbruch getötet und die Milz steril entnommen. Dazu wird die Maus kurz in 70 %igen Ethanol eingetaucht und mit sterilem Besteck präpariert. Die Milz wird vorsichtig entnommen und auf ein feines Nylonnetz gelegt. Dort wird sie mit einer Schere fein zerschnitten und dann mit Hilfe eines 5 ml Spritzenstempels vorsichtig durch das Netz gedrückt, ohne dabei zu viele Zellen zu zerstören. Während des ganzen Vorgangs wird das Netz mit BSS gespült.

Die so gewonnene Zellsuspension wird in 50 ml Plastikröhrchen gegeben und 10 Min. bei 300 g und einer Temperatur von 5°C zentrifugiert (MSE Zentrifuge, Modell Chilspin; UK). Die Zellen werden anschliessend 2 x mit je 20 ml BSS gewaschen (10 Min.; 300 g; 5°C; MES Chilspin) und das Zellpellet wird nach der Zentrifugation in 10 ml BSS resuspendiert.

Bis zur Fusion mit PAI Myelomzellen werden die Milzzellen auf Eis belassen.

3.4. Fusion: Milzzellen und PAI Myelomzellen

Das Verhältnis von Myelomzellen zu Milzzellen sollte für die Fusion 1:10 betragen.

Milzzellen (in BSS-Puffer) und PAI Myelomzellen (in BSS-Puffer) werden in dem angegebenen Verhältnis zusammengegeben und 10 Min. bei 300 g und einer Temperatur von 5°C zentrifugiert (MSE Zentrifuge, Modell Chilspin). Das Pellet wird nochmals in BSS-Puffer resuspendiert und die Suspension anschliessend erneut zentrifugiert. Das Pellet wird vorsichtig aufgerührt und in 37°C warmes Wasserbad gestellt. Es wird dann 1 ml vorgewärmtes und steriles PEG-4000 (MERCK) über einen Zeitraum von 60 Sek. tropfenweise zu den Zellen zugegeben, wobei der gesamte Ansatz ständig bewegt wird. Die Zellen werden anschliessend noch 30 Sek. lang weitergeschüttelt, bevor 5 ml einer

zuvor erwärmten BSS-Puffers (ohne $Ca^{2+}$, $Mg^{2+}$) ebenfalls tropfenweise über einen Zeitraum von ca. 5 Min. unter ständigem Rühren dazugegeben werden.

Die auf die beschriebene Weise fusionierten Zellen werden dann abzentrifugiert (10 Min.; 300 g; 20°C, MSE Zentrifuge, Modell Chilspin), der Ueberstand wird abgezogen und verworfen. Das Zellpellet wird in 50 ml HAT-Medium resuspendiert und die so erhaltene Zellsuspension auf die vorbereiteten 4 Costarplatten (Mikrotiterplatten mit 24 Vertiefungen, Durchmesser pro Vertiefung 24 mm; Gesamtfläche für Zellwachstum 2,0 $cm^2$) verteilt (0,5 ml/Vertiefung).

Die Inkubation der Costarplaten erfolgt bei einer Temperatur von 37°C und bei einer $CO_2$-Konzentration von 6 %.

Beispiel 4: Kultivierung der Hybridzellen

Am 1. Tag nach der Zellfusion wird 1 ml HAT-Medium pro Vertiefung zu den Kulturplatten zugegeben. 3 bis 4 Tage nach der Zellfusion erfolgt eine mikroskopische Kontrolle der fusionierten Zellen. Gleichzeitig wird das verbrauchte Medium abgesaugt und durch 1 ml frisches HAT-Medium ersetzt. Nach weiteren 3 Tagen (6 - 7 Tage nach der Zellfusion) erfolgt ein erneuter Wechsel des Kulturmediums. Ab dem 7. bis 10. Tag nach der Zellfusion wird jede Vertiefung mikroskopisch nach Hybriden abgesucht und 2 bis 3x wöchentlich das Medium erneuert.

Sobald in einer Vertiefung Hybride gewachsen sind, gewöhnlich nach 2 bis 4 Wochen, kann das HAT Medium dort durch HT-Medium ersetzt werden. Der Ueberstand von gewachsenen Hybridkulturen (mindestens 10 % der Vertiefung) wird mit einer sterilen Pasteurpipette abgehoben und auf das Vorhandensein von Antikörpern getestet.

Sobald die Vertiefung mit positiven Hybridkolonien voll bewachsen sind, können diese auf neue Costarplatten in RPMI 1640-Medium transferriert werden, wobei der Inhalt einer vollbewachsenen Vertiefung auf 2 bis 3 neue Vertiefungen verteilt wird.

Beispiel 5: Klonierung der positiven Hybridzellen

Die Zellen einer positiven Vertiefung werden mit Hilfe einer Pipette gelöst und in 1 ml Medium in ein Röhrchen überführt. Anschliessend wird ein Aliquot zur Bestimmung der Zellzahl entnommen und mit FDA angefärbt (Verdünnung 1 : 2 mit FDA: 50 µl Zellen + 50 µl Farbstoff). Die bevorzugte Zellzahl liegt bei $10^5$ bis $10^6$ Zellen/ml. Anschliessend werden die Hybridzellen in einem Verhältnis von 1 : 100 mit HT-Medium verdünnt (z.B. 100 µl Zellen + 9,9 ml HT-Medium).

In zwei 50 ml Falconröhrchen werden je 25 ml HT-Medium vorgelegt und mit 5 ml einer Macrophagensuspension auf insgesamt 30 ml pro Röhrchen aufgefüllt. Die Macrophagen werden zuvor aus einer Maus isoliert und in 10 ml HT-Medium resuspendiert (vgl. Abschnitt 3.1).

In diesen die Macrophagen enthaltenden Falcon-Röhrchen werden die Hybridzellen verdünnt, bis eine Zelldichte von (i) 270 Zellen/30 ml bzw. (ii) 90 Zellen/30 ml erreicht ist. Anschliessend werden diese Ansätze auf Costarplatten (Mikrotiterplatten mit 96 Vertiefungen) verteilt, wobei je 200 µl pro Vertiefung zugegeben werden. Dies entspricht einer Zellzahl von (i) 1,8 Zellen/Vertiefung bzw. (ii) 0,6 Zellen/Vertiefung. Pro Verdünnung benötigt man auf diese Weise 1,5 Mikrotiterplatten.

Nach 7 Tagen werden die einzelnen Vertiefungen mikroskopisch kontrolliert und die Vertiefungen die Zellklone enthalten erfasst. Diejenige Verdünnung, bei der ca. 50 % der Vertiefungen Zellklone enthalten wird für den ELISA-Test verwendet. Dies sollte in der Regel die Verdünnung mit 0,6 Zellen/Vertiefung sein. Nach ca. 7 - 10 Tagen werden die Ueberstände der positiven Vertiefungen (mit Klonen) in einem ELISA-Test auf die Anwesenheit von monoklonalen Antikörpern getestet und die positiven Klone auf Costarplatten (mit 24 Bohrungen) in RPMI 1640-Medium vermehrt. Aliquots dieser positiven Klone werden in flüssigem Stickstoff aufbewahrt.

Beispiel 6: Hybridoma-Screening (ELISA-Test)

Zunächst werden 100 µl einer Lösung von BSA-konjugiertem Hapten [2 µg/ml] in Natriumcarbonat-Puffer (50 mM, pH 9.6) in die einzelnen Vertiefungen einer Mikrotiterplatte gegeben und dieser Ansatz bei 4°C in einer feuchten Kammer über Nacht inkubiert. Anschliessend werden die Vertiefungen jeweils 5 x mit einem 0,1 % PBS-Tween Puffer gewaschen. Zur Blockierung der unbesetzten Bindungsstellen auf der Mikrotiterplatte werden 200 µl einer PBS-BSA Lösung (1 %) in jede Vertiefung gegeben. Dieser Ansatz wird 1 - 2 Stunden bei Raumtemperatur inkubiert und anschliessend mit einem 0,1 %igen PBS-Tween-Puffer gewaschen.

Man gibt dann zu jeder Vertiefung 200 µl des Hybridoma-Überstands, der im Verhältnis 1:2 mit PBS-Tween (0,1 %) verdünnt ist, hinzu und inkubiert den gesamten Ansatz für 2 Stunden bei Raumtemperatur. Anschliessend werden die Vertiefungen erneut 5 x mit einem 0,1 %igen PBS-Tween-Puffer gewaschen.

Es folgt die Inkubation mit Phosphatase-konjugiertem Ziegen anti-Maus Antikörper (Kirkegaard & Perry Lab.). Zunächst werden 100 µl eines über eine Affinitätschromatographie gereinigten Ziegen Antikörpers gegen Mäuse IgG, der 1 : 1500 in PBS-Tween (0,1 %) verdünnt vorliegt (Kirkegaard & Perry Laboratories) und mit alkalischer Phosphatase

markiert ist, zu jeder Vertiefung hinzugegeben.

Die Inkubationszeit beträgt 1,5 Stunden bei Raumtemperatur. Anschliessend werden die einzelnen Vertiefungen erneut mit PBS-Tween (0,1 %) gewaschen (5 x).

Danach werden 150 µl einer substrathaltigen Lösung (1 mg/ml p-Nitrophenylphosphat) in jede Vertiefung zugegeben. Nach einer Inkubationszeit von 2 Stunden im Dunkeln erfolgt die spektroskopische Bestimmung bei 405 nm. Positive Hybridoma-Zellen, die einen spezifischen Antikörper sezernieren geben ein starkes positives Signal bei der gewählten Wellenlänge. Diese werden dann mit Hilfe des limitierenden Verdünnungsverfahrens [Godings (1980)]kloniert. Reine monoklonale Antikörper können aus der Ascitesflüssigkeit entsprechend vorbehandelter Mäuse gewonnen werden [Campbell AM, (1984)]

Beispiel 7: Expansion der Hybridomazellen in der Maus

Für die Anregung der Ascites-Produktion werden weibliche Balb/c Mäuse (20 - 25 mg) (Tierfarm Sisseln, CH) mit 0.3 ml Pristan Oel (Aldrich Chemical) vorbehandelt, das intraperitoneal injiziert wird. 1 bis 3 Wochen nach der Pristan Applikation erhalten die Mäuse eine zweite Injektion (0,2 ml Pristan-Oel, i.p.). Gleichzeitig mit dieser 2. Injektion erhalten die Tiere 2 x $10^6$ Hybridomazellen in 0,2 ml PBS.

Die aus dieser Behandlung resultierende Ascitesflüssigkeit wird gesammelt, bei 800 g zentrifugiert und bei einer Temperatur von -20°C aufbewahrt. Nach dem Auftauen wird die Ascitesflüssigkeit 1 Stunde bei 30'000 g zentrifugiert. Die oberste Schicht, die vorwiegend Lipide enthält, wird entfernt. Anschliessend wird die Proteinkonzentration bestimmt und auf einen Wert von 10 mg/ml eingestellt durch Zugabe von PBS.

Die Immunglobulin G Fraktion (IgG) wird durch tropfenweise Zugabe von 0,9 Volumenteilen einer gesättigten Ammoniumsulfatlösung bei 0°C präzipitiert. Nach 1 Stunde wird die IgG-Fraktion pelletiert durch einstündige Zentrifugation bei 22'000 g. Das Pellet wird anschliessend in 20 mM-Tris-HCl Puffer, pH 7,9, der 50 mM NaCl enthält, aufgelöst und gegen den gleichen Puffer über Nacht bei 4°C dialysiert. Die weitere Aufarbeitung der IgG Fraktion geschieht mit Hilfe einer Anionen-Austauschchromatographie an einer DE-52 Diethylaminoethylcellulose (Whatman) Säule. Die Probe wird 1:2 (v/v) mit 20 mM Tris-HCl, pH 7,9 verdünnt, bis eine NaCl Endkonzentration von 25 mM erreicht ist und 10 mg Protein/ml Gel werden auf die Säule aufgetragen. Die Elution wird erreicht durch Erhöhung der NaCl-Konzentration von 25 mM auf 200 mM (linearer Gradient). Im allgemeinen erfolgt die Elution monoklonaler Antikörper im Bereich von 80 mM NaCl.

Die Fraktionen werden über Nacht bei einer Temperatur von 4°C gegen PBS dialysiert und bei -70°C aufbewahrt. Der Reinheitsgrad wird mit Hilfe einer Natriumdodecylsulphat Polyacrylamid-Gelelectrophorese (SDS-PAGE) bestimmt, sowie durch isoelektrische Fokussierung.

Im vorliegenden Fall liegt der Reinheitsgrad bei >90 %.

Beispiel 8: Triasulfuron-Nachweis

Der Nachweis von Triasulfuron erfolgt mit Hilfe eines zweistufigen, kompetitiven ELISA-Tests.

BSA-konjugiertes Hapten, hergestellt gemäss Beispiel 1.3.1 bzw. 1.3.2 wird zunächst in einem 50 mM Natriumcarbonatpuffer (pH 9,6) (200 ng BSA-konjugiertes Hapten/100 µl Natriumcarbonat-Puffer) an Mikrotiterplatten (z.B. Dynatech, Typ M 129A) adsorbiert und über Nacht bei einer Temperatur von 4°C inkubiert.

Die Platten werden anschliessend 5 x mit PBS-Puffer gewaschen, der mit 0,1 % (v/v) Polysorbat 20 (Tween 20) angereichert ist (PBS-Tween).

Die restlichen freien Bindungsstellen des festen Trägermaterials werden dann durch Zugabe von PBS-BSA in Form einer 1 % (w/v) Lösung blockiert. Nach zweistündiger Inkubation bei 22°C werden die Platten erneut mit PBS-Tween (0.1 %) gewaschen.

50 µl des Überstandes der zuvor klonierten Hybridomazellen (in einer Verdünnung von 1:2000 oder 1:4000) oder aber 50 µl der zuvor gereinigten monoklonalen Antikörper (40 - 120 ng/ml) werden a) mit 950 µl einer Standardlösung, die einen steigenden Anteil an Triasulfuron bzw. Triasulfuron-Analogen enthält, b) mit Triasulfuron-haltigen Wasserproben oder c) Triasulfuron-haltigen Bodenextrakten inkubiert. [Alle Verdünnungen werden in PBS-Tween (0.1 %) vorgenommen].

Nach einer Inkubationszeit von 1 Stunde bei Raumtemperatur (22° C) werden 200 µl des Antigen/Antikörper Gemisches zu jeder Vertiefung der Mikrotiterplatte zugegeben und der gesamte Ansatz wird für eine weitere Stunde inkubiert. Die Vertiefungen werden anschliessend 5 x mit PBS-Tween (0.1 %) gewaschen und mit 100 µl/Vertiefung Ziegen-anti-Maus IgG Antikörper, der konjugiert an alkalische Phosphatase vorliegt (Verdünnung 1:1500), beschickt und für einen Zeitraum von 1,5 Stunden inkubiert.

Nach erneutem Waschen werden 150 µl/Vertiefung des in 1 mg/ml Diethanolaminpuffer (1 mM, pH 9,8, angereichert mit 0,5 mM $MgCl_2$ x $6H_2O$) gelösten Substrates p-Nitrophenylphosphat zu den Vertiefungen zugegeben.
Nach einer Inkubationszeit von 2 Stunden bei einer Temperatur von 22° C lässt sich ein Farbumschlag beobachten,

der proportional ist zur Menge an Antikörper, der mit dem an die Festphase gebundenen Antigen reagiert hat. Die Intensität der eingetretenen Farbreaktion wird bei einer Wellenlänge von 405 nm bestimmt. Die Verdünnungen der einzelnen Proben werden so gewählt, dass man ohne Zugabe eines Inhibitors (Bo) Absorptionswerte in einem Bereich zwischen 0.3 und 0.5 erhält. Für die Kontrollen (ohne Antikörper) ergeben sich Werte von $A_{405} \leq 0.01$. Alle Proben werden in dreifacher Ausfertigung bestimmt.

Zur Ermittlung der in einer Probe enthaltenen Menge an Triasulfuron wird zunächst eine Eichkurve erstellt (Abb. 1), wobei B/Bo x 100 gegen die Konzentration an Inhibitor aufgetragen wird. (Bo bedeutet Absorptionsfähigkeit gemessen ohne Zugabe eines Triasulfuronininhibitors zum Antikörper, und B Absorptionsfähigkeit bei Zugabe verschieden konzentrierter Triasulfuron-Inhibitoren) Der $I_{50}$-Wert gibt diejenige Konzentration des Antigens an, bei der die Antikörperbindung an die Festphase zu 50 % gehemmt wird. Der $I_{50}$-Wert wird mit Hilfe eines auf die vorliegenden Verhältnisse speziell angepassten ENZFITTER (Leatherbarrow, Elsevier-Biosoft) Kurvenkalkulations-programms bestimmt, basierend auf einer 4 Parameter umfassenden logistischen Kurve (Raab GM, 1983). Auch die quantitative Triasulfuronbestimmung in Boden- oder Wasserproben im Rahmen des ELISA wird mit Hilfe des ENZFITTER-Programms durchgeführt, wobei die Anpassung der Kurve auf Standards basiert, die auf jeder Mikrotiterplatte mitlaufen.

Beispiel 8.1: Analyse von Bodenproben

Aliquots (2 g) standardisierter Bodenproben unterschiedlicher Herkunft werden gemäss der im folgenden beschriebenen Verfahren extrahiert:

Methode (A) [nach Iwanzik und Egli (1989)]: Ein 100 g Probe wird 2 Stunden in einem Extraktor durch Ausschütteln mit 300 ml eines 2:1 Gemisches (v/v) aus Methanol und einem wässrigen Phosphatpuffer (PB) [pH 7.0, Gesamt-Phosphatkonzentration 0.07 M] extrahiert. Nach Filtration und Ansäuern mit Phosphorsäure wird Triasulfuron 3 x mit 75 ml $CH_2Cl_2$ re-extrahiert. Nach Abdampfen des Lösungsmittels wird die Probe in 10 ml PB-Puffer gelöst und über eine Filtration gereinigt.

Methode (B): Die Extraktion nach Methode (A) wird mit zwei weiteren Proben wiederholt und die letzte organische Phase durch Ausschütteln mit einer wässrigen Hydrogencarbonat-Lösung (5 %) weiter gereinigt [Iwanzik und Egli ( 1989)]. Nach Zugabe von Tetrabuthylammoniumhydrogencarbonat wird Triasulfuron in Dichlormethan-n-hexan (80:20) re-extrahiert. Nach Abdampfen der organischen Phase wird Triasulfuron in 10 ml PB-puffer aufgenommen. Bevor die Probe im ELISA Test verwendet wird, wird sie in PBS-Tween 0.1 % im Verhältnis 1:20 oder 1:40 verdünnt.

Methode (C): In diesem Fall erfolgt das Ausschütteln mit Tetrabythylammoniumhydroxid direkt mit dem Methanol/PB Extrakt. Die wässrige Phase wird anschliessend auf ein flüssig-flüssig 'Partitioning Cartridge' [ClinElut® #1010, Analytichem International, Harbor City, CA] überführt und mit 30 ml n-Hexan gewaschen. Das Triasulfuron wird mit Dichlormethan/n-Hexan (60:40) eluiert. Die organische Phase wird verdampft und der zurückbleibende Rest in PBS aufgenommen.

Beispiel 8.2: Analyse von Wasserproben

Für den kompetitiven ELISA werden 100 µl eines 10fach konzentrierten PBS-Tween Puffers zu 850 µl einer Wasserprobe zugegeben. Dieser Ansatz wird schliesslich mit 50 µl des anti-Triasulfuron Antikörpers inkubiert.

II. Ergebnisse

1) Herstellung monokonaler Antikörper

(a) Ausgehend von 5 Fusionsereignissen unter Verwendung von Mäusen, die mit dem gemäss Beispiel 1.3.1 hergestellten KLH-Konjugat [Triasulfuron-Konjugat der Formel (IV)] immunisiert wurden, erhält man ein Hybridoma, welches eine monoklonalen Antikörper mit hoher Affinität gegenüber Triasulfuron produziert [MAb 4134-40-1]. Die Fusionseffizienz liegt bei ca. 82 %.

(b) Ausgehend von 5 Fusionsereignissen unter Verwendung von Mäusen, die mit dem in Beispiel 1.3.2 hergestellten KLH-Konjugat [Triasulfuron-Konjugat der Formel (V)] immunisiert wurden, erhält man 19 Hybridomas, welche eine monoklonalen Antikörper mit sehr hoher Affinität gegenüber Triasulfuron produzieren. Basierend auf ihren Kreuzreaktivitätsmustern lassen sich 2 Gruppen monklonaler Antikörper unterscheiden, die durch MAb 4147-19-4

und MAb 4149-1-1 repräsentiert werden. Beide MAbs gehören zum IgG1 Isotyp.

Die Kreuzreaktivitätsmuster dieser beiden MAbs ist sehr unterschiedlich, wie die Tabelle 1 zeigt. Im Gegensatz zu MAb 4147-19-4 zeigt MAb 4149-1-1 eine ausgeprägte Kreuzreaktivität mit den hydroxylierten Triasulfuronen.

MAb 4147-19-4 dagegen zeigt mit einigen anderen Triasulfuron-Analogen Kreuzreaktivitäten, insbesondere mit Cinosulfuron. Im letzten Fall liegt die Kreuzreaktivität bei 150%.

Die untere Nachweisgrenze für Triasulfuron unter Verwendung der erfindungsgemässen MAbs (in Puffer) liegt im Bereich von 0,01 bis 1 ng/ml Puffer. Der entsprechenden $I_{50}$-Wert beträgt 0.09 ng/ml für MAb 4147- 19-4 bzw. 0.05 ng/ml für MAb 4149-1-1.

Analyse von extrahierten Bodenproben

Triasulfuron wird in unterschiedlichen Konzentrationen Extrakten von 5 verschiedenen standardisierten Bodenproben bekannter Zusammensetzung zugegeben und anschliessen mit Hilfe eines ELISA unter Verwendung von MAb 4147- 19-4 und MAb 4149-1-1 bestimmt. Die Ergebnisse sind in den Tabellen 2, 3 und 4 wiedergegeben.

Die Ergebnisse zeigen, dass die erfindungsgemässen MAbs in hervorragender Weise geeignet sind für den Nachweis von Triasulfuron in Bodenproben unter Verwendung eines ELISA Assays. Die unter Nachweisegrenze liegt bei etwa 0.1 ppb.

## II. HINTERLEGUNG

Die im Rahmen der vorliegenden Erfindung hergestellten und verwendeten Hybridoma-Zellinien wurden bei der als Internationale Hinterlegungsstelle anerkannten 'European Collection of Animal Cell Cultures' (ECACC) in Salisbury, UK, entsprechend den Anforderungen des Budapester Vertrages für die Internationale Anerkennung der Hinterlegung von Mikroorganismen zum Zwecke der Patentierung, unter den Hinterlegungsnummern ECACC 9002 1702, ECACC 9002 1703 und ECACC 9002 1704 hinterlegt. Eine Erklärung zur Lebensfähigkeit der hinterlegten Proben wird durch die besagte Internationale Hinterlegungsstelle ausgefertigt.

| Zellinie | Hinterlegungsdatum | Hinterlegungsnummer | Datum der Lebensfähigkeitsbescheinigung |
|---|---|---|---|
| Hybridoma Klon 4134-40-1 | 17.02.1990 | 9002 1702 | 17.02.1990 |
| Hybridoma Klon 4147-19-4 | 17.02.1990 | 9002 1703 | 17.02.1990 |
| Hybridoma Klon 4149-1-1 | 17.02.1990 | 9002 1704 | 17.02.1990 |

## III. MEDIEN UND PUFFER

(A) RPMI 1640-Medium

RPMI 1640 (Seromed) mit folgenden Zusätzen:

| | |
|---|---|
| Kälberserum | 15 % |
| L-Glutamin | 4 mM |
| Gentamycin | 0.01% |
| Natrium-Pyruvat | 1 mM |
| 2-Mercaptoethanol | 50 µM |
| Insulin | 5 µM |
| Transferrin | 5 µM |
| Selen (ITS) | 5 µM |

(B) HAT-Medium

1 Liter RPMI 1640 Medium mit 20 ml Zusatz von HAT conc. (50 x) von Boehringer, das die folgende Zusammensetzung aufweist:

| | |
|---|---|
| Hypoxanthin 680 | 5.0 mg/l |
| Aminopterin | 8.8 mg/l |
| Thymidin | 193,8 mg/l |

(C) HT-Medium
1 Liter RPMI 1640 Medium mit 20 ml Zusatz von HT conc. (50 x) von Boehringer, das die folgende Zusammensetzung aufweist:

| | |
|---|---|
| Hypoxanthin 680 | 5.0 mg/l |
| Thymidin | 193,8 mg/l |

(D) BSS-Puffer [Earle's Salzlösung, ohne Ca und Mg, pH 7.4]

| | |
|---|---|
| KCl | 7.3 mM |
| NaCl | 116.0 mM |
| $NaHCO_3$ | 26.0 mM |
| $NaH_2PO_4 \cdot 2H_2O$ | 1.0 mM |
| Glucose | 5.5 mM |
| Phenolrot | 48.0 µM |

1% Zusatz (v/v) einer Penicillin/Streptomycin-Lösung (Seromed) [10'000E Penicillin, 10 mg/ml Streptomycin]

(E) Natriumcarbonat-Puffer [pH 9.6]

| | |
|---|---|
| $Na_2CO_3$ | 477.0 mg |
| $NaHCO_3$ | 879.0 mg |
| $NaN_3$ | 1.8 mg |
| ad 300 ml $H_2O$ | |

(F) PBS-Puffer [pH 7.0]

| | |
|---|---|
| NaCl | 8.5 g |
| $Na_2HPO_4 \cdot 2H_2O$ | 1.28 g |
| $NaH_2PO_4 \cdot 2H_2O$ | 0.436 g |
| ad 1000 ml $H_2O$ | |

(G) PBS-TWEEN-20 [0.1%]
1 ml Tween-20 (Serva) + 1000 ml PBS

(H) PBS-BSA [1%]

| | |
|---|---|
| BSA | 5.0 g |
| $NaN_3$ (0.5 M) | 3.0 ml |
| ad 500 ml PBS | |

(I) Substrat-Puffer [Diethanolamin-Puffer, pH 9.8]

| | |
|---|---|
| Diethanolamin | 97.0 ml |
| $NaN_3$ (0.5 M) | 6.0 ml |
| $MgCl_2 \cdot 6H_2O$ | 100.0 mg |
| ad 1000 ml $H_2O$, Einstellen des pH-Wertes auf pH 9.8 mit HCl conc. | |

Herstellung des Substrates: Unmittelbar vor Gebrauch wird eine Substrattablette (= 5 mg) des p-Nitrophenylphosphat-Substrates (Sigma 104) in 5 ml Substratpuffer gelöst.

## IV. LITERATURVERZEICHNIS

**Ahmad** I und Crawford G, J. Agric. Food Chem., 38: 138-141, 1990
**Campbell** AM, "Monoclonal Antibody Technology", in: Laboratory Techniques in Biochemistry and Molecular Biology; Burdon, RH; Knippenberg PH (Eds.); Elsevier: Amsterdam, 1984; Vol.13, pp. 120-184
**DeLuca,** "Immunofluorescence Analysis", in: Antibody As a Tool, Marchalonis et al, John Wiley & Sons, Ltd., pp 189-231 (1982)
**Ercegovich** CD et al, J. Agric. Food Chem., 29: 559-563, 1981
**Feng** *et al,* J. Agric. Food Chem., 38: 159-163, 1990
**Fleeker** J, J. Assoc. Off. Anal. Chem., 70: 874-878, 1987
**Hargrave** HS und Merkle MG, Weed Sci., 19: 1971
**Iwanzik** W *et al,* Z. PflKrankh. PflSchutz, Suppl. XI: 301-310, 1988
**Kawamura** H, Berzojsky JA, J. Immunol., 136: 58, 1986
**Kelley** M et al, J. Agric. Food Chem., 33: 962-965, 1985
**Köhler** G, Milstein, Nature, 256: 495-497, 1975
**Kulkarni** NP et al, Cancer Res., 41: 2700-2706, 1981
**Littlefield** JW, Science, 145: 709, 1964
**Newsome** WH, J. Agric. Food Chem., 33: 528-530, 1985
**Raab** GM, Clin. Chem., 29: 1757-1761, 1983
**Schlaeppi** J-M *et al,* J. Agric. Food Chem., 37: 1532-1538, 1989
**Shulman** M et al, Nature, 276: 269-270, 1978
**Stocker** JW *et al,* Hoffmann-La Roche Research Disclosure, 21713: 155-157, 1982
**van Rensburg E**, Analyst, 110: 733., 1985
**Wie** SI, Hammock BD, J. Agric. Food Chem., 30: 949-957, 1982
**Zahnow EW**, J. Agric. Food Chem., 30: 854-857, 1982

Patentliteratur

**US-P 4,530,786**
**EP-A 0,044,808**

## V. TABELLEN

Tabelle 1:

| Kreuzreaktivitäten verschiedener Triasulfuronanaloger mit MAb 4134-40-1, MAb 4147-19-4 and MAb 4149-1-1 | | | | | | |
|---|---|---|---|---|---|---|
| | MAb 4134-40-1 | | MAb 4147-19-4 | | MAb 4149-1-1 | |
| | (a) | (b) | (a) | (b) | (a) | (b) |
| Compound | $I_{50}$ (ng/ml) | Kreuzreaktivität (%) | $I_{50}$ (ng/ml) | Kreuzreaktivität (%) | $I_{50}$ (ng/ml) | Kreuzreaktivität (%) |
| Triasulfuron | 7.2 | 100.0 | 0.09 | 100.0 | 0.05 | 100.0 |
| A | 550 | 1.3 | 21.4 | 0.4 | 0.16 | 31.3 |
| B | >1000 | <0.7 | 85 | 0.1 | 0.04 | 125.0 |
| C | >1000 | <0.7 | >1000 | <0.01 | 1000 | <0.01 |
| D | >1000 | <0.7 | >1000 | <0.01 | >1000 | <0.01 |
| E | >1000 | <0.7 | >1000 | <0.01 | >1000 | <0.01 |
| F | >1000 | <0.7 | >1000 | <0.01 | >1000 | <0.01 |
| G | >1000 | <0.7 | 0.06 | 150.0 | 2.3 | 2.2 |
| H | 30 | 24.0 | 0.13 | 69.2 | 16.2 | 0.3 |
| I | >1000 | <0.7 | >1000 | <0.01 | >1000 | <0.01 |
| J | >1000 | <0.7 | >1000 | <0.01 | >1000 | <0.01 |

Tabelle 1: (fortgesetzt)

| Kreuzreaktivitäten verschiedener Triasulfuronanaloger mit MAb 4134-40-1, MAb 4147-19-4 and MAb 4149-1-1 | | | | | | |
|---|---|---|---|---|---|---|
| | MAb 4134-40-1 | | MAb 4147-19-4 | | MAb 4149-1-1 | |
| | (a) | (b) | (a) | (b) | (a) | (b) |
| Compound | $I_{50}$ (ng/ml) | Kreuzreaktivität (%) | $I_{50}$ (ng/ml) | Kreuzreaktivität (%) | $I_{50}$ (ng/ml) | Kreuzreaktivität (%) |
| K | >1000 | <0.7 | >1000 | <0.01 | >1000 | <0.01 |
| L | >1000 | <0.7 | 14.2 | 0.6 | >1000 | <0.01 |
| M | >1000 | <0.7 | 14.3 | 0.6 | 420 | 0.01 |
| N | >1000 | <0.7 | >1000 | <0.01 | >1000 | <0.01 |
| O | >1000 | <0.7 | 81.5 | 0.1 | 600 | <0.01 |
| P | >1000 | <0.7 | >1000 | <0.01 | >1000 | <0.01 |
| Q | >1000 | <0.7 | >1000 | <0.01 | >1000 | <0.01 |
| R | 2.1 | 342.9 | 1.9 | 4.7 | 0.02 | 250.00 |
| S | 6.0 | 120.0 | 2.6 | 3.5 | 0.02 | 250.00 |
| T | 0.9 | 800.0 | 17.6 | 0.5 | 0.01 | 500.00 |
| a) Inhibitorkonzentration, welche das ELISA Signal um 50 % im Vergleich zur Kontrolle reduziert.<br>b) (Triasulfuronkonz. für 50 % Hemmung / Konzentration des Triasulfuronanalogen für 50 % Hemmung) x 100 . | | | | | | |

Die Triasulfuron-Analogen A bis T sind im folgenden anhand ihrer Strukturformeln wiedergegeben.

(I) 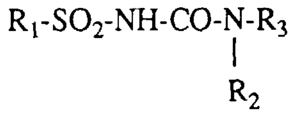

R$_1$ = 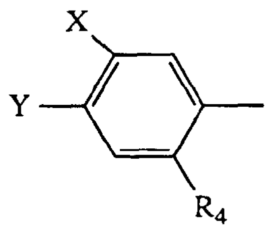 R$_3$ = 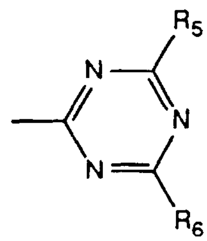

| | $R_4$ | $R_2$ | $R_5$ | $R_6$ | X | Y |
|---|---|---|---|---|---|---|
| (A) | $-OCH_2CH_2Cl$ | -H | $-OCH_3$ | $-CH_3$ | -OH | -H |
| (B) | $-OCH_2CH_2Cl$ | -H | $-OCH_3$ | $-CH_3$ | -H | -OH |
| (O) Methsulfuron-methyl | $-COOCH_3$ | -H | $-OCH_3$ | $-CH_3$ | -H | -H |
| (M) Chlorsulfuron | -Cl | -H | $-OCH_3$ | $-CH_3$ | -H | -H |
| (K) Tribenuron-methyl | $-COOCH_3$ | $-CH_3$ | $-OCH_3$ | $-CH_3$ | -H | -H |
| Triasulfuron | $-OCH_2CH_2Cl$ | -H | $-OCH_3$ | $-CH_3$ | -H | -H |
| (G) Cinosulfuron | $-OCH_2CH_2OCH_3$ | -H | $-OCH_3$ | $-OCH_3$ | -H | -H |
| (H) | $-SCH_2CH_2F$ | -H | $-OCH_3$ | $-CH_3$ | -H | -H |

$R_1$ = 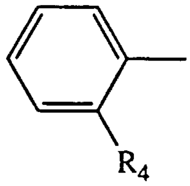

$R_3$ = 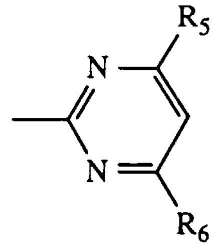

| | $R_4$ | $R_2$ | $R_5$ | $R_6$ |
|---|---|---|---|---|
| (J) Sulfometuron-methyl | $-COOCH_3$ | -H | $-CH_3$ | $-CH_3$ |
| (I) Primisulfuron | $-COOCH_3$ | -H | $-OCHF_2$ | $-OCHF_2$ |

$R_1$ = 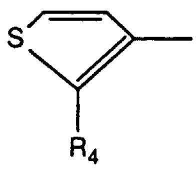

$R_3$ = 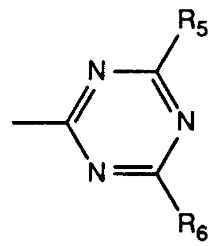

| | | | | |
|---|---|---|---|---|
| (L) Thiameturon-methyl | $-COOCH_3$ | -H | $-OCH_3$ | $-CH_3$ |

$R_1$ =

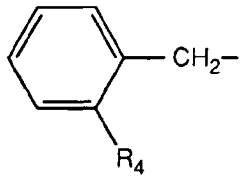

$R_3$ =

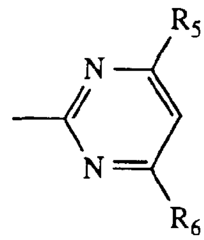

| | | | | |
|---|---|---|---|---|
| (N) Bensulfuron-methyl | $-COOCH_3$ | H | $-OCH_3$ | $-OCH_3$ |

$R_1$=

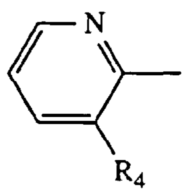

$R_3$=

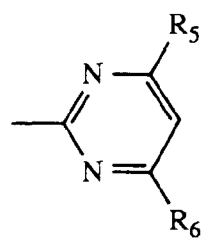

| | $R_4$ | $R_2$ | $R_5$ | $R_6$ |
|---|---|---|---|---|
| (P) Nicosulfuron-methyl | $-CON(CH_3)_2$ | -H | $-OCH_3$ | $-OCH_3$ |
| (Q) DPX 9636 | $-SO_2CH_2CH_3$ | -H | $-OCH_3$ | $-OCH_3$ |

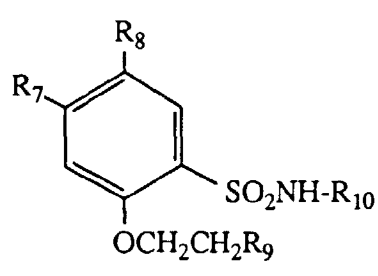

| | $R_7$ | $R_8$ | $R_9$ | $R_{10}$ |
|---|---|---|---|---|
| (C) | -H | -H | -Cl | -H |
| (D) | -H | -H | -OH | -H |
| (E) | -H | -OH | -Cl | -H |
| (S) | -H | -H | -Cl | $-CO-NH_2$ |
| (R) | -H | -H | -Cl | $-CO-O-CH_2CH_2CH_3$ |
| (T) | -H | -H | -Cl | $-CO-N(CH_2CH_3)_2$ |
| (F) | 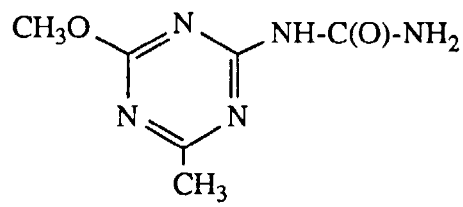 | | | |

TABELLE 2: Prozent des nach Zugabe in Extrakte aus standardisierten Bodenproben in den Extrakten wiedergefundenen Triasulfurons.

| | Bodenbestandteile | | | | | | MAb 4147-19-4 | | MAb 4149-1-1 | |
|---|---|---|---|---|---|---|---|---|---|---|
| Bodenprobe | Humus (%) | Sand (%) | Schlick (%) | Ton (%) | pH | Triasulfuron zugegeben, ppb | Triasulfuron (b) gemessen, ppb | Triasulfuron (c) wiedergefun. (%) | Triasulfuron (b) gemessen, ppb | Triasulfuron (c) wiedergefun. (%) |
| Vetroz (Schweiz) | 9.3 | 18.1 | 60.4 | 21.5 | 7.3 | 0 | 0.17 | | 0.17 | |
| | | | | | | 0.1 | 0.26 | 90 | 0.23 | 60 |
| | | | | | | 0.3 | 0.44 | 90 | 0.47 | 100 |
| | | | | | | 1 | 1.11 | 94 | 1.10 | 93 |
| Stein (Schweiz) | 5.0 | 43.0 | 17.4 | 34.6 | 7.1 | 0 | 0.06 | | 0.07 | |
| | | | | | | 0.1 | 0.13 | 70 | 0.20 | 130 |
| | | | | | | 0.3 | 0.39 | 110 | 0.39 | 107 |
| | | | | | | 1 | 1.14 | 108 | 1.04 | 97 |
| Collombey (Schweiz) | 1.4 | 83.9 | 13.6 | 2.5 | 7.4 | 0 | 0.09 | | 0.06 | |
| | | | | | | 1 | 1.08 | 99 | 1.04 | 98 |
| Les Evouettes (Schweiz) | 2.6 | 25.7 | 64.0 | 10.3 | 6.2 | 0 | 0.10 | | 0.11 | |
| | | | | | | 1 | 0.91 | 81 | 1.00 | 89 |
| Speyer (Deutschland) | 1.0 | 93.0 | 3.1 | 2.9 | 7.4 | 0 | 0.04 | | 0.07 | |
| | | | | | | 10 | 1.03 | 99 | 0.95 | 88 |

(a) Bodenextrakte hergestellt nach Methode A [vergl. Beispiel 8.1]

(b) Kalkuliert basierend auf in PBS-Tween hergestellten Standards von Triasulfuron (Mittelwert aus 5 Bestimmungen)

(c) [(ppb gemessen nach Zugabe - ppb vor Zugabe)/ppb zugegeben] x 100.

TABELLE 3:

| Boden-Matrix-Effect: Vergleich zwischen verschiedenen Extraktionsverfahren. | | | |
|---|---|---|---|
| | | MAb 4147-19-4 | Mab 4149-1-1 |
| | | Triasulfuron | Triasulfuron |
| | (a) | (b) | (b) |
| Bodenprobe | Extraktion-Verfahren | gemessen ppb | gemessen ppb |
| Vetroz | A | 0.17 | 0.17 |
| | B | 0.03 | 0.02 |
| | C (Expt. 1) | 0.02 | nd |
| | (Expt. 2) | 0.07 | 0.16 |
| Les Evouettes | A | 0.10 | 0.11 |
| | B | 0.02 | 0.01 |
| | C (Expt. 1) | 0.06 | nd |
| | (Expt. 2) | 0.07 | 0.19 |
| Stein | A | 0.06 | 0.07 |
| | C | 0.01 | 0.09 |
| Collombey | A | 0.09 | 0.06 |
| | C | 0.03 | 0.10 |
| Speyer | A | 0.04 | 0.07 |
| | C | 0.02 | 0.12 |

a) siehe Beispiel 8.1
b) Kalkuliert basierend auf in PBS-Tween hergestellten Standards von Triasulfuron (Mittelwert aus 4 bis 8 Bestimmungen pro Experiment)
nd: nicht bestimmt.

TABELLE 4: Wiedergewinnung von Triasulfuron aus verschiednenen, mit Triasulfuron angereicherten Bodenproben (a)

| Bodenprobe | Triasulfuron zugegeben, ppb | wiedergefundenes Triasulfuron (MAb 4147-19-4) (b) ppb | (c) (%) | SD | (d) (CV) |
|---|---|---|---|---|---|
| Vetroz (Schweiz) | 0.1 | 0.14 | (120) | 0.023 | (16.4) |
| | 0.5 | 0.34 | (64) | 0.033 | (9.7) |
| | 1 | 0.68 | (66) | 0.115 | (16.9) |
| | 10 | 6.60 | (66) | 0.801 | (12.1) |
| (Expt. 2) | 0.1 | 0.19 | (120) | 0.021 | (11.1) |
| | 0.3 | 0.31 | (80) | 0.017 | (5.5) |
| | 1 | 0.60 | (53) | 0.046 | (7.7) |
| Les Evouettes (Schweiz) | 0.5 | 0.37 | (62) | 0.074 | (20.0) |
| | 1 | 0.71 | (65) | 0.184 | (25.9) |
| | 10 | 7.41 | (74) | 1.129 | (15.2) |
| Stein (Schweiz) | 0.1 | 0.12 | (110) | 0.023 | (19.2) |
| | 0.3 | 0.25 | (80) | 0.008 | (3.2) |
| | 1 | 0.63 | (62) | 0.040 | (6.4) |

(a) Bodenproben extrahiert gemäss Methode C [vergl. Beispiel 8.1].

(b) Kalkuliert basierend auf einem in PBS-Tween hergestellten Standard (Mittelwert aus 4 Bestimmungen)

(c) [(ppb gemessen nach Zugabe - ppb vor Zugabe)/ppb zugegeben] x 100.

(d) SD, Standardabweichung,; CV, Variationskoeffizient

## Patentansprüche

### Patentansprüche für folgende Vertragsstaaten : AT, BE, CH, DE, DK, FR, GB, GR, IT, LI, LU, NL, SE

**1.** Verfahren zur Herstellung eines monoklonalen Antikörpers mit hoher Spezifität und Affinität gegenüber einem oder mehreren Sulfonylharnstoff-Herbiziden, dadurch gekennzeichnet, dass man

(a) eine Kupplungskomponente, die im wesentlichen aus dem Sulfonamidteil des Zielmoleküls besteht, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert.

**2.** Verfahren gemäss Anspruch 1, dadurch gekennzeichnet, dass es sich bei besagtem Sulfonamidteil um substituiertes Arylsulfonyl handelt.

**3.** Verfahren gemäss Anspruch 2, dadurch gekennzeichnet, dass es sich bei besagtem Arylrest um Phenyl, Pyridyl, Thienyl oder Pyrazolyl handelt.

**4.** Verfahren gemäss einem der Ansprüche 1 bis 3, dadurch gekennzeichnet, dass es sich bei besagtem Sulfonylharnstoff-Herbizid um eine Verbindung der Formel (A) handelt,

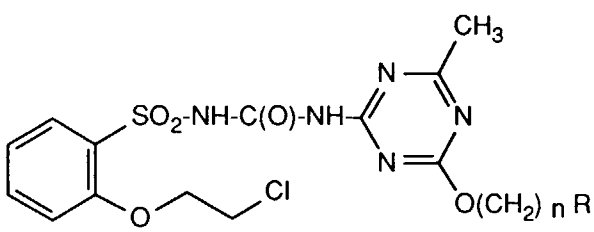

(A)

die in 4 Stellung des Triazinringes eine $R-(CH_2)_n-O-$ Gruppierung aufweist, worin

R für COOH steht, wenn n eine ganze Zahl von 1 bis 10 repräsentiert und
R für COOH, , $NH_2$ oder SH steht, wenn n eine ganze Zahl von 2 bis 10 repräsentiert.

**5.** Verfahren gemäss einem der Ansprüche 1 bis 4, dadurch gekennzeichnet, dass man als Sulfonamidteil das stabilisierte Fragment (B) einsetzt,

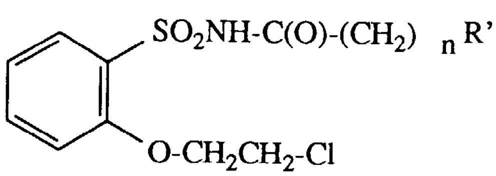

(B)

worin
R' für COOH, $NH_2$ oder SH steht und n eine ganze Zahl von 1 bis 10 repräsentiert.

**6.** Verfahren gemäss einem der Ansprüche 1 bis 5, dadurch gekennzeichnet, dass R in Formel (A) für $NH_2$ steht und

n eine ganze Zahl von 2 bis 6 repräsentiert.

**7.** Verfahren gemäss Anspruch 6, dadurch gekennzeichnet, dass R für $NH_2$ steht und n = 3 ist.

**8.** Verfahren gemäss einem der Ansprüche 1 bis 7, dadurch gekennzeichnet, dass R' in Formel (B) für COOH steht und n eine ganze Zahl von 1 bis 6 repräsentiert.

**9.** Verfahren gemäss Anspruch 8, dadurch gekennzeichnet, dass R' für COOH steht und n = 2 ist.

**10.** Monoklonaler Antikörper mit hoher Spezifität und Affinität gegenüber einem oder mehreren Sulfonylharnstoff-Herbiziden hergestellt nach einem Verfahren gemäss einem der Ansprüche 1 bis 9.

**11.** Hybridomazellinie, die einen monoklonalen Antikörper produziert, der eine hohe Spezifität und Affinität gegenüber Triasulfuron aufweist und der mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P (Nicosulfuron-methyl) und Q (DPX 9636) aus Tabelle 1 eine Kreuzreaktivität von < 1.0% zeigt.

**12.** Hybridomazellinie gemäß Anspruch 11, die einen monoklonalen Antikörper produziert, der eine hohe Spezifität und Affinität gegenüber Triasulfuron aufweist und der mit strukturell verwandten Triasulfuronanalogen ausgewählt aus der Gruppe bestehend aus den Verbindungen C, D, E, F, I (Prinüsulfuron), J (Sulfometuron-methyl), K (Tribenuron-methyl), N (Bensulfuron-methyl), P (Nicosulfuron-methyl) und Q (DPX 9636) aus Tabelle 1 eine Kreuzreaktivität von < 0.01% zeigt.

**13.** Hybridomazellinie gemäss Anspruch 12, welche monoklonale Antikörper produziert, die die in Anspruch 12 genannten Kreuzreaktivitäten zeigen und die unter der Nummer ECACC 9002 1703 hinterlegt ist, sowie Klone und Subklone davon.

**14.** Hybridomazellinie gemäss Anspruch 12, welche monoklonale Antikörper produziert, die die in Anspruch 12 genannten Kreuzreaktivitäten zeigen und die unter der Nummer ECACC 9002 1704 hinterlegt ist, sowie Klone und Subklone davon.

**15.** Mutanten und Varianten einer Hybridomazellinie gemäss einem der Ansprüche 11 bis 14, die noch in der Lage sind Antikörper mit den in den Ansprüchen 11 und 12 genannten Kreuzreaktivitäten zu synthetisieren und ins umgebende Medium zu sezernieren.

**16.** Monoklonaler Antikörper und Derivate davon, der eine hohe Spezifität und Affinität gegenüber Triasulfuron aufweist und der mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P (Nicosulfuron-methyl) und Q (DPX 9636) aus Tabelle 1 eine Kreuzreaktivität von < 1.0% zeigt.

**17.** Monoklonaler Antikörper und Derivate davon gemäß Anspruch 16, der eine hohe Spezifität und Affinität gegenüber Triasulfuron aufweist und der mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), N (Bensulfuron-methyl), P (Nicosulfuron-methyl) und Q (DPX 9636) aus Tabelle 1 eine Kreuzreaktivität von < 0.01% zeigt.

**18.** Monoklonaler Antikörper und Derivate davon gemäss Anspruch 17, dadurch gekennzeichnet, dass er die in Anspruch 17 genannten Kreuzreaktivitäten zeigt und aus einer Hybridomazellinie gemäss Anspruch 13 sowie aus Klonen und Subklonen davon erhältlich ist.

**19.** Monoklonaler Antikörper und Derivate davon gemäss Anspruch 17, dadurch gekennzeichnet, dass er die in Anspruch 17 genannten Kreuzreaktivitäten zeigt und aus einer Hybridomazellinie gemäss Anspruch 14 sowie aus Klonen und Subklonen davon erhältlich ist.

**20.** Verfahren gemäss einem der Ansprüche 1 bis 9, dadurch gekennzeichnet, dass man als Carriermolekül makromolekulare Verbindungen verwendet, die frei zugängliche reaktive Gruppen für die Kupplungsreaktion mit der Sulfonylharnstoff-Kupplungskomponente aufweisen und die in der Lage sind, der Sulfonylharnstoff-Komponente

eine immunogene Potenz zu verleihen.

**21.** Verfahren gemäss Anspruch 20, dadurch gekennzeichnet, dass man ein lysinreiches Protein mit einem Molekulargewicht zwischen 10'000 und 1'500'000 verwendet.

**22.** Verfahren gemäss einem der Ansprüche 1 bis 9, dadurch gekennzeichnet, dass die Konjugation der Sulfonylharnstoff-Kupplungskomponente an das Carriermolekül direkt oder aber über ein Brückenglied (Spacer) erfolgt, das eine oder mehrere reaktive Gruppen besitzt, die in der Lage sind mit den reaktiven Gruppen des Carriermoleküls in Wechselwirkung zu treten.

**23.** Verfahren gemäss Anspruch 22, dadurch gekennzeichnet, dass es sich bei besagten reaktiven Gruppen um Carboxyl-, Amino- oder SH-Gruppen handelt.

**24.** Verfahren gemäss einem der Ansprüche 1 bis 9, dadurch gekennzeichnet, dass die Kupplungsreaktion mit Hilfe der aktiven Estermethode oder des Diazoniumverfahrens durchgeführt wird.

**25.** Verfahren gemäss einem der Ansprüche 1 bis 9, dadurch gekennzeichnet, dass die Immunisierung der Donor-Tiere durch ein- bis mehrmalige Applikation eines Konjugates, bestehend aus Kupplungskomponente und Carrier-Molekül erfolgt.

**26.** Verfahren gemäss Anspruch 25, dadurch gekennzeichnet, dass die Applikation in Form einer intravenösen, intraperitonealen oder subcutanen Injektion oder einer Kombination davon erfolgt.

**27.** Verfahren gemäss einem der Ansprüche 1 bis 9, dadurch gekennzeichnet, dass man für die Selektion fusionierte Hybridzellen das HAT-Selektionsmedium verwendet.

**28.** Verfahren gemäss einem der Ansprüche 1 bis 9, dadurch gekennzeichnet, dass man positive, monoklonale Antikörper produzierende Hybridzellkulturen mit Hilfe des limitierenden Verdünnungsverfahrens vereinzelt und anschliessend in geeigneten Kultivierungsmedien kloniert.

**29.** Verfahren gemäss einem der Ansprüche 1 bis 9, dadurch gekennzeichnet, dass man die gemäss Anspruch 28 klonierten Hybridoma-Zellklone mit Hilfe eines Immunoassays auf die Bildung geeigneter monoklonaler Antikörper hin untersucht.

**30.** Verfahren zur Herstellung einer Hybridomazellinie, die einen monoklonalen Antikörper mit hoher Spezifität und Affinität gegenüber einem oder mehreren Sulfonylharnstoff-Herbiziden produziert, dadurch gekennzeichnet, dass man

(a) eine Kupplungskomponente, die im wesentlichen aus dem Sulfonamidteil des Zielmoleküls besteht, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert; und
(e) das entstehende Fusionsprodukt isoliert und nach Selektion diejenigen Hybridomazellen kloniert, die den gewünschten Antikörper produzieren.

**31.** Verfahren gemäss Anspruch 30, dadurch gekennzeichnet, dass es sich bei besagtem Sulfonamidteil um substituiertes Arylsulfonyl handelt.

**32.** Verfahren gemäss Anspruch 31, dadurch gekennzeichnet, dass es sich bei besagtem Arylrest um Phenyl, Pyridyl, Thienyl oder Pyrazolyl handelt.

**33.** Verfahren gemäss einem der Ansprüche 30 bis 32, dadurch gekennzeichnet, dass man als Sulfonamidteil das stabilisierte Fragment (B) einsetzt,

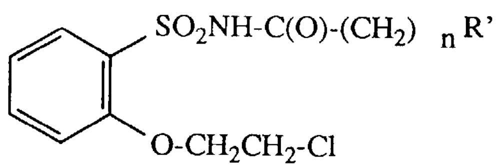

(B)

worin
R' für COOH, $NH_2$ oder SH steht und n eine ganze Zahl von 1 bis 10 repräsentiert.

**34.** Verfahren zum immunologischen Nachweis von Sulfonylharnstoff-Herbiziden, dadurch gekennzeichnet, dass man einen monoklonalen Antikörper gemäss Anspruch 10 verwendet.

**35.** Verfahren zum immunologischen Nachweis von Triasulfuron, dadurch gekennzeichnet, dass man einen monoklonalen Antikörper gemäss einem der Ansprüche 16 bis 19 verwendet.

**36.** Verfahren zum immunologischen Nachweis von Triasulfuron gemäss Anspruch 35, dadurch gekennzeichnet, dass man einen monoklonalen Antikörper verwendet, der die in Anspruch 17 genannten Kreuzreaktivitäten zeigt und aus einer Hybridomazellinie gemäss Anspruch 13 sowie aus Klonen und Subklonen davon erhältlich ist.

**37.** Verfahren zum immunologischen Nachweis von Triasulfuron gemäss Anspruch 35, dadurch gekennzeichnet, dass man einen monoklonalen Antikörper verwendet, der die in Anspruch 17 genannten Kreuzreaktivitäten zeigt und aus einer Hybridomazellinie gemäss Anspruch 14 sowie aus Klonen und Subklonen davon erhältlich ist.

**38.** Verfahren gemäss einem der Ansprüche 34 bis 37, dadurch gekennzeichnet, dass es sich um einen kompetitiven Immunassay handelt.

**39.** Mittel zum immunologischen Nachweis von Sulfonylharnstoff-Herbiziden in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen monoklonalen Antikörper gemäss Anspruch 10 als Reagenz enthält.

**40.** Mittel zum immunologischen Nachweis von Triasulfuron in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen monoklonalen Antikörper gemäss einem der Ansprüche 16 bis 19 als Reagenz enthält.

**41.** Mittel zum immunologischen Nachweis von Triasulfuron in Form eines gebrauchsfertigen Test-Kits gemäss Anspruch 40, dadurch gekennzeichnet, dass besagter Test-Kit neben den üblicherweise verwendeten Trägermaterialien, Reagentien und sonstigen Zusätzen mindestens einen monoklonalen Antikörper enthält, der die in Anspruch 17 genannten Kreuzreaktivitäten zeigt und aus einer Hybridomazellinie gemäss Anspruch 13 sowie aus Klonen und Subklonen davon erhältlich ist.

**42.** Mittel zum immunologischen Nachweis von Triasulfuron in Form eines gebrauchsfertigen Test-Kits gemäss Anspruch 40, dadurch gekennzeichnet, dass besagter Test-Kit neben den üblicherweise verwendeten Trägermaterialien, Reagentien und sonstigen Zusätzen mindestens einen monoklonalen Antikörper enthält, der die in Anspruch 17 genannten Kreuzreaktivitäten zeigt und aus einer Hybridomazellinie gemäss Anspruch 14 sowie aus Klonen und Subklonen davon erhältlich ist.

**43.** Triasulfuron-Kupplungskomponente der Formel (B),

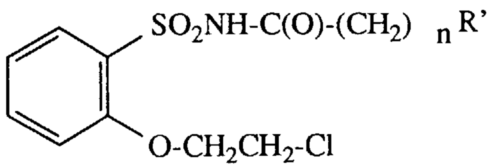

(B)

worin

R' für COOH, $NH_2$ oder SH steht und
n eine ganze Zahl von 1 bis 10 repräsentiert.

**44.** Triasulfuron-Kupplungskomponente gemäss Anspruch 43, dadurch gekennzeichnet, dass R' für COOH steht und n eine ganze Zahl von 1 bis 6 repräsentiert.

**45.** Triasulfuron-Kupplungskomponente gemäss Anspruch 44, dadruch gekennzeichnet, dass R' für COOH steht und n=2 ist.

**46.** Verfahren zur Herstellung einer Sulfonylharnstoff-Kupplungskomponenten, die im wesentlichen aus dem Sulfonamidteil des Sulfonylharnstoffmoleküls besteht und die gekoppelt an ein geeignetes, hochmolekulares Carrier-Molekül in der Lage ist eine spezifische, auf den Sulfonamidteil gerichtete Immunantwort in einem Donortier auszulösen, dadurch gekennzeichnet, dass man ein Sulfonylharnstoff-Herbizid an der Sulfonamid-Funktion nach an sich bekannten Methoden hydrolytisch spaltet und die auf diese Weise erhältliche $-SO_2-NH_2$ Gruppe mit einem reaktionsfähigen Carbonsäurederivat umsetzt und die so entstehende endständige COOH Gruppe gegebenfalls anhand bekannter Verfahren in eine $NH_2$- bzw. SH-Gruppe umwandelt.

**47.** Verfahren zur Herstellung einer Triasulfuron-Kupplungskomponente gemäß Anspruch 46 der Formel (B),

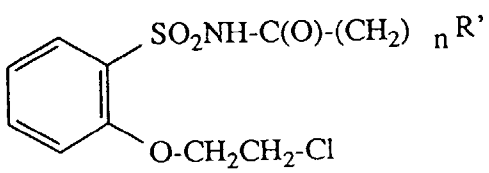

(B)

worin

R' für COOH, $NH_2$ oder SH steht und
n eine ganze Zahl von 1 bis 10 repräsentiert, dadurch gekennzeichnet, dass man ein Sulfonylharnstoff-Herbizid an der Sulfonamid-Funktion nach an sich bekannten Methoden hydrolytisch spaltet und die auf diese Weise erhältliche $-SO_2-NH_2$ Gruppe mit einem reaktionsfähigen Carbonsäurederivat umsetzt und die so entstehende endständige COOH Gruppe gegebenfalls anhand bekannter Verfahren in eine $NH_2$- bzw. SH-Gruppe umwandelt.

**Patentansprüche für folgenden Vertragsstaat : ES**

**1.** Verfahren zur Herstellung einer Hybridomazellinie, die einen monoklonalen Antikörper mit hoher Spezifität und Affinität gegenüber einem oder mehreren Sulfonylharnstoff-Herbiziden synthetisiert, dadurch gekennzeichnet, dass man

(a) eine Kupplungskomponente, die im wesentlichen aus dem Sulfonamidteil des Zielmoleküls besteht, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;

(d) besagte immunkompetente B-Zellen mit zu einer kontinuierlichen Zellteilung befähigten Tumorzellen fusioniert; und

(e) das entstehende Fusionsprodukt isoliert und nach Selektion diejenige Hybridomazelle kloniert, die den gewünschten Antikörper produziert.

**2.** Verfahren gemäss Anspruch 1, dadurch gekennzeichnet, dass es sich bei besagtem Sulfonamidteil um substituiertes Arylsulfonyl handelt.

**3.** Verfahren gemäss Anspruch 2, dadurch gekennzeichnet, dass es sich bei besagtem Arylrest um Phenyl, Pyridyl, Thienyl oder Pyrazolyl handelt.

**4.** Verfahren gemäss einem der Ansprüche 1 bis 3, dadurch gekennzeichnet, dass es sich bei besagtem Sulfonylharnstoff-Herbizid um eine Verbindung der Formel (A) handelt,

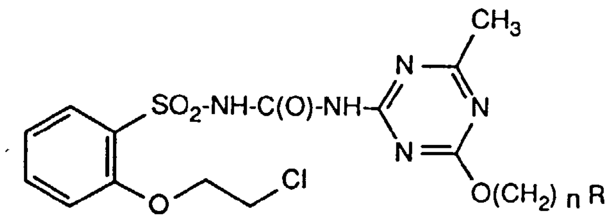

(A)

die in 4 Stellung des Triazinringes eine R-$(CH_2)_n$-O- Gruppierung aufweist, worin

R für COOH steht, wenn n eine ganze Zahl von 1 bis 10 repräsentiert und
R für COOH,, $NH_2$ oder SH steht, wenn n eine ganze Zahl von 2 bis 10 repräsentiert.

**5.** Verfahren gemäss einem der Ansprüche 1 bis 4, dadurch gekennzeichnet, dass man als Sulfonamidteil das stabilisierte Fragment (B) einsetzt,

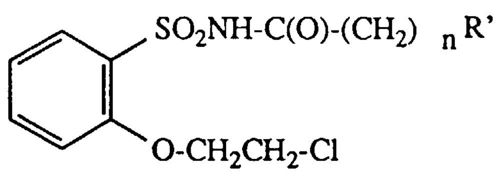

(B)

worin
R' für COOH, $NH_2$ oder SH steht und n eine ganze Zahl von 1 bis 10 repräsentiert.

**6.** Verfahren gemäss einem der Ansprüche 1 bis 5, dadurch gekennzeichnet, dass R in Formel (A) für $NH_2$ steht und n eine ganze Zahl von 2 bis 6 repräsentiert.

**7.** Verfahren gemäss Anspruch 6, dadurch gekennzeichnet, dass R für $NH_2$ steht und n = 3 ist.

**8.** Verfahren gemäss einem der Ansprüche 1 bis 7, dadurch gekennzeichnet, dass R' in Formel (B) für COOH steht und n eine ganze Zahl von 1 bis 6 repräsentiert.

**9.** Verfahren gemäss Anspruch 8, dadurch gekennzeichnet, dass R' für COOH steht und n = 2 ist.

**10.** Verfahren zur Herstellung eines monoklonalen Antikörpers mit hoher Spezifität und Affinität gegenüber einem oder mehreren Sulfonylharnstoff-Herbiziden, dadurch gekennzeichnet, dass man eine gemäss einem der Ansprüche 1 bis 9 hergestellte Hybridomazellinie *in vitro* oder *in vivo* kultiviert und die produzierten monoklonalen Antikörper aus der Kulturbrühe isoliert.

11. Verfahren gemäss einem der Ansprüche 1 bis 9 dadurch gekennzeichnet, dass man als Carriermolekül makromolekulare Verbindungen verwendet, die frei zugängliche reaktive Gruppen für die Kupplungsreaktion mit der Sulfonylharnstoff-Kupplungskomponente aufweisen und die in der Lage sind, der Sulfonylharnstoff-Komponente eine immunogene Potenz zu verleihen.

12. Verfahren gemäss Anspruch 11, dadurch gekennzeichnet, dass man ein lysinreiches Protein mit einem Molekulargewicht zwischen 10'000 und 1'500'000 verwendet.

13. Verfahren gemäss einem der Ansprüche 1 bis 9, dadurch gekennzeichnet, dass die Konjugation der Sulfonylharnstoff-Kupplungskomponente an das Carriermolekül direkt oder aber über ein Brückenglied (Spacer) erfolgt, das eine oder mehrere reaktive Gruppen besitzt, die in der Lage sind mit den reaktiven Gruppen des Carriermoleküls in Wechselwirkung zu treten.

14. Verfahren gemäss einem der Ansprüche 1 bis 9, dadurch gekennzeichnet, dass die Kupplungsreaktion mit Hilfe der aktiven Estermethode oder des Diazonium-verfahrens durchgeführt wird.

15. Verfahren gemäss einem der Ansprüche 1 bis 9, dadurch gekennzeichnet, dass die Immunisierung der Donor-Tiere durch ein- bis mehrmalige Applikation eines Konjugates, bestehend aus Kupplungskomponente und Carrier-Molekül, erfolgt.

16. Verfahren gemäss einem der Ansprüche 1 bis 9, dadurch gekennzeichnet, dass man Myelomazellinien verwendet, die die kennzeichnenden Charakteristika von Sp2/O-Ag14 oder X63-Ag8.653 besitzen.

17. Verfahren gemäss einem der Ansprüche 1 bis 9, dadurch gekennzeichnet, dass man Myelomazellinien verwendet, die die kennzeichnenden Charakteristika von PAI besitzen.

18. Verfahren gemäss einem der Ansprüche 1 bis 9, dadurch gekennzeichnet, dass man positive, monoklonale Antikörper produzierende Hybridzellkulturen mit Hilfe des limitierenden Verdünnungsverfahrens vereinzelt und anschliessend in geeigneten Kultivierungsmedien kloniert.

19. Verfahren gemäss einem der Ansprüche 1 bis 9, dadurch gekennzeichnet, dass man die gemäss Anspruch 18 klonierten Hybridoma-Zellklone mit Hilfe eines Immunoassays auf die Bildung geeigneter monoklonaler Antikörper hin untersucht.

20. Verfahren gemäss Anspruch 1, dadurch gekennzeichnet, dass besagter monoklonaler Antikörper eine hohe Spezifizität und Affinität gegenüber Triasulfuron aufweist und mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P Nicosulfuron-methyl) und Q (DPX 9636) aus Tabelle 1 eine Kreuzreaktivität von <1.0% zeigt.

21. Verfahren gemäss Anspruch 1, dadurch gekennzeichnet, dass besagter monoklonaler Antikörper eine hohe Spezifizität und Affinität gegenüber Triasulfuron aufweist und mit strukturell verwandten Triasulfuronanalogen ausgewählt aus der Gruppe bestehend aus den Verbindungen C, D, E, F, I (Primisulfuron), J (Sulfometuron-methyl), K (Tribenuron-methyl), L (Thiameturon-methyl), M (Chlorsulfuron), N (Bensulfuron-methyl), O (Metsulfuron-methyl), P (Nicosulfuron-methyl) und Q (DPX 9636) aus Tabelle 1 eine Kreuzreaktivität von <0.01% zeigt.

22. Verfahren gemäss Anspruch 21, dadurch gekennzeichnet, dass besagter monoklonaler Antikörper die genannten Kreuzreaktivitäten zeigt und aus einer Hybridomazellinie erhältlich ist, die unter der Nummer ECACC 9002 1703 hinterlegt worden ist, sowie aus Klonen und Subklonen davon.

23. Verfahren gemäss Anspruch 21, dadurch gekennzeichnet, dass besagter monoklonaler Antikörper die genannten Kreuzreaktivitäten zeigt und aus einer Hybridomazellinie erhältlich ist, die unter der Nummer ECACC 9002 1704 hinterlegt worden ist, sowie aus Klonen und Subklonen davon.

24. Verfahren zur Herstellung von Mutanten und Varianten einer Hybridomazellinie, die durch ein Verfahren gemäss einem der Ansprüche 1 bis 9 erhältlich ist, die noch in der Lage sind Antikörper mit in den Ansprüchen 20 und 21 genannten Kreuzreaktivitäten zu synthetisieren und ins umgebende Medium zu sezemieren, dadurch gekennzeichnet, dass besagte Mutanten und Varianten mit Hilfe an sich bekannter Verfahren hergestellt werden können.

**25.** Verfahren zur Herstellung monoklonaler Antikörper, dadurch gekennzeichnet, dass man eine gemäss einem der Ansprüche 12 bis 19 hergestellte Hybridomazellinie *in vivo* oder *in vitro* mit Hilfe bekannter Verfahren kultiviert und die produzierten monoklonalen Antikörper isoliert.

**26.** Verfahren gemäss einem der Ansprüche 10 oder 25, dadurch gekennzeichnet, dass man für die Kultivierung standardisierte Kulturmedien verwendet ausgewählt aus der Gruppe bestehend aus "Dulbecco's Modified Eagle Medium" (DMEM) oder RPMI 1640, die gegebenenfalls durch Zugabe von Säuger-Seren, durch wachstumsfördernde Zusätze oder durch Spurenelemente ergänzt werden können.

**27.** Verfahren gemäss einem der Ansprüche 10 oder 25, dadurch gekennzeichnet, dass man besagte monoklonale Antikörper durch Expansion einer gemäss einem der Ansprüche 11 bis 22 hergestellten Hybridomazellinie in einem Donor-Tier *in vivo* produziert.

**28.** Verfahren zum immunologischen Nachweis von Sulfonylharnstoff-Herbiziden, dadurch gekennzeichnet, dass man einen gemäss Anspruch 10 hergestellten monoklonalen Antikörper verwendet.

**29.** Verfahren zum immunologischen Nachweis von Triasulfuron, dadurch gekennzeichnet, dass man einen gemäss einem der Ansprüche 25 bis 27 hergestellten monoklonalen Antikörper verwendet.

**30.** Verfahren zum immunologischen Nachweis von Triasulfuron gemäss Anspruch 28, dadurch gekennzeichnet, dass man einen monoklonalen Antikörper verwendet, der aus einer Hybridomazellinie erhältlich ist, welche die kennzeichnenden Charakteristika von ECACC 9002 1703 besitzt oder aus Klonen oder Subklonen davon.

**31.** Verfahren zum immunologischen Nachweis von Triasulfuron gemäss Anspruch 28, dadurch gekennzeichnet, dass man einen monoklonalen Antikörper verwendet, der aus einer Hybridomazellinie erhältlich ist, welche die kennzeichnenden Charakteristika von ECACC 9002 1704 besitzt oder aus Klonen oder Subklonen davon.

**32.** Verfahren gemäss einem der Ansprüche 28 bis 31, dadurch gekennzeichnet, dass es sich um einen kompetitiven Immunassay handelt.

**33.** Verfahren gemäss einem der Ansprüche 28 bis 31, dadurch gekennzeichnet, dass es sich bei besagtem Immunassay um einen Radioimmunassay (RIA), einen enzymgekoppelten Assay (ELISA) oder einen Chemilumineszenzassay handelt.

**34.** Mittel zum immunologischen Nachweis von Sulfonyulharnstoff-Herbiziden in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen gemäss Anspruch 10 hergestellten monoklonalen Antikörper als Reagenz enthält.

**35.** Mittel zum immunologischen Nachweis von Triasulfuron in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen gemäss einem der Ansprüche 25 bis 27 hergestellten monoklonalen Antikörper als Reagenz enthält.

**36.** Mittel zum immunologischen Nachweis von Triasulfuron in Form eines gebrauchsfertigen Test-Kits gemäss Anspruch 34, dadurch gekennzeichnet, dass besagter Test-Kit neben den üblicherweise verwendeten Trägermaterialien, Reagentien und sonstigen Zusätzen mindestens einen monoklonalen Antikörper enthält, der aus einer Hybridomazellinie, welche die kennzeichnenden Charakteristika von ECACC 9002 1703 besitzt oder aus Klonen oder Subklonen davon, erhältlich ist.

**37.** Mittel zum immunologischen Nachweis von Triasulfuron in Form eines gebrauchsfertigen Test-Kits gemäss Anspruch 34, dadurch gekennzeichnet, dass besagter Test-Kit neben den üblicherweise verwendeten Trägermaterialien, Reagentien und sonstigen Zusätzen mindestens einen monoklonalen Antikörper enthält, der aus einer Hybridomazellinie, welche die kennzeichnenden Charakteristika von ECACC 9002 1704 besitzt oder aus Klonen oder Subklonen davon, erhältlich ist.

**38.** Verfahren zur Herstellung einer Sulfonylharnstoff-Kupplungskomponenten, die im wesentlichen aus dem Sulfonamidteil des Sulfonylharnstoffmoleküls besteht und die gekoppelt an ein geeignetes, hochmolekulares Carrier-Molekül in der Lage ist eine spezifische, auf den Sulfonamidteil gerichtete Immunantwort in einem Donortier auszulösen, dadurch gekennzeichnet, dass man ein Sulfonylharnstoff-Herbizid an der Sulfonamid-Funktion nach an sich bekannten Methoden hydrolytisch spaltet und die auf diese Weise erhältliche $-SO_2-NH_2$ Gruppe mit einem reaktionsfähigen Carbonsäurederivat umsetzt und die so entstehende endständige COOH Gruppe gegebenfalls anhand bekannter Verfahren in eine $NH_2$- bzw. SH-Gruppe umwandelt.

**39.** Verfahren gemäss Anspruch 38, dadurch gekennzeichnet dass es sich bei besagter Kupplungskomponente um eine Triasulfuron-Kupplungskomponente der Formel (B) handelt,

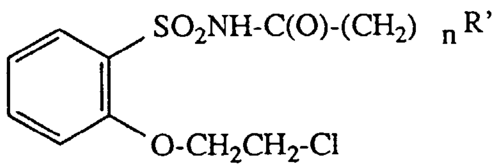

(B)

worin

R' für COOH, $NH_2$ oder SH steht und
n eine ganze Zahl von 1 bis 10 repräsentiert.

**Claims**

**Claims for the following Contracting States : AT, BE, CH, DE, DK, FR, GB, GR, IT, LI, LU, NL, SE**

**1.** A method for the preparation of a monoclonal antibody having a high degree of specificity and affinity towards one or more sulfonylurea herbicides, wherein

(a) a linking component that consists essentially of the sulfonamide moiety of the target molecule is conjugated with a suitable high molecular weight carrier molecule;
(b) a donor animal is immunised with the conjugate prepared in accordance with (a);
(c) immunocompetent B cells are isolated from the immunised donor animal;
(d) the said immunocompetent B cells are fused with tumour cells capable of continuous cell division;
(e) the resulting fusion products are isolated and, after selection, the hybridoma cells that produce the desired antibody are cloned and
(f) the said hybridoma cells are cultured *in vitro* or *in vivo* in order to produce monoclonal antibodies.

**2.** A method according to claim 1 wherein the said sulfonamide moiety is substituted arylsulfonyl.

**3.** A method according to claim 2 wherein the said aryl radical is phenyl, pyridyl, thienyl or pyrazolyl.

**4.** A method according to any one of claims 1 to 3 wherein the said sulfonylurea herbicide is a compound of formula (A)

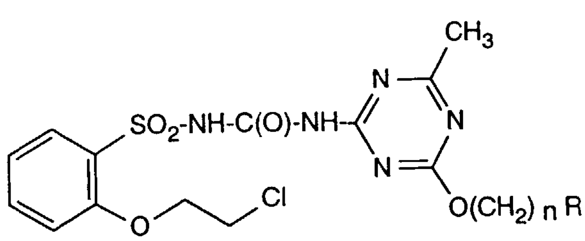

(A)

that has at the 4-position of the triazine ring an $R-(CH_2)_n-O-$ grouping wherein

R is COOH when n is an integer from 1 to 10, and
R is COOH, $NH_2$ or SH when n is an integer from 2 to 10.

**5.** A method according to any one of claims 1 to 4 wherein there is used as sulfonamide moiety the stabilised fragment (B)

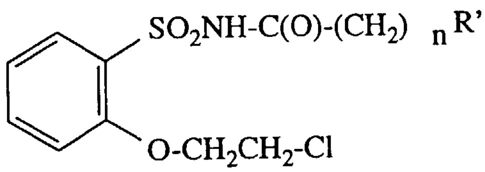

(B),

wherein
R' is COOH, $NH_2$ or SH and n is an integer from 1 to 10.

**6.** A method according to any one of claims 1 to 5 wherein R in formula (A) is $NH_2$ and n is an integer from 2 to 6.

**7.** A method according to claim 6 wherein R is $NH_2$ and n = 3.

**8.** A method according to any one of claims 1 to 7 wherein R' in formula (B) is COOH and n is an integer from 1 to 6.

**9.** A method according to claim 8 wherein R' is COOH and n = 2.

**10.** A monoclonal antibody having a high degree of specificity and affinity towards one or more sulfonylurea herbicides, prepared in accordance with a method according to any one of claims 1 to 9.

**11.** A hybridoma cell line that produces a monoclonal antibody having a high degree of specificity and affinity towards triasulfurone and exhibiting a cross-reactivity of < 1.0 % with structurally related triasulfurone analogs selected from the group consisting of compounds C, D, E, F, I (primisulfurone), J (sulfometurone-methyl), K (tribenurone-methyl), L (thiameturone-methyl), M (chlorosulfurone), N (bensulfurone-methyl), O (metsulfurone-methyl), P (nicosulfurone-methyl) and Q (DPX 9636) from Table 1.

**12.** A hybridoma cell line according to claim 11 that produces a monoclonal antibody having a high degree of specificity and affinity towards triasulfurone and exhibiting a cross-reactivity of < 0.01 % with structurally related triasulfurone analogs selected from the group consisting of compounds C, D, E, F, I (primisulfurone), J (sulfometurone-methyl), K (tribenurone-methyl), N (bensulfurone-methyl), P (nicosulfurone-methyl) and Q (DPX 9636) from Table 1.

**13.** A hybridoma cell line according to claim 12 that produces a monoclonal antibody that exhibits the cross-reactivity mentioned in claim 12 and is filed under the number ECACC 9002 1703, or a clone or a subclone thereof.

**14.** A hybridoma cell line according to claim 12 that produces a monoclonal antibody that exhibits the cross-reactivity mentioned in claim 12 and is filed under the number ECACC 9002 1704, or a clone or a subclone thereof.

**15.** A mutant or variant of a hybridoma cell line according to any one of claims 11 to 14 that is still capable of synthesising and secreting into the surrounding medium the antibody having the cross-reactivity mentioned in claims 11 and 12.

**16.** A monoclonal antibody or a derivative thereof, the said antibody having a high degree of specificity and affinity towards triasulfurone and exhibiting a cross-reactivity of < 1.0 % with structurally related triasulfurone analogs selected from the group consisting of compounds C, D, E, F, I (primisulfurone), J (sulfometurone-methyl), K (tribenurone-methyl), L (thiameturone-methyl), M (chlorosulfurone), N (bensulfurone-methyl), O (metsulfurone-methyl), P (nicosulfurone-methyl) and Q (DPX 9636) from Table 1.

**17.** A monoclonal antibody or a derivative thereof according to claim 16, the said antibody having a high degree of specificity and affinity towards triasulfurone and exhibiting a cross-reactivity of < 0.01 % with structurally related triasulfurone analogs selected from the group consisting of C, D, E, F, I (primisulfurone), J (sulfometurone-methyl), K (triben-urone-methyl), N (bensulfurone-methyl), P (nicosulfurone-methyl) and Q (DPX 9636) from Table 1.

**18.** A monoclonal antibody or a derivative thereof according to claim 17, the said antibody exhibiting the cross-reactivity mentioned in claim 17 and being obtainable from a hybridoma cell line according to claim 13, or from a clone or a subclone thereof.

**19.** A monoclonal antibody or a derivative thereof according to claim 17, the said antibody exhibiting the cross-reactivity mentioned in claim 17 and being obtainable from a hybridoma cell line according to claim 14, or from a clone or a subclone thereof.

**20.** A method according to any one of claims 1 to 9 wherein there is used as carrier molecule a macromolecular compound that comprises freely accessible reactive groups for the linking reaction with the sulfonylurea linking component and that is capable of imparting an immunogenic potential to the sulfonylurea component.

**21.** A method according to claim 20 wherein a lysine-rich protein having a molecular weight of from 10 000 to 1 500 000 is used.

**22.** A method according to any one of claims 1 to 9 wherein the conjugation of the sulfonylurea linking component with the carrier molecule takes place directly or by way of a spacer fragment (spacer) having one or more reactive groups that are capable of interacting with the reactive groups of the carrier molecule.

**23.** A method according to claim 22 wherein the said reactive groups are carboxy, amino or SH groups.

**24.** A method according to any one of claims 1 to 9 wherein the linking reaction is carried out using the active ester method or the diazonium method.

**25.** A method according to any one of claims 1 to 9 wherein the immunisation of the donor animals is effected by means of one or more administrations of a conjugate consisting of linking component and carrier molecule.

**26.** A method according to claim 25 wherein the administration is in the form of an intravenous, intraperitoneal or subcutaneous injection, or a combination thereof.

**27.** A method according to any one of claims 1 to 9 wherein the HAT selection medium is used for the selection of fused hybrid cells.

**28.** A method according to any one of claims 1 to 9 wherein positive monoclonal-antibody-producing hybrid cell cultures are separated by means of the limiting dilution method and then cloned in suitable culture media.

**29.** A method according to any one of claims 1 to 9 wherein the hybridoma cell clones cloned in accordance with claim 28 are examined by means of an immunoassay for the formation of suitable monoclonal antibodies.

**30.** A method for the preparation of a hybridoma cell line that produces a monoclonal antibody having a high degree of specificity and affinity towards one or more sulfonylurea herbicides wherein

a) a linking component that consists essentially of the sulfonamide moiety of the target molecule is conjugated with a suitable high molecular weight carrier molecule;
b) a donor animal is immunised with the conjugate prepared in accordance with (a);
c) immunocompetent B cells are isolated from the immunised donor animal;
d) the said immunocompetent B cells are fused with tumour cells capable of continuous cell division; and
e) the resulting fusion products are isolated and, after selection, the hybridoma cells that produce the desired antibody are cloned.

**31.** A method according to claim 30 wherein the said sulfonamide moiety is substituted arylsulfonyl.

**32.** A method according to claim 31 wherein the said aryl radical is phenyl, pyridyl, thienyl or pyrazolyl.

**33.** A method according to any one of claims 30 to 32 wherein there is used as sulfonamide moiety the stabilised fragment (B)

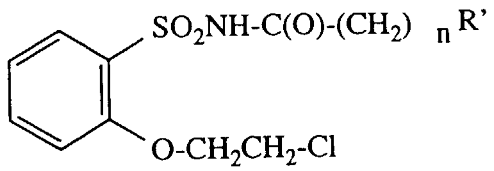

(B),

wherein
R' is COOH, $NH_2$ or SH and n is an integer from 1 to 10.

**34.** A method for the immunological detection of sulfonylurea herbicides wherein a monoclonal antibody according to claim 10 is used.

**35.** A method for the immunological detection of triasulfurone wherein a monoclonal antibody according to any one of claims 16 to 19 is used.

**36.** A method for the immunological detection of triasulfurone according to claim 35 wherein the monoclonal antibody used exhibits the cross-reactivity mentioned in claim 17 and is obtainable from a hybridoma cell line according to claim 13, or from a clone or a subclone thereof.

**37.** A method for the immunological detection of triasulfurone according to claim 35 wherein the monoclonal antibody used exhibits the cross-reactivity mentioned in claim 17 and is obtainable from a hybridoma cell line according to claim 14, or from a clone or a subclone thereof.

**38.** A method according to any one of claims 34 to 37 that is a competitive immunoassay.

**39.** A composition for the immunological detection of sulfonylurea herbicides in the form of a ready-to-use test kit, the said test kit comprising, in addition to the carrier materials, reagents and other additives customarily used, at least one monoclonal antibody according to claim 10 as reagent.

**40.** A composition for the immunological detection of triasulfurone in the form of a ready-to-use test kit, the said test kit comprising, in addition to the carrier materials, reagents and other additives customarily used, at least one monoclonal antibody according to any one of claims 16 to 19 as reagent.

**41.** A composition for the immunological detection of triasulfurone in the form of a ready-to-use test kit according to claim 40, the said test kit comprising, in addition to the carrier materials, reagents and other additives customarily used, at least one monoclonal antibody that exhibits the cross-reactivity mentioned in claim 17 and is obtainable from a hybridoma cell line according to claim 13, or from a clone or a subclone thereof.

**42.** A composition for the immunological detection of triasulfurone in the form of a ready-to-use test kit according to claim 40, the said test kit comprising, in addition to the carrier materials, reagents and other additives customarily used, at least one monoclonal antibody that exhibits the cross-reactivity mentioned in claim 17 and is obtainable from a hybridoma cell line according to claim 14, or from a clone or a subclone thereof.

**43.** A triasulfurone linking component of formula (B)

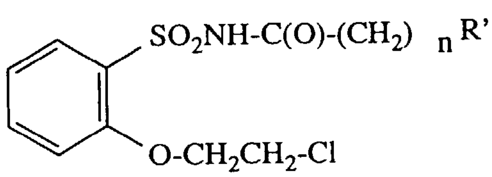

(B)

wherein

R' is COOH, $NH_2$ or SH and

n is an integer from 1 to 10.

**44.** A triasulfurone linking component according to claim 43 wherein R' is COOH and n is an integer from 1 to 6.

**45.** A triasulfurone linking component according to claim 44 wherein R' is COOH and n=2.

**46.** A method for the preparation of a sulfonylurea linking component that consists essentially of the sulfonamide moiety of the sulfonylurea molecule and that, linked to a suitable high molecular weight carrier molecule, is capable of triggering in a donor animal a specific immune response directed towards the sulfonamide moiety, wherein a sulfonylurea herbicide is cleaved hydrolytically at the sulfonamide function by methods known *per se* and the $-SO_2-NH_2$ group obtainable in that manner is reacted with a reactive carboxylic acid derivative and the resulting terminal COOH group is optionally converted by means of known methods into an $NH_2$ group or an SH group.

**47.** A method for the preparation of a triasulfone linking component according to claim 46 of formula (B)

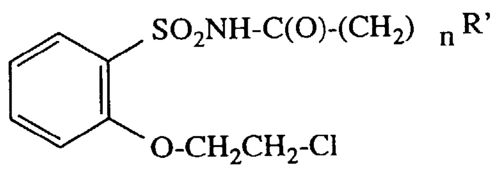

(B),

wherein

R' is COOH, $NH_2$ or SH and
n is an integer from 1 to 10,

wherein a sulfonylurea herbicide is cleaved hydrolytically at the sulfonamide function by methods known *per se* and the $-SO_2-NH_2$ group obtainable in that manner is reacted with a reactive carboxylic acid derivative and the resulting terminal COOH group is optionally converted by means of known methods into an $NH_2$ group or an SH group.

**Claims for the following Contracting State : ES**

**1.** A method for the preparation of a hybridoma cell line that synthesises a monoclonal antibody having a high degree of specificity and affinity towards one or more sulfonylurea herbicides, wherein

(a) a linking component that consists essentially of the sulfonamide moiety of the target molecule is conjugated with a suitable high molecular weight carrier molecule;
(b) a donor animal is immunised with the conjugate prepared in accordance with (a);
(c) immunocompetent B cells are isolated from the immunised donor animal;
(d) the said immunocompetent B cells are fused with tumour cells capable of continuous cell division; and
(e) the resulting fusion products are isolated and, after selection, the hybridoma cells that produce the desired antibody are cloned.

**2.** A method according to claim 1 wherein the said sulfonamide moiety is substituted arylsulfonyl.

**3.** A method according to claim 2 wherein the said aryl radical is phenyl, pyridyl, thienyl or pyrazolyl.

**4.** A method according to any one of claims 1 to 3 wherein the said sulfonylurea herbicide is a compound of formula (A)

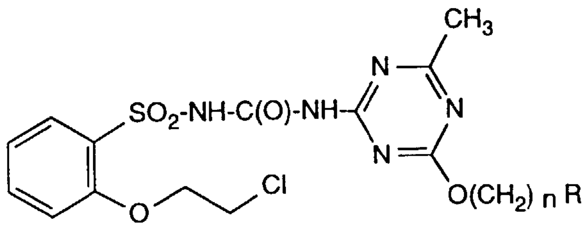

(A)

that has at the 4-position of the triazine ring an $R-(CH_2)_n-O-$ grouping wherein

R is COOH when n is an integer from 1 to 10, and
R is COOH, $NH_2$ or SH when n is an integer from 2 to 10.

**5.** A method according to any one of claims 1 to 4 wherein there is used as sulfonamide moiety the stabilised fragment (B)

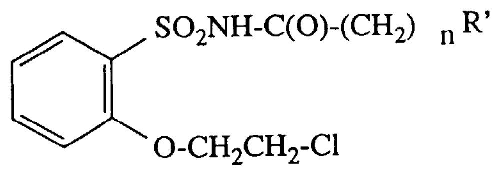

(B),

wherein
R' is COOH, $NH_2$ or SH and n is an integer from 1 to 10.

**6.** A method according to any one of claims 1 to 5 wherein R in formula (A) is $NH_2$ and n is an integer from 2 to 6.

**7.** A method according to claim 6 wherein R is $NH_2$ and n = 3.

**8.** A method according to any one of claims 1 to 7 wherein R' in formula (B) is COOH and n is an integer from 1 to 6.

**9.** A method according to claim 8 wherein R' is COOH and n = 2.

**10.** A method for the production of a monoclonal antibody having a high degree of specificity and affinity towards one or more sulfonylurea herbicides, wherein a hybridoma cell line prepared in accordance with any one of claims 1 to 9 is cultured *in vitro* or *in vivo* and the monoclonal antibodies produced are isolated from the culture broth.

**11.** A method according to any one of claims 1 to 9 wherein there is used as carrier molecule a macromolecular compound that comprises freely accessible reactive groups for the linking reaction with the sulfonylurea linking component and that is capable of imparting an immunogenic potential to the sulfonylurea component.

**12.** A method according to claim 11 wherein a lysine-rich protein having a molecular weight of from 10 000 to 1 500 000 is used.

**13.** A method according to any one of claims 1 to 9 wherein the conjugation of the sulfonylurea linking component with the carrier molecule takes place directly or by way of a spacer fragment (spacer) having one or more reactive groups that are capable of interacting with the reactive groups of the carrier molecule.

**14.** A method according to any one of claims 1 to 9 wherein the linking reaction is carried out using the active ester method or the diazonium method.

**15.** A method according to any one of claims 1 to 9 wherein the immunisation of the donor animals is effected by means of one or more administrations of a conjugate consisting of linking component and carrier molecule.

**16.** A method according to any one of claims 1 to 9 wherein myeloma cell lines having the distinguishing characteristics of Sp2/O-Ag14 or X63-Ag8.653 are used.

**17.** A method according to any one of claims 1 to 9 wherein myeloma cell lines having the distinguishing characteristics of PAI are used.

**18.** A method according to any one of claims 1 to 9 wherein positive monoclonal-antibody-producing hybrid cell cultures are separated by means of the limiting dilution method and then cloned in suitable culture media.

**19.** A method according to any one of claims 1 to 9 wherein the hybridoma cell clones cloned in accordance with claim 18 are examined by means of an immunoassay for the formation of suitable monoclonal antibodies.

**20.** A method according to claim 1 wherein the said monoclonal antibody has a high degree of specificity and affinity towards triasulfurone and exhibits a cross-reactivity of < 1.0 % with structurally related triasulfurone analogs selected from the group consisting of compounds C, D, E, F, I (primisulfurone), J (sulfometurone-methyl), K (tribenurone-methyl), L (thiameturone-methyl), M (chlorosulfurone), N (bensulfurone-methyl), O (metsulfurone-methyl), P (nicosulfurone-methyl) and Q (DPX 9636) from Table 1.

**21.** A method according to claim 1 wherein the said monoclonal antibody has a high degree of specificity and affinity towards triasulfurone and exhibits a cross-reactivity of < 0.01 % with structurally related triasulfurone analogs selected from the group consisting of compounds C, D, E, F, I (primisulfurone), J (sulfometurone-methyl), K (tribenurone-methyl), L (thiameturone-methyl), M (chlorosulfurone), N (bensulfurone-methyl), O (metsulfurone-methyl), P (nicosulfurone-methyl) and Q (DPX 9636) from Table 1.

**22.** A method according to claim 21 wherein the said monoclonal antibody exhibits the mentioned cross-reactivity and is obtainable from a hybridoma cell line filed under the number ECACC 9002 1703, or from a clone or a subclone thereof.

**23.** A method according to claim 21 wherein the said monoclonal antibody exhibits the mentioned cross-reactivity and is obtainable from a hybridoma cell line filed under the number ECACC 9002 1704, or from a clone or a subclone thereof.

**24.** A method for the preparation of a mutant or variant of a hybridoma cell line obtainable by a method according to any one of claims 1 to 9 that is still capable of synthesising and secreting into the surrounding medium an antibody having the cross-reactivity mentioned in claims 20 and 21, wherein the said mutant or variant can be prepared by means of methods known *per se*.

**25.** A method for the production of a monoclonal antibody wherein a hybridoma cell line prepared in accordance with any one of claims 12 to 19 is cultured *in vitro* or *in vivo* by means of known methods and the monoclonal antibodies produced are isolated.

**26.** A method according to either claim 10 or claim 25 wherein there are used for the culturing standard culture media selected from the group consisting of Dulbecco's Modified Eagle Medium (DMEM) or RPMI 1640 which may, if desired, be supplemented by the addition of mammalian sera, with growth-promoting additives or with trace elements.

**27.** A method according to either claim 10 or claim 25 wherein the said monoclonal antibodies are produced *in vivo* in a donor animal by expansion of a hybridoma cell line prepared in accordance with any one of claims 11 to 22.

**28.** A method for the immunological detection of sulfonylurea herbicides wherein a monoclonal antibody produced in accordance with claim 10 is used.

**29.** A method for the immunological detection of triasulfurone wherein a monoclonal antibody produced in accordance with any one of claims 25 to 27 is used.

**30.** A method for the immunological detection of triasulfurone according to claim 28 wherein the monoclonal antibody used is obtainable from a hybridoma cell line having the distinguishing characteristics of ECACC 9002 1703, or from a clone or a subclone thereof.

**31.** A method for the immunological detection of triasulfurone according to claim 28 wherein the monoclonal antibody used is obtainable from a hybridoma cell line having the distinguishing characteristics of ECACC 9002 1704, or from a clone or a subclone thereof.

**32.** A method according to any one of claims 28 to 31 that is a competitive immunoassay.

**33.** A method according to any one of claims 28 to 31 wherein the said immunoassay is a radioimmunoassay (RIA), an enzyme-linked assay (ELISA) or a chemiluminescence assay.

**34.** A composition for the immunological detection of sulfonylurea herbicides in the form of a ready-to-use test kit, the said test kit comprising, in addition to the carrier materials, reagents and other additives customarily used, at least one monoclonal antibody prepared in accordance with claim 10 as reagent.

**35.** A composition for the immunological detection of triasulfurone in the form of a ready-to-use test kit, the said test kit comprising, in addition to the carrier materials, reagents and other additives customarily used, at least one monoclonal antibody prepared in accordance with any one of claims 25 to 27 as reagent.

**36.** A composition for the immunological detection of triasulfurone in the form of a ready-to-use test kit according to claim 34, the said test kit comprising, in addition to the carrier materials, reagents and other additives customarily used, at least one monoclonal antibody obtainable from a hybridoma cell line having the distinguishing characteristics of ECACC 9002 1703, or from a clone or a subclone thereof.

**37.** A composition for the immunological detection of triasulfurone in the form of a ready-to-use test kit according to claim 34, the said test kit comprising, in addition to the carrier materials, reagents and other additives customarily used, at least one monoclonal antibody obtainable from a hybridoma cell line having the distinguishing characteristics of ECACC 9002 1704, or from a clone or a subclone thereof.

**38.** A method for the preparation of a sulfonylurea linking component that consists essentially of the sulfonamide moiety of the sulfonylurea molecule and that, linked to a suitable high molecular weight carrier molecule, is capable of triggering in a donor animal a specific immune response directed towards the sulfonamide moiety, wherein a sulfonylurea herbicide is cleaved hydrolytically at the sulfonamide function by methods known *per se* and the -$SO_2$-$NH_2$ group obtainable in that manner is reacted with a reactive carboxylic acid derivative and the resulting terminal COOH group is optionally converted by means of known methods into an $NH_2$ group or an SH group.

**39.** A method according to claim 38 wherein the said linking component is a triasulfurone linking component of formula (B)

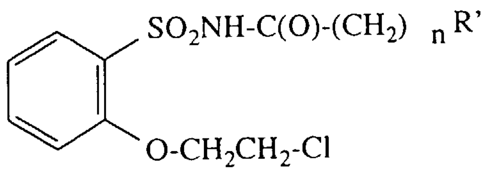

(B),

wherein

R' is COOH, $NH_2$ or SH and
n is an integer from 1 to 10.

**Revendications**

**Revendications pour les Etats contractants suivants : AT, BE, CH, DE, DK, FR, GB, GR, IT, LI, LU, NL, SE**

**1.** Procédé pour la préparation d'un anticorps monoclonal présentant une spécificité et une affinité élevée vis-à-vis d'un ou plusieurs herbicides sulfonylurées, caractérisé

(a) en ce que l'on conjugue un composant de couplage essentiellement constitué de la partie sulfonamide de la molécule visée avec une molécule porteuse de poids moléculaire élevé, appropriée ;

(b) en ce que l'on immunise un animal donneur avec le conjugué préparé selon (a) ;

(c) en ce que l'on isole les cellules B immunocompétentes de l'animal donneur immunisé ;

(d) en ce que l'on fusionne lesdites cellules B immunocompétentes avec des cellules tumorales aptes à une division cellulaire continue ;

(e) en ce que l'on isole le produit de fusion obtenu et en ce que l'on clone, après sélection, les cellules d'hybridomes produisant l'anticorps désiré ; et

(f) en ce que l'on cultive in vitro ou in vivo lesdites cellules d'hybridomes pour la préparation des anticorps monoclonaux.

2. Procédé selon la revendication 1, caractérisé en ce que ladite partie sulfonamide est un arylsulfonyle substitué.

3. Procédé selon la revendication 2, caractérisé en ce que ledit reste aryle est un reste phényle, pyridyle, thiényle ou pyrazolyle.

4. Procédé. selon l'une des revendications 1 à 3, caractérisé en ce que ledit herbicide sulfonylurée est un composé de formule (A)

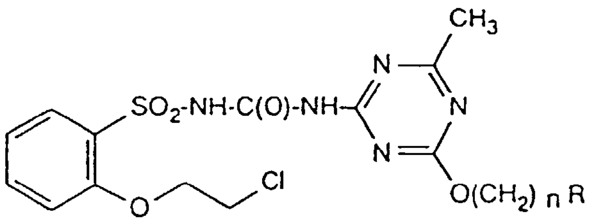

(A)

présentant en position 4 sur le noyau triazinique un groupement R-$(CH_2)_n$-O- où R représente COOH, lorsque n est un nombre entier allant de 1 à 10 et R représente COOH, $NH_2$ ou SH, lorsque n est un mmbre entier allant de 2 à 10.

5. Procédé selon l'une des revendications 1 à 4, caractérisé en ce que l'on utilise comme partie sulfonamide le fragment (B) stabilisé

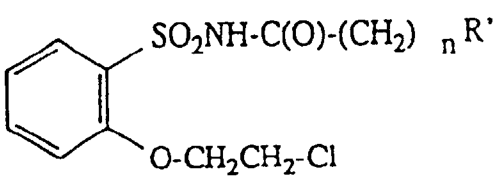

(B)

où R' représente COOH, $NH_2$ ou SH et n est un nombre entier allant de 1 à 10.

6. Procédé selon l'une des revendications 1 à 5, caractérisé en ce que R représente $NH_2$ dans la formule (A) et n est un nombre entier allant de 2 à 6.

7. Procédé selon la revendication 6, caractérisé en ce que R représente $NH_2$ et n est égal à 3.

8. Procédé selon l'une des revendications 1 à 7, caractérisé en ce que R' représente COOH dans la formule (B) et

n est un nombre entier allant de 1 à 6.

**9.** Procédé selon la revendication 8, caractérisé en ce que R' représente COOH et n est égal à 2.

**10.** Anticorps monoclonal présentant une spécificité et une affinité élevées vis-à-vis d'un ou plusieurs herbicides sulfonylurées, préparés par un procédé selon l'une des revendications 1 à 9.

**11.** Ligrnée cellulaire d'hybridomes produisant un anticorps monoclonal présentant une spécificité et une affinité élevées vis-à-vis du triasulfuron et une réactivité croisée inférieure à 1 % avec les analogues du triasulfuron structurellement apparentés choisis dans le groupe constitué par les composés C, D, E, F, I (primisulfuron), J (sulfometuron-méthyle), K (tribénuron-méthyle), L (thiameturon-méthyle), M (chlorsulfuron), N (bensulfuron-méthyle), O (metsulfuron-méthyle), P (nicosulfuronéthyle) et Q (DPX 9636) du Tableau 1.

**12.** Lignée cellulaire d'hybridomes selon la revendication 11, produisant un anticorps monoclonal présentant une spécificité et une affinité élevées vis-à-vis du triasulfuron et une réactivité croisée inférieure à 0,01 % avec les analogues du triasulfuron structurellement apparentés choisis dans le groupe constitué par les composés C, D, E, 1, I (primisulfuron), J (sulfometuronméthyle), K (tribénuron-méthyle), N (bensulfuron-méthyle), P (nicosulfuron-méthyle) et Q (DPX 9636) du Tableau 1.

**13.** Lignée cellulaire d'hybridomes selon la revendication 12, produisant des anticorps monoclonaux présentant les réactivités croisées indiquées dans la revendication 12 et déposée sous le numéro ECACC 9002 1703, ainsi que ses clones et sous-clones.

**14.** Lignée cellulaire d'hybridomes selon la revendication 12, produisant des anticorps monoclonaux présentant les réactivités croisées indiquées dans la revendication 12 et déposée sous le numéro ECACC 9002 1704, ainsi que ses clones et sous-clones.

**15.** Les mutants et variants, d'une lignée cellulaire d'hybridomes selon l'une des revendications 11 à 14, lesdits mutants et variants étant encore en mesure de synthétiser des anticorps présentant les réactivités croisées indiquées dans les revendications 11 et 12 et de les sécréter dans le milieu environnant.

**16.** Anticorps monoclonal et ses dérivés, présentant une spécificité et une affinité élevées vis-à-vis du triasulfuron et une réactivité croisée inférieure à 1,0 % avec les analogues du triasulfuron structurellement apparentés choisis dans le groupe constitué par les composés C, D, E, F, I (primisulfuron), J (sulfometuron-méthyle), K (tribénuron-méthyle), L (thiameturon-méthyle), M (chlorsulfuron), N (bensulfuron-méthyle), O (metsulfuron-méthyle), P (nicosulfuron-méthyle) et Q (DPX 9636) du Tableau 1.

**17.** Anticorps monoclonal et ses dérivés, selon la revendication 16, présentant une spécificité et une affinité élevées vis-à-vis du triasulfuron et une réactivité croisée inférieure à 0,01 % avec les analogues du triasulfuron structurellement aptarentés choisis dans le groupe constitué par les composés C, D, E, F, I (primisulfuron), J (sulfometuron-méthyle), K (tribénuron-méthyle), N (bensulfuron-méthyle), P (nicosulfuronméthyle) et Q (DPX 9636) du Tableau 1.

**18.** Anticorps monoclonal et ses dérivés, selon la revendication 17, caractérisés en ce qu'ils présentent les réactivités croisées indiquées dans la revendication 17 et en ce qu'ils sont obtenus à partir d'une lignée cellulaire d'hybridomes selon la revendication 13, ainsi qu'à partir de ses clones et sous-clones.

**19.** Anticorps monoclonal et ses dérivés, selon la revendication 17, caractérisés en ce qu'ils présentent les réactivités croisées indiquées dans la revendication 17 et en ce qu'ils sont obtenus à partir d'une lignée cellulaire d'hybridomes selon la revendication 14, ainsi qu'à partir de ses clones et sous- clones.

**20.** Procédé selon l'une des revendications 1 à 9, caractérisé en ce que l'on utilise des composés macromoléculaires comme molécule porteuse, présentant des groupes réactifs librement accessibles pour la réaction de couplage avec le composant de couplage sulfonylurée et étant en mesure de conférer au composant sulfonylurée un pouvoir immunogène.

**21.** Procédé selon la revendication 20, caractérisé en ce que l'on utilise une protéine riche en lysine ayant un poids moléculaire compris entre 10.000 et 1.500.000.

**22.** Procédé selon l'une des revendications 1 à 9, caractérisé, en ce que la conjugaison du composant de couplage sulfonylurée à la molécule porteuse s'effectue directement ou par l'intermédiaire d'un pont (espaceur) possédant un ou plusieurs groupes réactifs pouvant interagir avec les groupes réactifs de la molécule porteuse.

**23.** Procédé selon la revendication 22, caractérisé en ce que lesdits groupes réactifs sont les groupes carboxylique, amino ou SH.

**24.** Procédé selon l'une des revendications 1 à 9, caractérisé en ce que la réaction de couplage est effectuée par la méthode de l'ester actif ou par le procédé au diazonium.

**25.** Procédé selon l'une des revendications 1 à 9, caractérisé en ce que l'imrnunisation des animaux donneurs est réalisée par l'administration en une ou plusieurs fois d'un conjugué constitué par le composant de couplage et la molécule porteuse.

**26.** Procèdé selon la revendication 25, caractérisé en ce que l'administration est effectuée sous forme d'une injection par voies intraveineuse, intrapéritonéale ou sous-cutanée ou par une combinaison de ces différentes voies.

**27.** Procédé selon l'une des revendications 1 à 9, caractérisé en ce que l'on utilise pour la sélection des cellules hybrides fusionnées le milieu de sélection HAT.

**28.** Procédé selon l'une des revendications 1 à 9, caractérisé en ce que l'on isole les cultures de cellules hybrides produisant des anticorps monoclonaux positifs par le procédé de dilution limite, puis en ce que l'on clone dans des milieux de culture appropriés.

**29.** Procédé selon l'une des revendications 1 à 9, caractérisé en ce que l'on étudie les clones de cellules d'hybridomes clonés selon la revendication 28 par un immunoessai pour la formation d'anticorps monoclonaux appropriés.

**30.** Procédé pour la préparation d'une lignée cellulaire d'hybridomes produisant un anticorps monoclonal présentant une spécificité et une affinité élevées vis-à-vis d'un ou plusieurs herbicides sulfonylurées, caractérisé

(a) en ce que l'on conjugue un composant de couplage essentiellement constitué de la partie sulfonamide de la molécule visée avec une molécule porteuse de poids moléculaire élevé, appropriée ;

(b) en ce que l'on immunise un animal donneur avec le conjugué préparé selon (a) ;

(c) en ce que l'on isole les cellules B immunocompétentes de l'animal donneur immunisé ;

(d) en ce que l'on fusionne lesdites cellules B immunocompétentes avec des cellules tumorales aptes à une division cellulaire continue ; et

(e) en ce que l'on isole le produit de fusion obtenu et en ce que l'on clone, après sélection, les cellules d'hybridomes produisant l'anticorps désiré.

**31.** Procédé selon la revendication 30, caractérisé en ce que ladite partie sulfonamide est un arylsulfonyle substitué.

**32.** Procédé selon la revendication 31, caractérisé en ce que ledit reste aryle est un reste phényle, pyridyle, thiényle ou pyrazolyle.

**33.** Procédé selon l'une des revendications 30 à 32, caractérisé en ce que l'on utilise comme partie sulfonamide le fragment (B) stabilisé

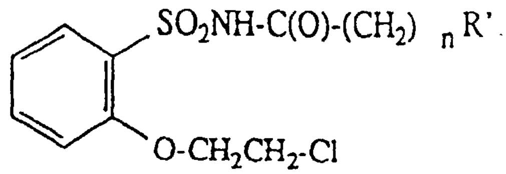

(B)

où R' représente COOH, $NH_2$ ou SH et n est un nombre entier allant de 1 à 10.

**34.** Procédé pour la détection immunologique des herbicides sulfonylurées, caractérisé en ce que l'on utilise un anticorps monoclonal selon la revendication 10.

**35.** Procédé pour la détection immunologique du triasulfuron, caractérisé en ce que l'on utilise un anticorps monoclonal selon l'une des revendications 16 à 19.

**36.** Procédé pour la détection immunologique du triasulfuron selon la revendication 35, caractérisé en ce que l'on utilise un anticorps monoclonal présentant les réactivités croisées indiquées dans la revendication 17 et étant obtenu à partir d'une lignée cellulaire d'hybridomes selon la revendication 13, ainsi qu'à partir de ses clones et sous-clones.

**37.** Procédé pour la détection immunologique du triasulfuron selon la revendication 35, caractérisé en ce que l'on utilise un anticorps monoclonal présentant les réactivités croisées indiquées dans la revendication 17 et étant obtenu à partir d'une lignée cellulaire d'hybridomes selon la revendication 14, ainsi qu'à partir de ses clones et sous-clones.

**38.** Procédé selon l'une des revendications 34 à 37, caractérisé en ce qu'il s'agit d'un immunoessai compétitif.

**39.** Moyens pour la détection immunologique des herbicides sulfonylurées sous forme d'une trousse d'essai prête à l'emploi, caractérisés en ce que ladite trousse d'essai contient à côté des supports, réactifs et autres additifs, utilisés de façon classique, au moins un anticorps monoclonal selon la revendication 10 comme réactif.

**40.** Moyens pour la détection immunologique du triasulfuron sous forme d'une trousse d'essai prête à l'emploi, caractérisés en ce que ladite trousse d'essai contient à côté des supports, réactifs et autres additifs, utilisés de façon classique, au moins un anticorps monoclonal selon l'une des revendications 16 à 19 comme réactif.

**41.** Moyens pour la détection immunologique au triasulfuron sous forme d'une trousse d'essai prête à l'emploi, caractérisés en ce que ladite trousse d'essai contient à côté des supports, réactifs et autres additifs, utilisés de façon classique, au moins un anticorps monoclonal présentant les réactivités croisées indiquées dans la revendication 17 et étant obtenu à partir d'une lignée de cellules d'hybridomes selon la revendication 13, ainsi qu'à partir de ses clones et sous-clones.

**42.** Moyens pour la détection immunologique du triasulfuron sous forme d'une trousse d'essai prête à l'emploi, caractérisés en ce que ladite trousse d'essai contient à côté des supports, réactifs et autres additifs, utilisés de façon classique, au moins un anticorps monoclonal présentant les réactivités croisées indiquées dans la revendication 17 et étant obtenu à partir d'une lignée cellulaires d'hybridomes selon la revendication 14, ainsi qu'à partir de ses clones et sous-clones.

**43.** Composant de couplage du triasulfuron de formule (B)

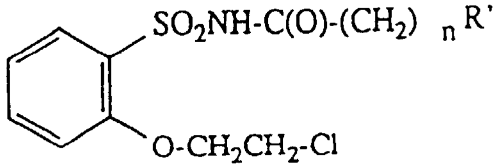

(B)

où R' représente COOH, $NH_2$ ou SH et n est un nombre entier allant de 1 à 10.

**44.** Composant de couplage du triasulfuron selon la revendication 43, caractérisé en ce que R' représente COOH et n est un nombre entier allant de 1 à 6.

**45.** Composant de couplage du triasulfuron selon la revendication 44, caractérisé en ce que R' représente COOH et n est égal à 2.

**46.** Procédé pour la préparation d'un composant de couplage de sulfonylurée essentiellement constitué de la partie sulfonamide de la molécule de sulfonylurée et qui couplé à une molécule porteuse de poids moléculaire élevé, appropriée, est en mesure de déclencher une réponse immunitaire spécifique, dirigée sur la partie sulfonamide, chez l'animal donneur, caractérisé en ce que l'on clive par hydrolyse la fonction sulfonamide sur l'herbicide sulfonylurée par des méthodes connues en sol, en ce que l'on fait réagir le groupe $-SO_2-NH_2$ ainsi obtenu avec un dérivé d'un acide carboxylique réactif et en ce que l'on transforme éventuellement le groupe COOH terminal ainsi formé en un groupe $NH_2$ ou SH par des procédés connus .

**47.** Procédé pour la préparation d'un composant de couplage de triasulfuron selon la revendication 46 de formule (B)

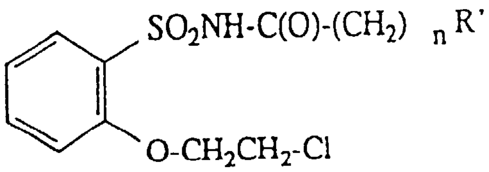

(B)

où

R' représente COOH, $NH_2$ ou SH et n est un nombre entier allant de 1 à 10, caractérisé en ce que l'on clive par hydrolyse la fonction sulfonamide sur l'herbicide sulfonylurée par des méthodes connues en soi, en ce que l'on fait réagir le groupe $-SO_2-NH_2$ ainsi obtenu avec un dérivé d'un acide carboxylique réactif et en ce que l'on transforme éventuellement le groupe COOH terminal ainsi formé en un groupe $NH_2$ ou SH par des procédés connus.

**Revendications pour l'Etat contractant suivant : ES**

**1.** Procédé pour la préparation d'une lignée cellulaire d'hybridome synthétisant un anticorps monoclonal présentant une spécificité et une affinité élevées vis-à-vis d'un ou plusieurs herbicides sulfonylurées, caractérisé

(a) en ce que l'on conjugue un composant de couplage essentiellement constitué de la partie sulfonamide de la molécule visée avec une molécule porteuse de poids moléculaire élevé, appropriée ;

(b) en ce que l'on immunise un animal donneur avec le conjugué préparé selon (a) ;

(c) en ce que l'on isole les cellules B immunocompétentes de l'animal donneur immunisé ;

(d) en ce que l'on fusionne lesdites cellules B immunocompétentes avec des cellules tumorales aptes à une division cellulaire continue ; et

(e) en ce que l'on isole le produit de fusion obtenu et en ce que l'on clone, après sélection, les cellules d'hybridomes produisant l'anticorps désiré.

**2.** Procédé selon la revendication 1 , caractérisé en ce que ladite partie sulfonamide est un arylsulfonyle substitué.

**3.** Procédé selon la revendication 2 , caractérisé en ce quc ledit reste aryle est un reste phényle, pyridyle, thiényle ou pyrazolyle.

**4.** Procédé selon l'une des revendications 1 à 3, caractérisé en ce que ledit herbicide sulfonylurée est un composé de formule (A)

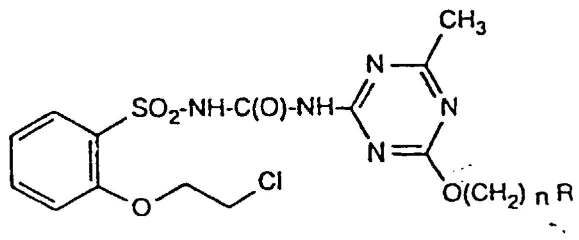

(A)

présentant en position 4 sur le noyau triazinique un groupement R-$(CH_2)_n$-O- où R représente COOH, lorsque n est un nombre entier allant de 1 à 10 et R représente COOH, $NH_2$ ou SH, lorsque n est un mmbre entier allant de 2 à 10.

**5.** Procédé selon l'une des revendications 1 à 4, caractérisé en ce que l'on utilise comme partie sulfonamide le fragment (B) stabilisé

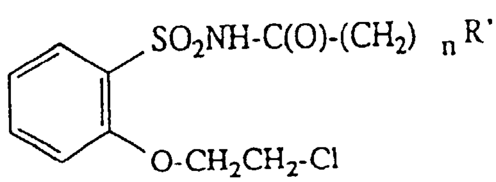

(B)

où R' représente COOH, $NH_2$ ou SH et n est un nombre entier allant de 1 à 10.

**6.** Procédé selon l'une des revendications 1 à 5, caractérisé en ce que R représente $NH_2$ dans la formule (A) et n est un nombre entier allant de 2 à 6.

**7.** Procédé selon la revendication 6, caractérisé en ce que R représente $NH_2$ et n est égal à 3.

**8.** Procédé selon l'une des revendications 1 à 7, caractérisé en ce que R' représente COOH dans la formule (B) et n est un nombre entier allant de 1 à 6.

**9.** Procédé selon la revendication 8, caractérisé en ce que R' représente COOH et n est égal à 2.

**10.** Procédé pour la préparation d'un anticorps monoclonal présentant une spécificité et une affinité élevées vis-à-vis d'un ou plusieurs herbicides sulfonylurées, caractérisé en ce que l'on cultive in vitro ou in vivo une lignée cellulaire d'hybridomes préparée selon l'une des revendications 1 à 9 et en ce que l'on isole les anticorps monoclonaux produits de la bouillie de culture.

**11.** Procédé selon l'une des revendications 1 à 9, caractérisé en ce que l'on utilise des composés macromoléculaires comme molécule porteuse, présentant des groupes réactifs librement accessibles pour la réaction de couplage avec le composant de couplage sulfonylurée et étant en mesure de conférer au composant sulfonylurée un pouvoir immunogène.

**12.** Procédé selon la revendication 11, caractérisé en ce que l'on utilise une protéine riche en lysine ayant un poids

moléculaire compris entre 10.000 et 1.500.000.

**13.** Procédé selon l'une des revendications 1 à 9, caractérisé, en ce que la conjugaison du composant de couplage sulfonylurée à la molécule porteuse s'effectue directement ou par l'intermédiaire d'un pont (espaceur) possédant un ou plusieurs groupes réactifs pouvant interagir avec les groupes réactifs de la molécule porteuse.

**14.** Procédé selon l'une des revendications 1 à 9, caractérisé en ce que la réaction de couplage est effectuée par la méthode de l'ester actif ou par le procédé au diazonium.

**15.** Procédé selon l'une des revendications 1 à 9, caractérisé en ce que l'imrnunisation des animaux donneurs est réalisée par l'administration en une ou plusieurs fois d'un conjugué constitué par le composant de couplage et la molécule porteuse.

**16.** Procédé selon l'une des revendications 4 à 9, caractérisé en ce que l'on utilise des lignées cellulaires de myélomes présentant les 9 de Sp2/O-Ag14 ou X63-Ag8.653.

**17.** Procédé selon l'une des revendications 1 à 9, caractérisé en ce que l'on utilise des lignées cellulaires de myélomes présentant les caractéristiques de PAI.

**18.** Procédé selon l'une des revendications 1 à 9, caractérisé en ce que l'on isole les cultures de cellules hybrides produisant des anticorps monoclonaux positifs par le procédé de dilution limite, puis en ce que l'on clone dans des milieux de culture appropriés.

**19.** Procédé selon l'une des revendications 1 à 9, caractérisé en ce que l'on étudie les clones de cellules d'hybridomes clonés selon la revendication 18 par un immunoessai pour la formation d'anticorps monoclonaux appropriés.

**20.** Procédé selon la revendication 1, caractérisé en ce que ledit anticorps monoclonal présente une spécificité et une affinité élevées vis-à-vis du triasulfuron et une réactivité croisée inférieure à 0,1 % avec les analogues du triasulfuron structurellement apparentés choisis dans le groupe constitué par les composés C, D, E, F, I (primisulfuron), J (sulfometuronméthyle), K (tribénuron-méthyle), L (thiameturon-méthyle), M (chlorsulfuron), N (bensulfuron-méthyle), O (metsulfuronnléthyle), P (nicosulfuron-méthylq) et Q (DPX 9636) du Tableau 1.

**21.** Procédé selon la revendication 1, caractérisé en ce que ledit anticorps monoclonal présente une spécificité et une affinité élevées vis-à-vis du triasulfuron et une réactivité croisée inféreure à à 0,01 % avec les analogues du triasulfuron struxcturellement apparentés choisis dans le groupe constitué par les composés C, D, E, F, I (primisulfuron), J (sulfometuron-méthyle), K (tribénuron-méthyle), L (thiameturon-méthyle), M (chlorsulfuron), N (bensulfuron-méthyle), O (metsulfuron-méthyle), P (nicosulfuron-méthyle) et Q (DPX 9636) du Tableau 1.

**22.** Procédé selon la revendication 21, caractérisé en ce que ledit anticorps monoclonal présente les réactivités croisées indiquées et en ce qu'il est obtenu à partir d'une lignée cellulaire d'hybridomes, ddposée sous le numéro ECACC 9002 1703, ainsi qu'à partir de ses clones et sous-clones.

**23.** Procédé selon la revendication 21, caractérisé en ce que ledit anticorps monoclonal présente les réactivités croisées indiquées et en ce qu'il est obtenu à partir d'une lignée cellulaire d'hybridomes, déposée sous le numéro ECACC 9002 1704, ainsi qu'à partir de ses clones et sous-clones.

**24.** Procédé pour la préparation des mutants et variants d'une lignée cellulaire d'hybridomes obtenue par un procédé selon l'une des revendications 1 à 9, lesdits mutants et variants étant encore en mesure de synthétiser des anticorps présentant les réactivités croisées indiquées dans les revendications 20 et 21 et de les sécréter dans le milieu environnant, caractérisé en ce que lesdits mutants et variants peuvent être préparés par des procédés connus en soi.

**25.** Procédé pour la préparation d'anticorps monoclonaux, caractérisé en ce que l'on cultive in vivo ou in vitro une lignée cellulaire d'hybridomes préparée selon l'une des revendications 12 à 19 par des procédés connus et en ce que l'on isole les anticorps monoclonaux produits.

**26.** Procédé selon la revendication 10 ou 25, caractérisé en ce que l'on utilise pour la culture, des milieux de culture standardisés choisis dans le groupe constitué par "Dulbecco's Modified Eagle Medium" (DMEM) ou RPMI 1640,

pouvant être éventuellement complétés par addition de sérums de mammifèress par des additifs favorisant la croissance ou par des oligoéléments.

**27.** Procédé selon la revendication 10 ou 25, caracctérisé en ce que l'on produit in vivo chez un animal donneur lesdits anticorps monoclonaux par expansion d'une lignée cellulaire d'hybridomes préparée selon l'une des revendications 11 à 22.

**28.** Procédé pour la détection immunologique des herbicides sulfonylurées, caractérisé en ce que l'on utilise un anticorps monoclonal préparé selon la revendication 10.

**29.** Procédé pour la détection immunologique du triasulfuron, caractérisé en ce que l'on utilise un anticorps monoclonal préparé selon l'une des revendications 25 à 27.

**30.** Procédé pour la détection immunologique du triasulfuron selon la revendication 28, caractérisé en ce que l'on utilise un anticorps monoclonal obtenu à partir d'une lignée cellulaire d'hybridomes possédant les caractéristiques de ECACC 9002 1703 ou à partir de ses clones ou sous-clones.

**31.** Procédé pour la détection immunologique du triasulfuron selon la revendication 28, caractérisé en ce que l'on utilise un anticorps monoclonal obtenu à partir d'une lignée cellulaire d'hybridomes possédant les caractéristiques de ECACC 9002 1704 ou à partir de ses clones ou sous-clones.

**32.** Procédé selon l'une des revendications 28 à 31, caractérisé en ce qu'il s'agit d'un immunoessai compétitif.

**33.** Procédé selon l'une des revendications 28 à 31, caractérisé en ce que ledit immunoessai est un radio-inmunoessai (RIA), ou un essai couplé une enzyme (ELISA) ou un essai chimioluminescent.

**34.** Moyens pour la détection immunologique des herbicides sulfonylurées sous forme d'une trousse d'essai prête à l'emploi, caractérisés en ce que ladite trousse d'essai contient à côté des supports, réactifs et autres additifs, utilisés de façon classique, au moins un anticorps monoclonal préparé selon la revendication 10 comme réactif.

**35.** Moyens pour la détection immunologique du triasulfuron sous forme d'une trousse d'essai prête à l'emploi, caractérisés en ce que ladite trousse d'essai contient à côté des supports, réactifs et autres additifs, utilisés de façon classique, au moins un anticorps monoclonal préparé selon l'une des revendications 25 à 27 comme réactif.

**36.** Moyens pour la détection immunologique du triasulfuron sous forme d'une trousse d'essai prête à l'emploi selon la revendication 34, caractérisés en ce que ladite trousse d'essai contient à côté des supports, réactifs et autres additifs, utilisés de façon classique, au moins un anticorps monoclonal obtenu à partir d'une lignée cellulaire d'hybridomes possédant les caractéristiques de ECACC 902 1703 ou à partir de ses clones ou sous-clones.

**37.** Moyens pour la détection immunologique du triasulfuron sous forme d'une trousse d'essai prête à l'emploi selon la revendication 34, caractérisés en ce que ladite trousse d'essai contient à côté des supports, réactits et autres additifs, utilisés de façon classique, au moins un anticorps monoclonal obtenu à partir d'une lignée cellulaire d'hybridomes possédant les caractéristiques de ECACC9002 1704 ou à partir de ses clones ou sous-clones.

**38.** Procédé pour la préparation d'un composant de couplage de sulfonylurée essentiellement constitué de la partie sulfonamide de la molécule de sulfonylurée et qui couplé, à une molécule porteuse de poids moléculaire élevé, appropriée, est en mesure de déclencher une réponse immunitaire spécifique, dirigée sur la partie sulfonamide, chez l'animal donneur, caractérisé en ce que l'on clive par hydrolyse la fonction sulfonamide sur l'herbicide sulfonylurée par des méthodes connues en soi, en ce que l'on fait réagir le groupe $-SO_2-NH_2$ ainsi obtenu avec un dérivé d'un acide carboxylique réactif et en ce que l'on transforme éventuellement le groupe COOH terminal ainsi formé en un groupe $NH_2$ ou SH par des procédés connus.

**39.** Procédé selon la revendication 38, caractérisé en ce que ledit composant de couplage est le composant de couplage du triasulfuron de formule (B)

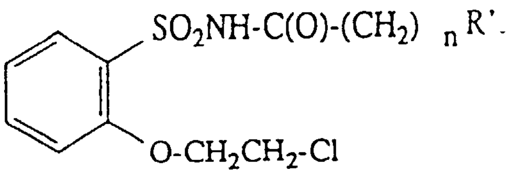

(B)

où

R' représente COOH, $NH_2$ ou SH et
n est un nombre entier allant de 1 à 10.